# EUROPEAN PATENT SPECIFICATION

(11) **EP 4 045 506 B1**
(45) Date of publication and mention of the grant of the patent: **23.07.2025**
(21) Application number: 20876511.5
(22) Date of filing: 14.10.2020
(51) Int. Cl.: C07D 471/04, A61P 35/00, A61K 31/437

(54) **DERIVATIVES OF 4-(IMIDAZO[1,2-A]PYRIDIN-3-YL)-N-(PYRIDIN-3-YL) PYRIMIDIN-2- AMINE FOR TREATING PROLIFERATIVE DISEASES AND CONDITIONS**
4-(IMIDAZO[1,2-A]PYRIDIN-3-YL)-N-(PYRIDIN-3-YL)PYRIMIDIN-2-AMIN-DERIVATE ZUM BEHANDELN PROLIFERATIVER KRANKHEITEN UND ZUSTÄNDE
DÉRIVÉS DE 4-(IMIDAZO[1,2-A] PYRIDIN-3-YL)-N-(PYRIDIN-3-YL)PYRIMIDIN-2-AMINE POUR LE TRAITEMENT DE MALADIES ET D'AFFECTIONS PROLIFÉRATIVES

(30) Priority: 15.10.2019 AU 2019903877
(43) Date of publication of application: 24.08.2022
(73) Proprietor: Aucentra Therapeutics Pty Ltd, Adelaide, SA 5000 (AU)
(72) Inventor: WANG, Shudong, Adelaide, South Australia 5000 (AU); LONG, Yi, Campbelltown, South Australia 5074 (AU); YU, Mingfeng, Marden, South Australia 5070 (AU); AL HAJ DIAB, Sarah, North Melbourne, Victoria 3051 (AU)
(74) Representative: Gill Jennings & Every LLP
(86) International application number: PCT/AU2020/000122
(87) International publication number: WO 2021/072475

(56) References cited:
- WO-A1-2008/008059
- WO-A1-2010/138575
- WO-A2-2010/009155

## Description

### TECHNICAL FIELD

The present disclosure relates to a novel class of inhibitors of protein kinases useful in the treatment of proliferative cell diseases and conditions including cancers.

### PRIORITY DOCUMENT

The present application claims priority from Australian Provisional Patent Application No 2019903877 titled "Inhibitors of protein kinases for use in therapy" filed on 15 October 2019.

### BACKGROUND

There is an ongoing need to identify and develop new compounds for treating proliferative diseases and conditions including cancers. Among the numerous "targets" for potential anti-proliferative compounds under investigation are the group of enzymes known as protein kinases.

Cyclin-dependent kinases (CDKs) are a type of protein kinase. They are known to be associated with various cyclin subunits, playing pivotal roles in the regulation of a variety of important regulatory pathways in cells, including cell-cycle control, apoptosis, neuronal physiology, differentiation and transcription. There are more than 20 CDKs which may be classified into two major groups, reflecting their functions, namely, the cell cycle regulator CDKs and the transcription regulator CDKs. The class of the cell cycle regulator CDKs includes CDK1, CDK2, CDK3, CDK4 and CDK6, and they function with their cyclin partners (eg cyclin A, B, D1, D2, D3, E and F) to regulate promotion of the cell cycle. The class of the transcription regulator CDKs includes CDK7, CDK8, CDK9 and CDK11, which work together with cyclin C, H, K, L1, L2, T1 and T2 and tend to play roles in transcriptional regulation (Wang S et al. Trends in Pharms Sci, 302-312, 2008). Given the functions of these two CDK classes, it is perhaps not surprising that CDKs have been implicated in cell proliferation diseases and conditions, particularly cancer. Cell proliferation is a result of the direct or indirect deregulation of the cell division cycle and the CDKs play a critical role in the regulation of the various phases of this cycle. Therefore, inhibitors of CDKs and their associated cyclins are considered to be useful targets for cancer therapy.

Another type of protein kinase considered to be a useful target for cancer therapy is the tropomyosin receptor kinase family comprising TRKA, TRKB, and TRKC encoded by the *NTRK1,* 2, and 3 genes, respectively. NTRK chromosomal alterations, most commonly gene fusions, have been identified as driver mutations in a broad range of malignancies. Inhibitors of TRK, including larotrectinib, have shown broad clinical activity across multiple tumor types with NTRK fusion events (Cocco E. et al. Nat Rev Clin Oncol 15(12):731-747, 2018).

Another type is the dual-specificity tyrosine phosphorylation-regulated kinase (DYRK) family proteins, which are related to the mitogen-activated protein kinase (MAPK family). DYRK1A and DYRK1B are emerging therapeutic targets in cancer therapy. Because they drive cell-cycle exit and quiescence of tumour cells, DYRK1 inhibitors are expected to promote cell-cycle re-entry of quiescent tumour cells and thereby increase their susceptibility to chemotherapy or radiotherapy (Soppa U. et al., Curr Biol 25(12):R488-9, 2015). Moreover, increased activity of DYRK1A has been correlated with abnormal brain development, cognitive disabilities and an early onset of Alzheimer's disease in Down syndrome, suggesting that DYRK1 inhibitors may also represent a new opportunity for treating these disorders (Soppa, U. et al., Cell Cycle 13(13):2084-2100, 2014).

FMS-like tyrosine kinase 3 (FLT3), also known as CD135 antigen and foetal liver kinase-2 (Flk2) is expressed on the surface of many haematopoietic cells and the signalling of FLT3 is known to play an important role in the normal development of these cells. The overly activated FLT3 and its mutations thereof have been associated with certain cancers, particularly blood cancers (ie haematological malignancies). For example, high levels of FLT3 have been found in the blast cells of some patients with acute myeloid leukaemia (AML) (Zheng R et al., Blood 103(1):267-274, 2004), and aberrant FLT3 expression has also been found in acute lymphocytic leukemia (ALL) and chronic lymphocytic leukemia (CLL) (Rosnet O et al., Leukemia 10:238-248, 1996). It has been speculated that aberrant- or over-expression of FLT3 might lead to constitutive dimerisation and activation of the receptor (Brasel K et al., Leukemia 9:1212-1218, 1995). Moreover, a number of FLT3 "activating" mutations have been identified, most notably the internal tandem duplication (ITD) found in the juxta-membrane domain (JM) and point mutations in the tyrosine kinase domain (TKD), both leading to constitutive activation of FLT3 kinase activity, and thereby the downstream signalling pathways supporting the myeloproliferative phenotype (Yokota S. et al., Leukemia 11(10): 1605-1609, 1997). These mutations are known to be associated with very poor prognosis in patients with AML (Kottaridis PD et al., Blood 98(6):1752-1759, 2001). A number of FLT3 inhibitor compounds have been evaluated for the treatment of haematological malignancies such as AML and other FLT3-activated cancers. WO 2010/009155 discloses azacyles having inhibitory CDK activity.

The present applicant has now identified a new class of pyrimidine compounds for use in the prevention and/or treatment of proliferative diseases and conditions including cancers. While not wishing to be bound by theory, it is considered that these novel compounds are capable of inhibiting cell proliferation by inhibiting the activity of CDKs, especially one or more of CDK2, CDK4, CDK6 and CDK9, and/or other protein kinases such as TRKs, DYRKs and FLT3 and/or mutant forms thereof.

**SUMMARY** Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

According to a first aspect, the present disclosure provides a compound of formula I: 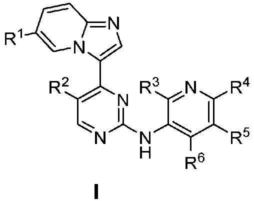 wherein:
R¹, R², R³, R⁴, R⁵ and R⁶ are each independently selected from the group consisting of H, alkyl, alkyl-R⁷, aryl, aryl-R⁷, aralkyl, aralkyl-R⁷, alicyclic, heterocyclic, halogen, NO₂, CN, CF₃, OH, O-alkyl, COR⁷, COOR⁷, O-aryl, O-R⁷, NH₂, NH-alkyl, NH-aryl, N-(alkyl)₂, N-(aryl)₂, N-(alkyl)(aryl), NH-R⁷, NH-alkyl-N(alkyl)₂, N-(R⁷)(R⁸), N-(alkyl)(R⁷), N-(aryl)(R⁷), COOH, CONH₂, CONH-alkyl, CONH-aryl, CONH-alicyclic, CON-(alkyl)(R⁷), CON(aryl)(R⁷), CONH-R⁷, CON-(R⁷)(R⁸), SH-alkyl, SO₃H, SO₂-alkyl, SO₂-alkyl-R⁷, SO₂-aryl, SO₂-aryl-R⁷, SO₂NH₂, SO₂NH-R⁷, SO₂N-(R⁷)(R⁸), CF₃, CO-alkyl, COO-alkyl, CO-alkyl-R⁷, CO-aryl, CO-aryl-R⁷ and R⁹, wherein said alkyl, aryl, aralkyl, alicyclic and heterocyclic groups may be optionally substituted with one or more groups selected from halogen, CN, OH, O-C₁₋₆ alkyl, NH₂, COOH, C₂₋₅ carboxylate, CONH₂ and haloalkyl; and
R⁷, R⁸ and R⁹ are independently selected from water solubilising groups; (as defined in the claims) or a pharmaceutically acceptable salt, solvate thereof.

In a second aspect, the present disclosure provides the use of a compound as defined in the first aspect or a pharmaceutically acceptable salt, solvate thereof, for treating a disease or condition such as, for example, cancer or another proliferative disorder or condition.

In a third aspect, the present disclosure provides a method of treating a disease or condition such as, for example, cancer or another proliferative disorder or condition in a subject, the method comprising administering to said subject a therapeutically effective amount of a compound as defined in the first aspect or a pharmaceutically acceptable salt, solvate thereof, optionally in combination with a pharmaceutically acceptable carrier, diluent and/or excipient.

In a fourth aspect, the present disclosure provides the use of a compound as defined in the first aspect, or a pharmaceutically acceptable salt, solvate thereof, in the manufacture of a medicament for treating a disease or condition such as, for example, cancer or another proliferative cell disease or condition.

In a fifth aspect, the present disclosure provides a pharmaceutical composition or medicament comprising a compound as defined in the first aspect, or a pharmaceutically acceptable salt, solvate thereof, and a pharmaceutically acceptable carrier, diluent and/or excipient.

In a sixth aspect, the present disclosure provides a method for modulating protein kinase activity in a cell, comprising introducing to or contacting said cell with an effective amount of a compound as defined in the first aspect or a pharmaceutically acceptable salt, solvate thereof.

### DETAILED DESCRIPTION

The present applicant has now identified a new class of pyrimidin-2-amine derivatives, particularly derivatives of 4-(imidazo[1,2-a]pyridin-3-yl)-N-(pyridin-3-yl)pyrimidin-2-amine, suitable for use in the prevention and/or treatment of proliferative disorders and conditions including cancers, which possess desirable biological activity (eg the compounds may inhibit cell proliferation and cause cancer cell apoptosis by inhibiting the activity of cell cycle and transcriptional CDKs, and other protein kinases such as FLT3).

According to a first aspect, the present disclosure provides a compound of formula I shown below: 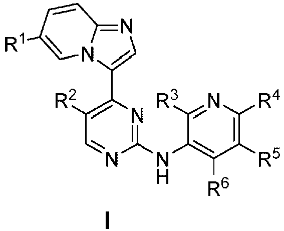 wherein:
R¹, R², R³, R⁴, R⁵ and R⁶ are each independently selected from the group consisting of H, alkyl, alkyl-R⁷, aryl, aryl-R⁷, aralkyl, aralkyl-R⁷, alicyclic, heterocyclic, halogen, NO₂, CN, CF₃, OH, O-alkyl, COR⁷, COOR⁷, O-aryl, O-R⁷, NH₂, NH-alkyl, NH-aryl, N-(alkyl)₂, N-(aryl)₂, N-(alkyl)(aryl), NH-R⁷, NH-alkyl-N(alkyl)₂, N-(R⁷)(R⁸), N-(alkyl)(R⁷), N-(aryl)(R⁷), COOH, CONH₂, CONH-alkyl, CONH-aryl, CONH-alicyclic, CON-(alkyl)(R⁷), CON(aryl)(R⁷), CONH-R⁷, CON-(R⁷)(R⁸), SH-alkyl, SO₃H, SO₂-alkyl, SO₂-alkyl-R⁷, SO₂-aryl, SO₂-aryl-R⁷, SO₂NH₂, SO₂NH-R⁷, SO₂N-(R⁷)(R⁸), CF₃, CO-alkyl, COO-alkyl, CO-alkyl-R⁷, CO-aryl, CO-aryl-R⁷ and R⁹, wherein said alkyl, aryl, aralkyl, alicyclic and heterocyclic groups may be optionally substituted with one or more groups selected from halogen, CN, OH, O-C₁₋₆ alkyl, NH₂, COOH, C₂₋₅ carboxylate, CONH₂ and haloalkyl; and
R⁷, R⁸ and R⁹ are independently selected from water solubilising groups;
or a pharmaceutically acceptable salt, solvate thereof.

In some embodiments, the compounds of formula I may preferably comprise at least one water solubilising group R⁷, R⁸ and R⁹. That is, in such embodiments, the compound is as defined above in the preceding paragraph with the proviso that said compound comprises at least one of said R⁷, R⁸ and R⁹ groups or, more preferably, comprises at least one of said R⁷ or R⁸ groups. The present applicant has found that notwithstanding the addition of such solubilising group(s), the compounds possess desirable biological activity (eg by inhibiting the activity of CDKs and/or FLT3). The presence of at least one water solubilising group may enhance *in vivo* absorption and oral bioavailability.

The compounds of formula I have been found to possess anti-proliferative activity and are therefore considered to be of use in the treatment of proliferative cell diseases and conditions such as cancer, leukaemia, lymphoma and other diseases and conditions associated with uncontrolled cell proliferation (or, in other words, requires control of the cell cycle) such as, for example, some cardiovascular diseases or conditions such as restenosis and cardiomyopathy, some auto-immune diseases such as glomerulonephritis and rheumatoid arthritis, dermatological conditions such as psoriasis, and fungal or parasitic disorders. As used herein, an anti-proliferative effect within the scope of the present disclosure may be demonstrated by the ability to inhibit cell proliferation in an *in vitro* whole cell assay. An example(s) of such an assay, including methods for performance, are described in more detail in the examples provided hereinafter.

The compounds of formula I may inhibit any of the steps or stages in the cell cycle, for example, formation of the nuclear envelope, exit from the quiescent phase of the cell cycle (G0), G1 progression, chromosome decondensation, nuclear envelope breakdown, START, initiation of DNA replication, progression of DNA replication, termination of DNA replication, centrosome duplication, G2 progression, activation of mitotic or meiotic functions, chromosome condensation, centrosome separation, microtubule nucleation, spindle formation and function, interactions with microtubule motor proteins, chromatid separation and segregation, inactivation of mitotic functions, formation of contractile ring, and cytokinesis functions. In particular, the compounds of formula I may influence certain gene functions such as chromatin binding, formation of replication complexes, replication licensing, phosphorylation or other secondary modification activity, proteolytic degradation, microtubule binding, actin binding, septin binding, microtubule organising centre nucleation activity and binding to components of cell cycle signalling pathways.

Thus, in a second aspect, the present disclosure provides the use of a compound as defined in the first aspect or a pharmaceutically acceptable salt, solvate thereof, for treating a disease or condition such as, for example, cancer or another proliferative disorder or condition.

In a third aspect, the present disclosure provides a method of treating a disease or condition such as, for example, cancer or another proliferative disorder or condition in a subject, the method comprising administering to said subject a therapeutically effective amount of a compound as defined in the first aspect or a pharmaceutically acceptable salt, solvate thereof, optionally in combination with a pharmaceutically acceptable carrier, diluent and/or excipient.

In a fourth aspect, the present disclosure provides the use of a compound as defined in the first aspect, or a pharmaceutically acceptable salt, solvate thereof, in the manufacture of a medicament for treating a disease or condition such as, for example, cancer or another proliferative cell disease or condition.

In a fifth aspect, the present disclosure provides a pharmaceutical composition or medicament comprising a compound as defined in the first aspect, or a pharmaceutically acceptable salt, solvate thereof, and a pharmaceutically acceptable carrier, diluent and/or excipient.

In a sixth aspect, the present disclosure provides a method for modulating protein kinase activity in a cell, comprising introducing to or contacting said cell with an effective amount of a compound as defined in the first aspect or a pharmaceutically acceptable salt, solvate thereof.

Preferably, the method of the sixth aspect modulates the activity of one or more protein kinases selected from CDKs such as CDK2, CDK4, CDK6 and/or CDK9, and/or other protein kinases such as FLT3 and its mutants.

In this specification, a number of terms are used which are well known to those skilled in the art. Nevertheless, for the purposes of clarity, a number of these terms are hereinafter defined.

As used herein, the term "treating" includes prophylaxis as well as the alleviation of established symptoms of a condition. As such, the act of "treating" a disease or condition therefore includes: (1) preventing or delaying the appearance of clinical symptoms of the disease or condition developing in a subject afflicted with or predisposed to the disease or condition; (2) inhibiting the disease or condition (ie arresting, reducing or delaying the development of the disease or condition or a relapse thereof, in case of a maintenance treatment, or at least one clinical or subclinical symptom thereof; and (3) relieving or attenuating the disease or condition (ie causing regression of the disease or condition or at least one of its clinical or subclinical symptoms).

As used herein, the term "alkyl" includes both straight chain and branched alkyl groups having from 1 to 8 carbon atoms (eg methyl, ethyl propyl, isopropyl, butyl, isobutyl, tert-butyl, pentyl, hexyl etc).

As used herein, the term "aryl" refers to a substituted (mono- or poly-) or unsubstituted monoaromatic or polyaromatic group, wherein said polyaromatic group may be fused or unfused. The term therefore includes groups having from 6 to 10 carbon atoms (eg phenyl, naphthyl etc). It is also to be understood that the term "aryl" is synonymous with the term "aromatic".

As used herein, the term "aralkyl" is used as a conjunction of the terms alkyl and aryl as defined above.

The term "aliphatic" takes its normal meaning in the art and includes non-aromatic groups such as alkanes, alkenes and alkynes and substituted derivatives thereof.

As used herein, the term "alicyclic" refers to a cyclic aliphatic group.

The term "halogen" refers to fluoro, chloro, bromo and iodo.

As used herein, the term "heterocyclic" refers to a saturated or unsaturated cyclic group comprising one or more heteroatoms in the ring.

The term "derivative" as used herein, includes any chemical modification of an entity. Illustrative of such chemical modifications is the replacement of hydrogen by a halogen group, an alkyl group, an acyl group or an amino group.

As used herein, the phrase "manufacture of a medicament" includes the use of one or more of the compounds of formula I directly as the medicament or in any stage of the manufacture of a medicament comprising one or more of the compounds of formula I.

Some of the compounds of formula I may exist as single stereoisomers, racemates, and/or mixtures of enantiomers and /or diastereomers. All such single stereoisomers, racemates and mixtures thereof, are encompassed within the scope of the present disclosure. The isomeric forms such as diastereomers, enantiomers, and geometrical isomers can be separated by physical and/or chemical methods known to those skilled in the art.

The term "pharmaceutically acceptable salt" as used herein, refers to salts that retain the desired biological activity of the compounds of formula I, and include pharmaceutically acceptable acid addition salts and base addition salts. Suitable pharmaceutically acceptable acid addition salts of the compounds of formula I may be prepared from an inorganic acid or from an organic acid. Examples of such inorganic acids are hydrochloric, sulfuric and phosphoric acid. Appropriate organic acids may be selected from aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic and arylsulfonic. Additional information on pharmaceutically acceptable salts can be found in Remington's Pharmaceutical Sciences, 19th Edition, Mack Publishing Co., Easton, PA 1995.

The term "solvate" refers to any form of the compounds of formula I, resulting from solvation of with an appropriate solvent. Such a form may be, for example, a crystalline solvate or a complex that may be formed between the solvent and the dissolved compound.

The term "prodrug" (not part of the invention) means a compound that undergoes conversion to a compound of formula I within a biological system, usually by metabolic means (eg by hydrolysis, reduction or oxidation). For example, an ester prodrug of a compound of formula I containing a hydroxyl group may be convertible by hydrolysis *in vivo* to the compound of formula I. Suitable esters of the compounds of formula I containing a hydroxyl group may be, for example, acetates, citrates, lactates, tartrates, malonates, oxalates, salicylates, propionates, succinates, fumarates, maleates, methylene-bis-P-hydroxynaphthoates, gestisates, isethionates, di-p-toluoyltartrates, methanesulfonates, ethanesulfonates, benzenesulfonates, p-toluenesulfonates, cyclohexylsulfamates and quinates. As another example, an ester prodrug of a compound of formula I containing a carboxy group may be convertible by hydrolysis *in vivo* to the compound of formula I. Examples of ester prodrugs include those described by Leinweber FJ, Drug Metab Rev 18:379-439 (1987). Similarly, an acyl prodrug of a compound of formula I containing an amino group may be convertible by hydrolysis *in vivo* to the compound of formula I. Examples of prodrugs for these and other functional groups, including amines, are provided in Prodrugs: challenges and rewards, Valentino J Stella (ed), Springer, 2007.

In the case of compounds of formula I that are solid, it will be understood by those skilled in the art that the compounds (or pharmaceutically acceptable salts, solvates or prodrugs thereof) may exist in different crystalline or polymorphic forms, all of which are encompassed within the scope of the present disclosure.

The term "therapeutically effective amount" or "effective amount" is an amount sufficient to effect beneficial or desired clinical results. A therapeutically effective amount can be administered in one or more administrations. Typically, a therapeutically effective amount is sufficient for treating a disease or condition or otherwise to palliate, ameliorate, stabilise, reverse, slow or delay the progression of a disease or condition such as, for example, cancer or another proliferative cell disease or condition. By way of example only, a therapeutically effective amount of a compound of formula I, or a pharmaceutically acceptable salt, solvate thereof, may comprise between about 0.1 and about 250 mg/kg body weight per day, more preferably between about 0.1 and about 100 mg/kg body weight per day and, still more preferably between about 0.1 and about 25 mg/kg body weight per day. However, notwithstanding the above, it will be understood by those skilled in the art that the therapeutically effective amount may vary and depend upon a variety of factors including the activity of the particular compound (or salt, solvate thereof), the metabolic stability and length of action of the particular compound (or salt, solvate thereof), the age, body weight, sex, health, route and time of administration, rate of excretion of the particular compound (or salt, solvate thereof), and the severity of, for example, the cancer or other proliferative cell disease or condition to be treated.

The compounds of formula I, and pharmaceutically acceptable salts, solvates and prodrugs thereof, are capable of inhibiting protein kinases, especially CDKs and may show higher selectivity for (ie to inhibit) CDK9 over other protein kinases such as CDK2, CDK4 and/or CDK6, or *vice versa,* or selective for FLT3 over one or more CDKs. As mentioned above, CDK9 through its role as a transcription regulator may promote cancer cell proliferation and resist to apoptosis. As such, the compounds of formula I, and pharmaceutically acceptable salts, solvates and prodrugs thereof, which are believed to inhibit at least CDK9, have utility in both *in vitro* and *in vivo* applications (eg *in vitro* cell-based assays) and as the basis of a therapeutic method of treating cancer or another proliferative disorder or condition in a subject.

The compounds of formula I may bear at least one water solubilising group (eg provided by R⁷, R⁸ and/or R⁹). The term "water solubilising group" will be well understood by those skilled in the art as referring to any polar functional group which either ionises or is capable of forming hydrogen bonds with water molecules to increase the water solubility of the compound (ie relative to the water solubility of the corresponding compound lacking the water solubilising group). Examples of suitable water solubilising groups and methods and considerations for their introduction are described in, for example, Fundamentals of Medicinal Chemistry by Gareth Thomas (publisher: John Wiley & Sons).

Preferably, where present, R⁷ and R⁸ are independently selected from water solubilising groups of the group consisting of:
(i) mono-, di- and poly-hydroxylated alicyclic groups, di- or poly-hydroxylated aliphatic or aryl groups, N-, O- and/or S-containing heterocyclic groups optionally substituted with one or more hydroxyl, amino or alkoxy groups, aliphatic and aryl groups comprising one or more carboxamide, sulfoxide, sulfone or sulfonamide groups, and halogenated alkylcarbonyl groups; and
(ii) COOH, SO₃H, OSO₃H, SONHCH₃, SONHCH₂CH₃, SO₂CH₃, SO₂CH₂CH₃, PO₃H₂ and OPO₃H₂.

Preferably, where present, R⁹ is selected from water solubilising groups of the group consisting of:
(i) mono-, di- and poly-hydroxylated alicyclic groups, di- or poly-hydroxylated aliphatic or aryl groups; N-, O- and/or S-containing heterocyclic groups (including bicyclics such as a diazaspiro heterocycle groups) optionally substituted with one or more hydroxyl, sulfone, alkyl, (CH₂)ₙ-heterocycle (where n is selected from the integers 1 or 2 and the heterocycle is a saturated or unsaturated 5- or 6-membered cyclic group comprising one or two N, O or S heteroatoms, such as piperidine, phenylimidazole, pyran or furan), CO-heterocycle (wherein the heterocycle is a saturated or unsaturated 5- or 6-membered cyclic group comprising one or two N, O or S heteroatoms, such as such as piperidine, phenylimidazole, pyran or furan), NH-heterocycle (wherein the heterocycle is a saturated or unsaturated 5- or 6-membered cyclic group comprising one or two N, O or S heteroatoms, such as piperidine, phenylimidazole, pyran or furan), amino, alkoxy, carboxylate or alkylcarbonyl groups; aliphatic, alicyclic and aryl groups comprising one or more carboxamide, sulfoxide, sulfone or sulfonamide groups; and wherein the N-, O- and/or S-containing heterocyclic group may optionally comprise an alkyl bridge (eg a -CH₂- or -CH₂CH₂- bridge), amine bridge (eg -NH-, -NH-CH₂- and -NH-CH₂CH₂-), amide bridge (eg - C(=O)NH-), alkoxy bridge (eg -O-CH₂- and -O-CH₂CH₂-) or ketone bridge (eg a -C(=O)-bridge) to, for example, the carbon atom at position 4/5 of the pyridine ring of the Formula I compound; and halogenated alkylcarbonyl groups;
(ii) COOH, SO₃H, OSO₃H, PO₃H₂ and OPO₃H₂;
(iii) NHCO(CH₂)ₘ[NHCO(CH₂)_{m'}]ₚ[NHCO(CH₂)_{m"}]_{q}A and NHCO(CH₂)ₜNH(CH₂)_{t'}A wherein p and q are each independently selected from integers 0 or 1, and m, m', m", t and ' are each independently selected from integers 1 to 10, and A is selected from:
   (a) alicyclic, aryl and heterocyclic groups comprising one or more O-, S- or N- heteroatoms, which may further comprise an alkyl bridge (eg a -CH₂- or -CH₂CH₂ - bridge),
   (b) alicyclic groups comprising one or more of -O-, NH₂, -NH-, =N-, quaternary amine salt, and amidine, and
   (c) morpholine, piperazine or 1,4-diazepane groups, each of which may be optionally substituted by one or more substituents selected from SO₂-alkyl, alkyl optionally substituted by one or more OH groups, CO-alkyl, aralkyl, COO-alkyl, and an ether group optionally substituted by one or more OH groups;
(iv) (CH₂)ₙNR¹⁰COR¹¹, (CH₂)_{n'}NR¹⁰SO₂R¹¹ and SO₂R¹², wherein R¹¹ is selected from H and alkyl, R¹² and R¹² are each independently selected from alkyl groups optionally comprising one or more heteroatoms and/or optionally substituted with one or more substituents independently selected from OH, NH₂, halogen and NO₂, and n and n' are each independently selected from integers 0, 1, 2 and 3;
(v) ether and polyether groups optionally substituted with one or more OH groups or one or more A groups, wherein A is as defined above at (iii);
(vi) (CH₂)ᵣNH₂, wherein r is selected from integers 0, 1, 2 and 3;
(vii) (CH₂)_{r'}OH, wherein r' is selected from integers 0, 1, 2 and 3;
(viii) (CH₂)_{n"}NR¹³COR¹⁴, wherein R¹³ is H or alkyl, n" is selected from integers 0, 1, 2 and 3, and R¹⁴ is an aryl group optionally substituted with one or more substituents selected from halogen, NO₂, OH, alkoxy, NH₂, COOH, CONH₂ and CF₃; and
(ix) SO₂NR¹⁵R¹⁶, wherein R¹⁵ and R¹⁶ are each independently selected from H, alkyl and aryl, with the proviso that at least one of R¹⁵ and R¹⁶ is other than H, or R¹⁵ and R¹⁶ together form a cyclic group optionally comprising one or more heteroatoms selected from N, O and S, and wherein said alkyl, aryl or cyclic group is optionally substituted by one or more substituents selected from halogen, NO₂, OH, alkoxy, NH₂, COOH, CONH₂ and CF₃.

In some embodiments, R¹ and R² are each independently selected from the group consisting of H, alkyl, alkyl-R⁷, aryl, aryl-R⁷, aralkyl, aralkyl-R⁷, alicyclic, heterocyclic, halogen, NO₂, CN, CF₃, OH, O-alkyl, COR⁷, COOR⁷, O-aryl, O-R⁷, NH₂, NH-alkyl, NH-aryl, N-(alkyl)₂, N-(aryl)₂, N-(alkyl)(aryl), NH-R⁷, NH-alkyl-N(alkyl)₂, N-(R⁷)(R⁸), N-(alkyl)(R⁷), N-(aryl)(R⁷), COOH, CONH₂, CONH-alkyl, CONH-aryl, CON-(alkyl)(R⁷), CON(aryl)(R⁷), CONH-R⁷, CON-(R⁷)(R⁸), SH-alkyl, SO₃H, SO₂-alkyl, SO₂-alkyl-R⁷, SO₂-aryl, SO₂-aryl-R⁷, SO₂NH₂, SO₂NH-R⁷, SO₂N-(R⁷)(R⁸), CF₃, CO-alkyl, CO-alkyl-R⁷, CO-aryl, CO-aryl-R⁷ and R⁸, wherein said alkyl, aryl, aralkyl, alicyclic and heterocyclic groups may be optionally substituted with one or more groups selected from halogen, CN, OH, O-C₁₋₆ alkyl (eg O-methyl), NH₂, COOH, C₂₋₅ carboxylate (eg COOC(CH₃)₃), CONH₂ and haloalkyl (eg CHF₂ and CF₃); and R³, R⁴, R⁵ and R⁶ are each independently selected from the group consisting of H, alkyl-R⁷, aryl, aryl-R⁷, aralkyl, aralkyl-R⁷, alicyclic, heterocyclic, CF₃, COR⁷, COOR⁷, O-aryl, O-R⁷, NH-aryl, N-(aryl)₂, N-(alkyl)(aryl), COO-alkyl (eg COO-C₁₋₃ alkyl such as COOCH₃), O-alkyl (eg a O-C₁₋₃ alkyl such as O-CH₂CH₃), NH-alkyl (eg a NH-C₁₋₃ alkyl such as NH-CH₂CH₂CH₃), N-(alkyl)₂ (eg N(CH₃)₂), NH-R⁷, N-(R⁷)(R⁸), N-(alkyl)(R⁷), N-(aryl)(R⁷), CONH-alkyl, CONH-aryl, CON-(alkyl)(R⁷), CON(aryl)(R⁷), CONH-R⁷, CON-(R⁷)(R⁸), SO₃H, SO₂-alkyl-R⁷, SO₂-aryl, SO₂-aryl-R⁷, SO₂NH-R⁷, SO₂N-(R⁷)(R⁸), CO-alkyl-R⁷, CO-aryl, CO-aryl-R⁷ and R⁹, wherein said alkyl, aryl, aralkyl, alicyclic and heterocyclic groups may be optionally substituted with one or more groups selected from halogen, CN, OH, O-methyl, NH₂, COOH, CONH₂ and CF₃.

In some embodiments, R¹ is H, alkyl (eg a C₁₋₆ alkyl or, preferably, a C₁₋₃ alkyl such as methyl, ethyl and C(CH₃)₂), aryl, CN, CF₃, NH₂, heterocyclic (eg a saturated or unsaturated 5- or 6-membered cyclic group comprising one or two N, O or S heteroatoms), O-alkyl (eg a O-C₁₋₃ alkyl such as O-CH₃), NH-alkyl (eg a NH-C₁₋₆ alkyl such as NH(C₅H₉) (ie NH-cyclopentyl) or, preferably, a NH-C₁₋₃ alkyl such as NH-CH₃), NH-alkyl-N(alkyl)₂ (eg a NH-alkyl-(C₁₋₆ alkyl)₂ such as NH(C₁₋₃ alkyl)-N(C₁₋₃ alkyl)₂), NH-aryl, N-(alkyl)₂ such as N(CH₃)₂, N-(alkyl)(aryl), SH-alkyl (eg a SH-C₁₋₆ alkyl or, preferably, a SH-C₁₋₃ alkyl such as SHCH₃ and SHC(CH₃)), halogen (preferably F, Br or Cl) or R⁹. Where R¹ is R⁹, preferably R⁹ is a mono-, di- or poly-hydroxylated alicyclic group, or an N-, O- and/or S-containing heterocyclic group substituted with one or more hydroxyl or amino group. Most preferably, R¹ is H, C₁₋₃ alkyl (eg methyl), aryl (eg phenyl, optionally substituted with C₁₋₆ alkyl, O-C₁₋₆ alkyl (eg O-methyl), amino (eg NH₂) and/or halogen (preferably F)), CF₃, heterocyclic (eg pyridine, including dihydro- and tetrahydropyridine, optionally substituted with a C₁₋₆ alkyl, C₂₋₅ carboxylate (eg COOC(CH₃)₃) or halogen (preferably F), pyrimidine optionally substituted with a C₁₋₆ alkyl, O-C₁₋₆ alkyl (eg O-methyl), amino (eg NH₂) or halogen (preferably F), a piperazine, pyrrole or pyrazole optionally substituted with a C₁₋₆ alkyl or CHF₂, thiophene, furan optionally substituted with a C₁₋₆ alkyl, and including bicyclic structures such as indole, benzofuran and benzo-thiophenyl) or halogen (preferably F, Br or Cl).

In some embodiments, R² is H, alkyl (eg a C₁₋₆ alkyl or, preferably, a C₁₋₃ alkyl such as methyl or ethyl), CN or halogen (preferably F). Most preferably, R² is H.

In some embodiments, at least one of R³, R⁴, R⁵ and R⁶, but most preferably R⁴, is alkyl-R⁷ (eg a C₁₋₃ alkyl-R⁷) wherein R⁷ is preferably selected from COOH, SO₃H, OSO₃H, SONHCH₃, SONHCH₂CH₃, SO₂CH₃, SO₂CH₂CH₃, PO₃H₂ and OPO₃H₂, but more preferably is SONHCH₃ or SONHCH₂CH₃, COO-alkyl (eg COOC₁₋₃alkyl such as COOCH₃), O-alkyl (eg a O-C₁₋₃ alkyl such as O-CH₂CH₃), NH-alkyl (eg a NH-C₁₋₃ alkyl such as NH-CH₂CH₂CH₃), N-(alkyl)₂ (eg N(CH₃)₂), NH-R⁷ wherein R⁷ is preferably selected from COOH, SO₃H, OSO₃H, SONHCH₃, SONHCH₂CH₃, SO₂CH₃, SO₂CH₂CH₃, PO₃H₂ and OPO₃H₂, but more preferably is SO₂CH₃ or SO₂CH₂CH₃, CONH-alicyclic (eg a CONH-C₃₋₆ alicyclic such as CONH-cyclopropyl), halogen (preferably Cl) or is R⁹ wherein R⁹ is preferably an N-, O- and/or S-containing heterocyclic group substituted with one or more hydroxyl, sulfone (eg SO₂-C₁₋₃ alkyl), alkyl (eg C₁₋₃ alkyl such as methyl), amino (eg NH₂), alkoxy (eg -OCH₃) , carboxylate (eg COO-C₁₋₃ alkyl such as COOCH₃) or alkylcarbonyl (eg CO-C₁₋₃ alkyl such as CO-CH₃) groups. Preferably, the heteroatom(s) is/are N.

In some embodiments, where R³, R⁵ and R⁶ are H, R⁴ is preferably other than COOH, C(=O)NHOH, C(=O)NHO-tetrahydropyran or CONH-aryl substituted with NH₂.

In some embodiments, where at least one of R³, R⁴, R⁵ and R⁶ is R⁹, R⁹ is preferably selected from N-, O- and/or S-containing heterocyclic groups optionally substituted with one or more hydroxyl, sulfone (eg SO₂-C₁₋₃ alkyl), alkyl (eg C₁₋₃ alkyl such as methyl), amino (eg NH₂), alkoxy (eg -OCH₃) , carboxylate (eg COO-C₁₋₃ alkyl such as COOCH₃) or alkylcarbonyl (eg CO-C₁₋₃ alkyl such as CO-CH₃) groups, aliphatic and aryl groups comprising one or more carboxamide, sulfoxide, sulfone or sulfonamide groups, and halogenated alkylcarbonyl groups.

In some embodiments, where at least one of R³, R⁴, R⁵ and R⁶ is R⁹, R⁹ is preferably selected from the following:

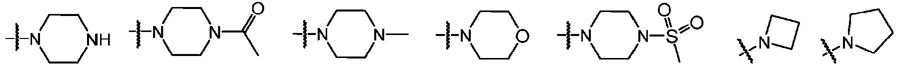

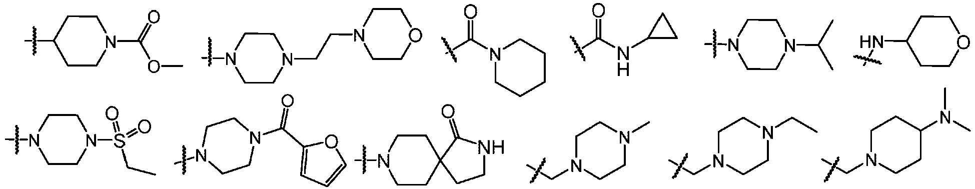

Optionally, the R⁹ substituents shown in the preceding paragraph may further comprise an alkyl bridge (eg a -CH₂- or -CH₂CH₂- bridge), amino bridge (eg -NH- or -NH-CH₂- or -NH-CH₂CH₂- ), amide bridge (eg -C(=O)NH-), alkoxy bridge (eg -O-CH₂- or -O-CH₂CH₂-), ether bridge (eg -O-) or ketone bridge (eg a -C(=O)- bridge) to the carbon atom at position 4/5 of the pyrimidine ring.

Where R³ is R⁹, R⁴, R⁵ and R⁶ are preferably H. Similarly, where R⁴ is R⁹, R³, R⁵ and R⁶ are preferably H. Also, where R⁵ is R⁹, R^{3,} R⁴ and R⁶ are preferably H. And where R⁶ is R⁹, R³, R⁴ and R⁵ are preferably H.

In some embodiments, R³ and R⁶ are H.

In some embodiments, R¹ is selected from halogen (preferably Br or Cl), aryl (eg phenyl), and heterocyclic optionally substituted with one or more groups selected from halogen, CN, OH, O-C₁₋₆ alkyl (eg O-methyl), NH₂, COOH, C₂₋₅ carboxylate (eg COOC(CH₃)₃), CONH₂ and haloalkyl (eg CHF₂ and CF₃) such as, for example, pyridine, pyrazole substituted with CHF₂, and thiophene; R², R³, R⁵ and R⁶ are all H; and R⁴ is selected from the following:

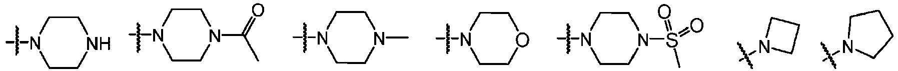

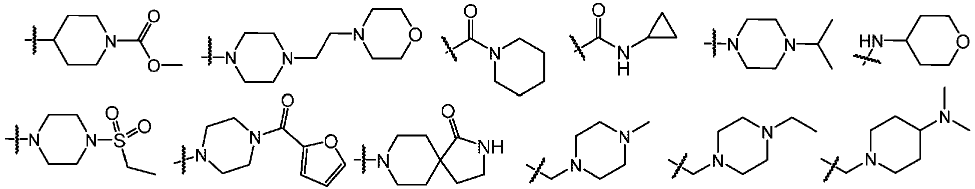

In one especially preferred embodiment, the compound is selected from the group consisting of: *N*-(6-(4-Methylpiperazin-1-yl)pyridin-3-yl)-4-(6-phenylimidazo[1,2-*a*]pyridin-3-yl)pyrimidin-2-amine, *N*-(6-(4-Methylpiperazin-1-yl)pyridin-3-yl)-4-(6-(pyridin-3-yl)imidazo[1,2-a]pyridin-3-yl)pyrimidin-2-amine, *N*-(6-(4-(Methylsulfonyl)piperazin-1-yl)pyridin-3-yl)-4-(6-(pyridin-3-yl)imidazo[1,2-a]pyridin-3-yl)pyrimidin-2-amine, *N*-(6-(4-(Methylsulfonyl)piperazin-1-yl)pyridin-3-yl)-4-(6-(thiophen-2-yl)imidazo [1,2-a]pyridin-3-yl)pyrimidin-2-amine, 4-(6-Chloroimidazo[1,2-a]pyridin-3-yl)-*N*-(6-(4-methylpiperazin-1-yl)pyridin-3-yl)pyrimidin-2-amine, *N*-(6-(Azetidin-1-yl)pyridin-3-yl)-4-(6-phenylimidazo[1,2-a] pyridin-3-yl)pyrimidin-2-amine, 4-(6-Bromoimidazo[1,2-a]pyridin-3-yl)-*N*-(6-(4-isopropylpiperazin-1-yl)pyridin-3-yl)pyrimidin-2-amine, *N*-(6-(4-Isopropylpiperazin-1-yl)pyridin-3-yl)-4-(6-phenylimidazo [1,2-a]pyridin-3-yl)pyrimidin-2-amine, 4-(6-(1-(Difluoromethyl)-1H-pyrazol-4-yl)imidazo[1,2-a]pyridin-3-yl)-*N*-(6-(4-methylpiperazin-1-yl)pyridin-3-yl)pyrimidin-2-amine, and 4-(6-Bromoimidazo[1,2-a] pyridin-3-yl)-*N*-(6-((4-ethylpiperazin-1-yl)methyl)pyridin-3-yl)pyrimidin-2-amine. These compounds are examples of particularly potent inhibitors of at least CDK9, TRKC, DYRK and FLT3.

In some preferred embodiments, the compounds of formula I exhibit anti-proliferative activity in human cell lines, as measured by a standard cytotoxicity assay. Preferably, the compound exhibits an IC₅₀ value of less than 5 µM, even more preferably less than 1 µM as measured by a standard cell viability assay. More preferably still, the compound exhibits an IC₅₀ value of less than 0.5 µM.

In some preferred embodiments, the compounds of formula I inhibit one or more protein kinases, as measured by any standard assay well known to those skilled in the art. Preferably, the compound exhibits an IC₅₀ value of less than 1 µM or less than 0.5 µM as measured by the kinase assay described in Example 2 hereinafter, more preferably still less than 0.1 µM.

Particular examples of compounds according to the first aspect are shown in Table 1 below.

**Table 1 Chemical structure of selected compounds of the present disclosure**

| **No.** | **Structure** | **Name** | **Mass** |
|---|---|---|---|
| **1** | 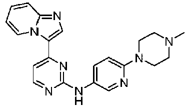 | 4-(Imidazo[1,2-*a*]pyridin-3-yl)-*N*-(6-(4-methylpiperazin-1-yl)pyridin-3-yl)pyrimidin-2-amine | 386.5 |
| **2** | 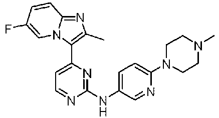 | 4-(6-Fluoro-2-methylimidazo[1,2-*a*]pyridin-3-yl)-*N*-(6-(4-methylpiperazin-1-yl)pyridin-3-yl)pyrimidin-2-amine | 418.5 |
| **3** | 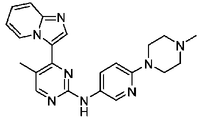 | 4-(Imidazo[1,2-*a*]pyridin-3-yl)-5-methyl-*N*-(6-(4-methylpiperazin-1-yl)pyridin-3-yl)pyrimidin-2-amine | 400.5 |
| **4** | 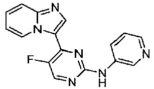 | 5-Fluoro-4-(imidazo[1,2-*a*]pyridin-3-yl)-*N*-(pyridin-3-yl)pyrimidin-2-amine | 306.3 |
| **5** | 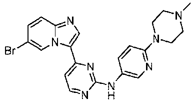 | 4-(6-Bromoimidazo[1,2-*a*]pyridin-3-yl)-*N*-(6-(4-methylpiperazin-1-yl)pyridin-3-yl)pyrimidin-2-amine | 465.4 |
| **6** | 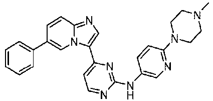 | *N*-(6-(4-Methylpiperazin-1-yl)pyridin-3-yl)-4-(6-phenylimidazo[1,2-*a*]pyridin-3-yl)pyrimidin-2-amine | 462.6 |
| **7** | 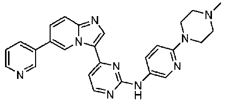 | *N*-(6-(4-Methylpiperazin-1-yl)pyridin-3-yl)-4-(6-(pyridin-3-yl)imidazo[1,2-*a*]pyridin-3-yl)pyrimidin-2-amine | 463.5 |
| **8** | 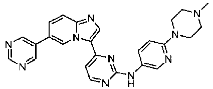 | *N*-(6-(4-Methylpiperazin-1-yl)pyridin-3-yl)-4-(6-(pyrimidin-5-yl)imidazo[1,2-*a*]pyridin-3-yl)pyrimidin-2-amine | 464.5 |
| **9** | 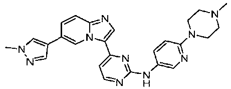 | 4-(6-(1-Methyl-1H-pyrazol-4-yl)imidazo[1,2-*a*]pyridin-3-yl)-*N-*(6-(4-methylpiperazin-1-yl)pyridin-3-yl)pyrimidin-2-amine | 466.6 |
| **10** | 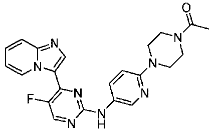 | 1-(4-(5-((5-Fluoro-4-(imidazo[1,2-*a*]pyridin-3-yl)pyrimidin-2-yl)amino)pyridin-2-yl)piperazin-1-yl)ethan-1-one | 432.5 |
| **11** | 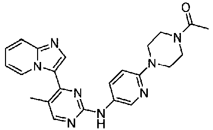 | 1-(4-(5-((4-(Imidazo[1,2-*a*]pyridin-3-yl)-5-methylpyrimidin-2-yl)amino)pyridin-2-yl)piperazin-1-yl)ethan-1-one | 428.5 |
| **12** | 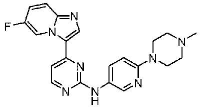 | 4-(6-Fluoroimidazo[1,2-*a*]pyridin-3-yl)-*N*-(6-(4-methylpiperazin-1-yl)pyridin-3-yl)pyrimidin-2-amine | 404.5 |
| **13** | 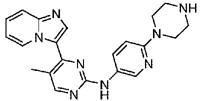 | 4-(Imidazo[1,2-*a*]pyridin-3-yl)-5-methyl-*N*-(6-(piperazin-1-yl)pyridin-3-yl)pyrimidin-2-amine | 386.5 |
| **14** | 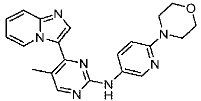 | 4-(Imidazo[1,2-*a*]pyridin-3-yl)-5-methyl-*N*-(6-morpholinopyridin-3-yl)pyrimidin-2-amine | 387.4 |
| **15** | 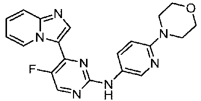 | 5-Fluoro-4-(imidazo[1,2-*a*]pyridin-3-yl)-*N*-(6-morpholinopyridin-3-yl)pyrimidin-2-amine | 391.4 |
| **16** | 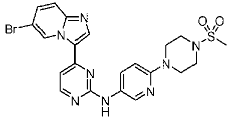 | 4-(6-Bromoimidazo[1,2-*a*]pyridin-3-yl)-*N*-(6-(4-(methylsulfonyl)piperazin-1-yl)pyridin-3-yl)pyrimidin-2-amine | 529.4 |
| **17** | 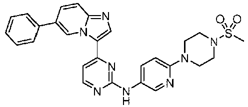 | *N*-(6-(4-(Methylsulfonyl)piperazin-1-yl)pyridin-3-yl)-4-(6-phenylimidazo[1,2-*a*]pyridin-3-yl)pyrimidin-2-amine | 526.6 |
| **18** | 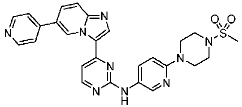 | *N*-(6-(4-(Methylsulfonyl)piperazin-1-yl)pyridin-3-yl)-4-(6-(pyridin-4-yl)imidazo[1,2-*a*]pyridin-3-yl)pyrimidin-2-amine | 527.6 |
| **19** | 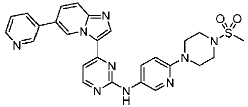 | *N*-(6-(4-(Methylsulfonyl)piperazin-1-yl)pyridin-3-yl)-4-(6-(pyridin-3-yl)imidazo[1,2-*a*]pyridin-3-yl)pyrimidin-2-amine | 527.6 |
| **20** | 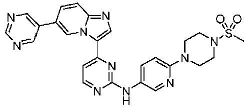 | *N*-(6-(4-(Methylsulfonyl)piperazin-1-yl)pyridin-3-yl)-4-(6-(pyrimidin-5-yl)imidazo[1,2-*a*]pyridin-3-yl)pyrimidin-2-amine | 528.6 |
| **21** | 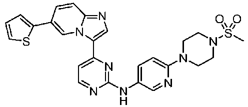 | *N*-(6-(4-(Methylsulfonyl)piperazin-1-yl)pyridin-3-yl)-4-(6-(thiophen-2-yl)imidazo[1,2-*a*]pyridin-3-yl)pyrimidin-2-amine | 532.6 |
| **22** | 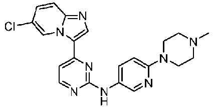 | 4-(6-Chloroimidazo[1,2-*a*]pyridin-3-yl)-*N*-(6-(4-methylpiperazin-1-yl)pyridin-3-yl)pyrimidin-2-amine | 420.9 |
| **23** | 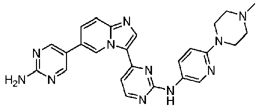 | 4-(6-(2-Aminopyrimidin-5-yl)imidazo[1,2-*a*]pyridin-3-yl)-*N*-(6-(4-methylpiperazin-1-yl)pyridin-3-yl)pyrimidin-2-amine | 479.6 |
| **24** | 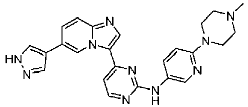 | 4-(6-(1*H*-Pyrazol-4-yl)imidazo[1,2-*a*]pyridin-3-yl)-*N*-(6-(4-methylpiperazin-1-yl)pyridin-3-yl)pyrimidin-2-amine | 452.5 |
| **25** | 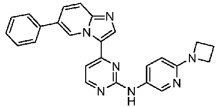 | *N*-(6-(Azetidin-1-yl)pyridin-3-yl)-4-(6-phenylimidazo[1,2-*a*]pyridin-3-yl)pyrimidin-2-amine | 419.5 |
| **26** | 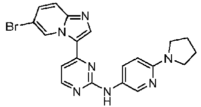 | 4-(6-Bromoimidazo[1,2-*a*]pyridin-3-yl)-*N*-(6-(pyrrolidin-1-yl)pyridin-3-yl)pyrimidin-2-amine | 436.3 |
| **27** | 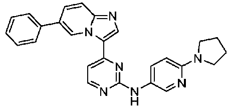 | 4-(6-Phenylimidazo[1,2-*a*]pyridin-3-yl)-*N*-(6-(pyrrolidin-1-yl)pyridin-3-yl)pyrimidin-2-amine | 433.5 |
| **28** | 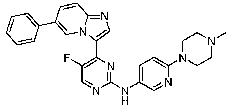 | 5-Fluoro-*N*-(6-(4-methylpiperazin-1-yl)pyridin-3-yl)-4-(6-phenylimidazo[1,2-*a*]pyridin-3-yl)pyrimidin-2-amine | 480.6 |
| **29** | 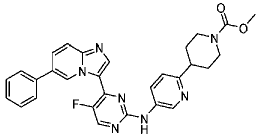 | Methyl4-(5-((5-fluoro-4-(6-phenylimidazo[1,2-*a*]pyridin-3-yl)pyrimidin-2-yl)amino)pyridin-2-yl)piperidine-1-carboxylate | 523.6 |
| **30** | 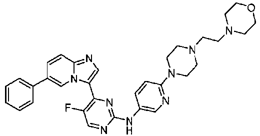 | 5-Fluoro-N-(6-(4-(2-morpholinoethyl)piperazin-1-yl)pyridin-3-yl)-4-(6-phenylimidazo[1,2-*a*]pyridin-3-yl)pyrimidin-2-amine | 579.7 |
| **31** | 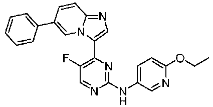 | *N*-(6-Ethoxypyridin-3-yl)-5-fluoro-4-(6-phenylimidazo[1,2-*a*]pyridin-3-yl)pyrimidin-2-amine | 426.5 |
| **32** | 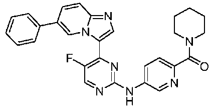 | (5-((5-Fluoro-4-(6-phenylimidazo[1,2-*a*]pyridin-3-yl)pyrimidin-2-yl)amino)pyridin-2-yl)(piperidin-1-yl)methanone | 493.5 |
| **33** | 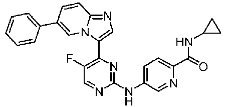 | *N*-Cyclopropyl-5-((5-fluoro-4-(6-phenylimidazo[1,2-*a*]pyridin-3-yl)pyrimidin-2-yl)amino)picolinamide | 465.5 |
| **34** | 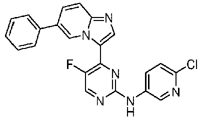 | *N*-(6-Chloropyridin-3-yl)-5-fluoro-4-(6-phenylimidazo[1,2-*a*]pyridin-3-yl)pyrimidin-2-amine | 416.8 |
| **35** | 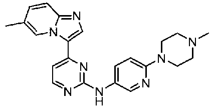 | 4-(6-Methylimidazo[1,2-*a*]pyridin-3-yl)-*N*-(6-(4-methylpiperazin-1-yl)pyridin-3-yl)pyrimidin-2-amine | 400.5 |
| **36** | 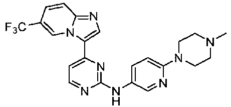 | *N*-(6-(4-Methylpiperazin-1-yl)pyridin-3-yl)-4-(6-(trifluoromethyl)imidazo[1,2-*a*]pyridin-3-yl)pyrimidin-2-amine | 454.5 |
| **37** | 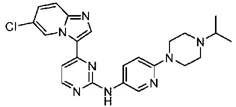 | 4-(6-Chloroimidazo[1,2-*a*]pyridin-3-yl)-*N*-(6-(4-isopropylpiperazin-1-yl)pyridin-3-yl)pyrimidin-2-amine | 449.0 |
| **38** | 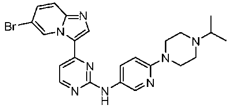 | 4-(6-Bromoimidazo[1,2-*a*]pyridin-3-yl)-*N*-(6-(4-isopropylpiperazin-1-yl)pyridin-3-yl)pyrimidin-2-amine | 493.4 |
| **39** | 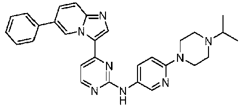 | *N*-(6-(4-Isopropylpiperazin-1-yl)pyridin-3-yl)-4-(6-phenylimidazo[1,2-*a*]pyridin-3-yl)pyrimidin-2-amine | 490.6 |
| **40** | 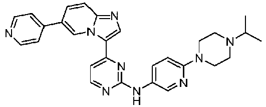 | *N*-(6-(4-Isopropylpiperazin-1-yl)pyridin-3-yl)-4-(6-(pyridin-4-yl)imidazo[1,2-*a*]pyridin-3-yl)pyrimidin-2-amine | 491.6 |
| **41** | 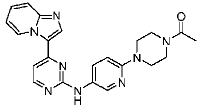 | 1-(4-(5-((4-(Imidazo[1,2-*a*]pyridin-3-yl)pyrimidin-2-yl)amino)pyridin-2-yl)piperazin-1-yl)ethan-1-one | 414.5 |
| **42** | 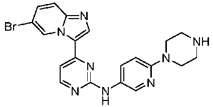 | 4-(6-Bromoimidazo[1,2-*a*]pyridin-3-yl)-*N*-(6-(piperazin-1-yl)pyridin-3-yl)pyrimidin-2-amine | 451.3 |
| **43** | 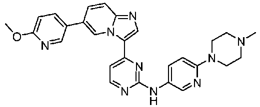 | 4-(6-(6-Methoxypyridin-3-yl)imidazo[1,2-*a*]pyridin-3-yl)-*N-*(6-(4-methylpiperazin-1-yl)pyridin-3-yl)pyrimidin-2-amine | 493.6 |
| 44 | 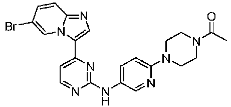 | 1-(4-(5-((4-(6-Bromoimidazo[1,2-*a*]pyridin-3-yl)pyrimidin-2-yl)amino)pyridin-2-yl)piperazin-1-yl)ethan-1-one | 493.4 |
| **45** | 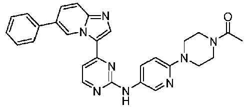 | 1-(4-(5-((4-(6-Phenylimidazo[1,2-*a*]pyridin-3-yl)pyrimidin-2-yl)amino)pyridin-2-yl)piperazin-1-yl)ethan-1-one | 490.6 |
| **46** | 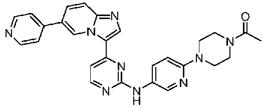 | 1-(4-(5-((4-(6-(Pyridin-4-yl)imidazo[1,2-*a*]pyridin-3-yl)pyrimidin-2-yl)amino)pyridin-2-yl)piperazin-1-yl)ethan-1-one | 491.6 |
| **47** | 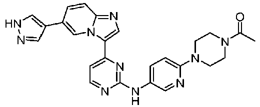 | 1-(4-(5-((4-(6-(1*H*-Pyrazol-4-yl)imidazo[1,2-*a*]pyridin-3-yl)pyrimidin-2-yl)amino)pyridin-2-yl)piperazin-1-yl)ethan-1-one | 480.5 |
| **48** | 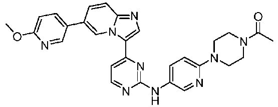 | 1-(4-(5-((4-(6-(6-Methoxypyridin-3-yl)imidazo[1,2-*a*]pyridin-3-yl)pyrimidin-2-yl)amino)pyridin-2-yl)piperazin-1-yl)ethan-1-one | 521.6 |
| **49** | 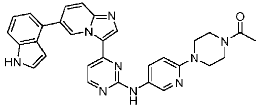 | 1-(4-(5-((4-(6-(1*H*-Indol-4-yl)imidazo[1,2-*a*]pyridin-3-yl)pyrimidin-2-yl)amino)pyridin-2-yl)piperazin-1-yl)ethan-1-one | 529.6 |
| **50** | 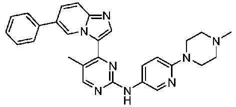 | 5-Methyl-*N*-(6-(4-methylpiperazin-1-yl)pyridin-3-yl)-4-(6-phenylimidazo[1,2-*a*]pyridin-3-yl)pyrimidin-2-amine | 476.6 |
| **51** | 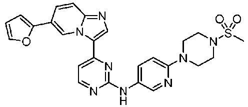 | 4-(6-(Furan-2-yl)imidazo[1,2-*a*]pyridin-3-yl)-*N*-(6-(4-(methylsulfonyl)piperazin-1-yl)pyridin-3-yl)pyrimidin-2-amine | 516.6 |
| **52** | 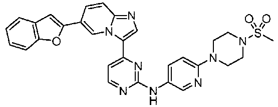 | 4-(6-(Benzofuran-2-yl)imidazo[1,2-*a*]pyridin-3-yl)-*N*-(6-(4-(methylsulfonyl)piperazin-1-yl)pyridin-3-yl)pyrimidin-2-amine | 566.6 |
| **53** | 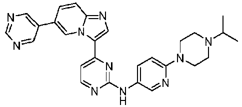 | *N*-(6-(4-Isopropylpiperazin-1-yl)pyridin-3-yl)-4-(6-(pyrimidin-5-yl)imidazo[1,2-*a*]pyridin-3-yl)pyrimidin-2-amine | 492.6 |
| **54** | 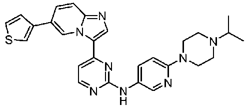 | *N*-(6-(4-Isopropylpiperazin-1-yl)pyridin-3-yl)-4-(6-(thiophen-3-yl)imidazo[1,2-*a*]pyridin-3-yl)pyrimidin-2-amine | 496.6 |
| **55** | 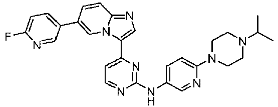 | 4-(6-(6-Fluoropyridin-3-yl)imidazo[1,2-*a*]pyridin-3-yl)-*N*-(6-(4-isopropylpiperazin-1-yl)pyridin-3-yl)pyrimidin-2-amine | 509.6 |
| **56** | 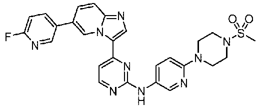 | 4-(6-(6-Fluoropyridin-3-yl)imidazo[1,2-*a*]pyridin-3-yl)-*N*-(6-(4-(methylsulfonyl)piperazin-1-yl)pyridin-3-yl)pyrimidin-2-amine | 545.6 |
| **57** | 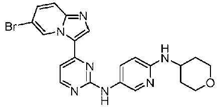 | *N*⁵-(4-(6-Bromoimidazo[1,2-*a*]pyridin-3-yl)pyrimidin-2-yl)-*N*²-(tetrahydro-2H-pyran-4-yl)pyridine-2,5-diamine | 466.3 |
| **58** | 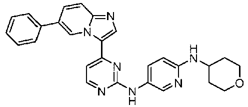 | *N*⁵-(4-(6-Phenylimidazo[1,2-*a*]pyridin-3-yl)pyrimidin-2-yl)-*N*²-(tetrahydro-2*H*-pyran-4-yl)pyridine-2,5-diamine | 463.5 |
| **59** | 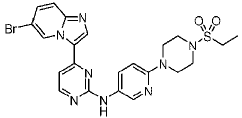 | 4-(6-Bromoimidazo[1,2-*a*]pyridin-3-yl)-*N*-(6-(4-(ethylsulfonyl)piperazin-1-yl)pyridin-3-yl)pyrimidin-2-amine | 543.4 |
| **60** | 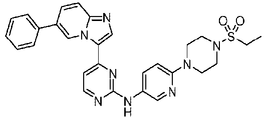 | *N*-(6-(4-(Ethylsulfonyl)piperazin-1-yl)pyridin-3-yl)-4-(6-phenylimidazo[1,2-*a*]pyridin-3-yl)pyrimidin-2-amine | 540.6 |
| **61** | 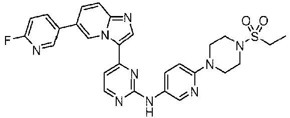 | *N*-(6-(4-(Ethylsulfonyl)piperazin-1-yl)pyridin-3-yl)-4-(6-(6-fluoropyridin-3-yl)imidazo[1,2-*a*]pyridin-3-yl)pyrimidin-2-amine | 559.6 |
| **62** | 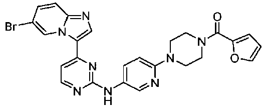 | (4-(5-((4-(6-Bromoimidazo[1,2-*a*]pyridin-3-yl)pyrimidin-2-yl)amino)pyridin-2-yl)piperazin-1-yl)(furan-2-yl)methanone | 545.4 |
| **63** | 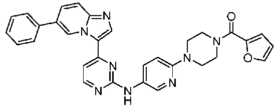 | Furan-2-yl(4-(5-((4-(6-phenylimidazo[1,2-*a*]pyridin-3-yl)pyrimidin-2-yl)amino)pyridin-2-yl)piperazin-1-yl)methanone | 542.6 |
| **64** | 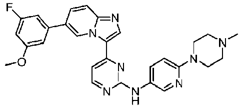 | 4-(6-(3-Fluoro-5-methoxyphenyl)imidazo[1,2-*a*]pyridin-3-yl)-*N-*(6-(4-methylpiperazin-1-yl)pyridin-3-yl)pyrimidin-2-amine | 510.6 |
| **65** | 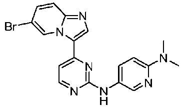 | *N*⁵-(4-(6-Bromoimidazo[1,2-*a*]pyridin-3-yl)pyrimidin-2-yl)-*N*²,*N*²-dimethylpyridine-2,5-diamine | 410.3 |
| **66** | 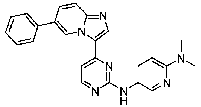 | *N*²,*N*²-Dimethyl-*N*⁵-(4-(6-phenylimidazo[1,2-*a*]pyridin-3-yl)pyrimidin-2-yl)pyridine-2,5-diamine | 407.5 |
| **67** | 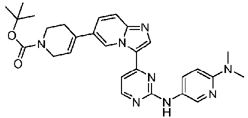 | *Tert*-butyl 4-(3-(2-((6-(dimethylamino)pyridin-3-yl)amino)pyrimidin-4-yl)imidazo[1,2-*a*]pyridin-6-yl)-3,6-dihydropyridine-1(2*H*)-carboxylate | 512.6 |
| **68** | 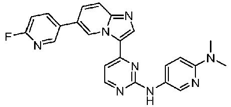 | *N*⁵-(4-(6-(6-Fluoropyridin-3-yl)imidazo[1,2-*a*]pyridin-3-yl)pyrimidin-2-yl)-*N*²,*N*²-dimethylpyridine-2,5-diamine | 426.5 |
| **69** | 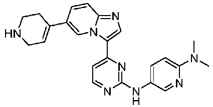 | *N*²,*N*²-Dimethyl-*N*⁵-(4-(6-(1,2,3,6-tetrahydropyridin-4-yl)imidazo[1,2-*a*]pyridin-3-yl)pyrimidin-2-yl)pyridine-2,5-diamine | 412.5 |
| **70** | 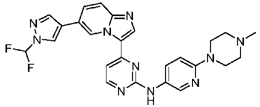 | 4-(6-(1-(Difluoromethyl)-1*H*-pyrazol-4-yl)imidazo[1,2-*a*]pyridin-3-yl)-N-(6-(4-methylpiperazin-1-yl)pyridin-3-yl)pyrimidin-2-amine | 502.5 |
| **71** | 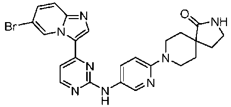 | 8-(5-((4-(6-Bromoimidazo[1,2-*a*]pyridin-3-yl)pyrimidin-2-yl)amino)pyridin-2-yl)-2,8-diazaspiro[4.5]decan-1-one | 519.4 |
| **72** | 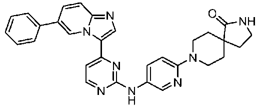 | 8-(5-((4-(6-Phenylimidazo[1,2-*a*]pyridin-3-yl)pyrimidin-2-yl)amino)pyridin-2-yl)-2,8-diazaspiro[4.5]decan-1-one | 516.6 |
| **73** | 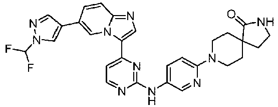 | 8-(5-((4-(6-(1-(Difluoromethyl)-1*H*-pyrazol-4-yl)imidazo[1,2-*a*]pyridin-3-yl)pyrimidin-2-yl)amino)pyridin-2-yl)-2,8-diazaspiro[4.5]decan-1-one | 556.6 |
| **74** | 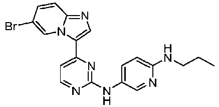 | *N*⁵-(4-(6-Bromoimidazo[1,2-*a*]pyridin-3-yl)pyrimidin-2-yl)-*N*²-propylpyridine-2,5-diamine | 424.3 |
| **74** | 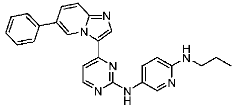 | *N*⁵-(4-(6-Phenylimidazo[1,2-*a*]pyridin-3-yl)pyrimidin-2-yl)-*N*²-propylpyridine-2,5-diamine | 421.5 |
| **76** | 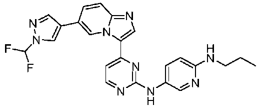 | *N*⁵-(4-(6-(1-(Difluoromethyl)-1*H*-pyrazol-4-yl)imidazo[1,2-*a*]pyridin-3-yl)pyrimidin-2-yl)-*N*²-propylpyridine-2,5-diamine | 461.5 |
| **77** | 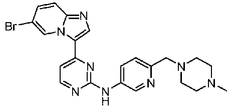 | 4-(6-Bromoimidazo[1,2-*a*]pyridin-3-yl)-*N*-(6-((4-methylpiperazin-1-yl)methyl)pyridin-3-yl)pyrimidin-2-amine | 479.4 |
| **78** | 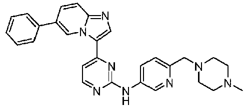 | *N*-(6-((4-Methylpiperazin-1-yl)methyl)pyridin-3-yl)-4-(6-phenylimidazo[1,2-*a*]pyridin-3-yl)pyrimidin-2-amine | 476.6 |
| **79** | 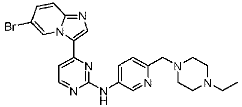 | 4-(6-Bromoimidazo[1,2-*a*]pyridin-3-yl)-*N*-(6-((4-ethylpiperazin-1-yl)methyl)pyridin-3-yl)pyrimidin-2-amine | 493.4 |
| **80** | 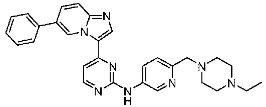 | *N*-(6-((4-Ethylpiperazin-1-yl)methyl)pyridin-3-yl)-4-(6-phenylimidazo[1,2-*a*]pyridin-3-yl)pyrimidin-2-amine | 490.6 |
| **81** | 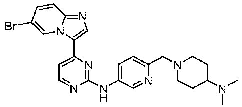 | 4-(6-Bromoimidazo[1,2-*a*]pyridin-3-yl)-*N*-(6-((4-(dimethylamino)piperidin-1-yl)methyl)pyridin-3-yl)pyrimidin-2-amine | 507.4 |
| **82** | 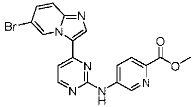 | Methyl 5-((4-(6-bromoimidazo[1,2-*a*]pyridin-3-yl)pyrimidin-2-yl)amino)picolinate | 425.2 |

The compounds (and pharmaceutically acceptable salts, solvates and prodrugs thereof) may be administered in combination with one or more additional agent(s) for the treatment of cancer or another proliferative disease or condition. For example, the compounds may be used in combination with other anti-cancer agents in order to inhibit more than one cancer signalling pathway simultaneously so as to make cancer cells more susceptible to anti-cancer therapies (eg treatments with other anti-cancer agents, chemotherapy, radiotherapy or a combination thereof). As such, the compounds of formula I may be used in combination with one or more of the following categories of anti-cancer agents:
- other anti-proliferative/antineoplastic drugs and combinations thereof, as used in medical oncology, such as alkylating agents (eg cis-platin, oxaliplatin, carboplatin, cyclophosphamide, nitrogen mustard, melphalan, chlorambucil, busulphan, temozolamide and nitrosoureas); antimetabolites (eg gemcitabine and antifolates such as fluoropyrimidines like 5-fluorouracil and tegafur, raltitrexed, methotrexate, cytosine arabinoside, fludarabine and hydroxyurea); antitumour antibiotics (eg anthracyclines such as adriamycin, bleomycin, doxorubicin, daunomycin, epirubicin, idarubicin, mitomycin-C, dactinomycin and mithramycin); antimitotic agents (eg vinca alkaloids such as vincristine, vinblastine, vindesine and vinorelbine and taxoids including taxol and taxotere and polokinase inhibitors); and topoisomerase inhibitors (eg epipodophyllotoxins such as etoposide and teniposide, amsacrine, topotecan and camptothecin);
- cytostatic agents such as antioestrogens (eg tamoxifen, fulvestrant, toremifene, raloxifene, droloxifene and iodoxyfene), antiandrogens (eg bicalutamide, flutamide, nilutamide and cyproterone acetate), LHRH antagonists or LHRH agonists (eg goserelin, leuprorelin and buserelin), progestogens (eg megestrol acetate), aromatase inhibitors (eg as anastrozole, letrozole, vorazole and exemestane) and inhibitors of 5α-reductase such as finasteride;
- anti-invasion agents (eg c-Src kinase family inhibitors such as 4-(6-chloro-2,3-methylene dioxyanilino)-7-[2-(4-methylpiperazin-1-yl)ethoxy]-5-tetrahydropyran-4-yloxyquinazoline (AZD0530; International Patent Publication No WO 01/94341), N-(2-chloro-6-methylphenyl)-2-{6-[4-(2-hydroxyethyl)piperazin-1-yl]-2-methylpyrimidin-4-ylamino}thiazole-5-carboxamide (dasatinib) and bosutinib (SKI-606)), and metalloproteinase inhibitors including marimastat, inhibitors of urokinase plasminogen activator receptor function or antibodies to heparanase;
- inhibitors of growth factor function (eg growth factor antibodies and growth factor receptor antibodies such as the anti-erbB2 antibody trastuzumab (Herceptin^{™}), the anti-EGFR antibody panitumumab, the anti-erbB1 antibody cetuximab (Erbitux, C225) and any growth factor or growth factor receptor antibodies disclosed by Stern et al., Crit Rev Oncol Hematol 54:11-29, 2005). Such inhibitors also include tyrosine kinase inhibitors such as inhibitors of the epidermal growth factor family (eg EGFR family tyrosine kinase inhibitors such as N-(3-chloro-4-fluoro phenyl)-7-methoxy-6-(3-morpholinopropoxy)quinazolin-4-amine (gefitinib, ZD1839), N-(3-ethynylphenyl)-6,7-bis(2-methoxyethoxy)quinazolin-4-amine (erlotinib, OSI-774) and 6-acryl amido-N-(3-chloro-4-fluorophenyl)-7-(3-morpholinopropoxy)-quinazolin-4-amine (CI 1033), erbB2 tyrosine kinase inhibitors such as lapatinib); inhibitors of the hepatocyte growth factor family; inhibitors of the insulin growth factor family; inhibitors of the platelet-derived growth factor family such as imatinib and/or nilotinib (AMN107); inhibitors of serine/threonine kinases (eg Ras/Raf signalling inhibitors such as farnesyl transferase inhibitors including sorafenib (BAY 43-9006), tipifarnib (R115777) and lonafarnib (SCH66336)), inhibitors of cell signalling through MEK and/or AKT kinases, c-kit inhibitors, abl kinase inhibitors, PI3 kinase inhibitors, Plt3 kinase inhibitors, CSF-1R kinase inhibitors, IGF receptor (insulin-like growth factor) kinase inhibitors; aurora kinase inhibitors (eg AZD1152, PH739358, VX-680, MLN8054, R763, MP235, MP529, VX-528 and AX39459) and other cyclin dependent kinase (CDK) inhibitors;
- antiangiogenic agents such as those which inhibit the effects of vascular endothelial growth factor (eg the anti-vascular endothelial cell growth factor antibody bevacizumab (Avastin^{™}) and VEGF receptor tyrosine kinase inhibitors such as vandetanib (ZD6474), vatalanib (PTK787), sunitinib (SU11248), axitinib (AG-013736), pazopanib (GW 786034) and 4-(4-fluoro-2-methylindol-5-yloxy)-6-methoxy-7-(3-pyrrolidin-1-ylpropoxy)quinazoline (AZD2171; Example 240 within International Patent Publication No WO 00/47212), compounds such as those disclosed in International Patent Publication Nos WO97/22596, WO 97/30035, WO 97/32856 and WO 98/13354, and compounds that work by other mechanisms (eg linomide, inhibitors of integrin αvβ3 function and angiostatin);
- vascular damaging agents such as Combretastatin A4 and compounds disclosed in International Patent Publication Nos WO 99/02166, WO 00/40529, WO 00/41669, WO 01/92224, WO 02/04434 and WO 02/08213;
- endothelin receptor antagonists such as zibotentan (ZD4054) or atrasentan;
- antisense therapies such as those which are directed to the targets listed above, such as ISIS 2503, an anti-ras antisense;
- gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
- immunotherapy approaches, including for example *ex vivo* and *in vivo* approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Where used in combination with other anti-cancer agents, a compound of formula I and the other anti-cancer agent can be administered in the same pharmaceutical composition or in separate pharmaceutical compositions. If administered in separate pharmaceutical compositions, the compound and the other anti-cancer agent may be administered simultaneously or sequentially in any order (eg within seconds or minutes or even hours (eg 2 to 48 hours)).

The compound of the formula I is typically applied to the treatment of cancer or another proliferative cell disease or condition in a human subject. However, the subject may also be selected from, for example, livestock animals (eg cows, horses, pigs, sheep and goats), companion animals (eg dogs and cats) and exotic animals (eg non-human primates, tigers, elephants etc).

Cancers and other proliferative cell diseases and conditions that may be treated in accordance with the present disclosure include biliary tract cancer, brain cancer and other cancers of the central nervous system (CNS) (including glioblastomas and medulloblastomas), neuroblastomas, breast cancer, cervical cancer, ovarian cancer (including those arising from epithelial cells, stromal cells, germ cells, and mesenchymal cells), choriocarcinoma, colorectal cancer, endometrial cancer, liver cancer, lung cancer, oesophageal cancer, gastric cancer, haematological neoplasms (including acute lymphocytic leukemia (ALL)), chronic lymphocytic leukemia (CLL) and chronic myelogenous leukemia (CML), and acute myeloid leukaemia (AML), multiple myeloma, AIDS-associated leukemias and adult T-cell leukemia lymphoma, lymphomas (including Non-Hodgkin's lymphoma, Hodgkin's disease and lymphocytic lymphomas)), intraepithelial neoplasms (including Bowen's disease and Paget's disease), oral cancer (including squamous cell carcinoma), pancreatic cancer, prostate cancer, sarcomas (including leiomyosarcoma, rhabdomyosarcoma, liposarcoma, fibrosarcoma, and osteosarcoma), skin cancer (including melanoma, Kaposi's sarcoma, basocellular cancer, and squamous cell cancer), testicular cancer (including germinal tumours such as seminoma, non-seminoma teratomas, and choriocarcinomas), stromal tumours, germ cell tumours, thyroid cancer (including thyroid adenocarcinoma and medullar carcinoma), and renal cancer (including adenocarcinoma and Wilms' tumour).

In some embodiments, the compounds of formula I are used to treat cancer or another proliferative cell disease or condition selected from those characterised by over-activated (eg through over-expression) of CDKs, especially one or more of CDK2, CDK4, CDK6 and CDK9, and/or their respective cyclins.

In some embodiments, the compounds of formula I are used to treat cancer or another proliferative cell disease or condition selected from those characterised by over-expression of CDK9 and/or cyclin T1 including, for example, several B and T-cell lymphomas (Bellan et al., J Pathol 203:946-952 (2004)), as well as in neuroblastoma (De Falcao et al., Cancer Biol Ther 4:277-281 (2005), primary neuroectodermal tumour, rhabdomyosarcoma (Simone et al., Cell Death Differ 14:192-195 (2007)) and prostate cancer (Lee et al., J Biol Chem 276:9978-9984 (2001). CDK9 and/or cyclin T1 over-expression may be determined by, for example, assessing the amount of mRNA encoding CDK9 and/or cyclin T1 in a suitable sample using any of the techniques well known to those skilled in the art (eg quantitative amplification techniques such as qPCR).

In some embodiments, the compounds of formula I are used to treat a NTRK fusion-positive cancer or condition (Cocco E. *et al.,* 2018 *supra*)*.*

In some embodiments, the compounds of formula I are used to treat disorders associated with DYRKs including cancers and/or Alzheimer's disease (Hu, J. et al., Int J Cancer 132(10):2258-2269, 2013; Soppa U. *et al.,* 2014 *supra*) which includes microglial proliferation (Gomez-Nicola D et al., Methods Mol Biol 1303:185-193, 2016).

In some embodiments, the compounds of formula I are used to treat cancer or another disease or condition characterised by expression or over-expression of FLT3 or FLT3-ITD including, for example, several haematologic malignancies (Stirewalt DL and JP Radich, Nat Rev Cancer 3:650-665 (2003) such as AML and other FLT3-activated cancers.

The compounds of formula I may be formulated into a pharmaceutical composition with a pharmaceutically acceptable carrier, diluent and/or excipient. Examples of suitable carriers and diluents are well known to those skilled in the art, and are described in, for example, Remington's Pharmaceutical Sciences, Mack Publishing Co., Easton, PA 1995. Examples of suitable excipients for the various different forms of pharmaceutical compositions described herein may be found in the Handbook of Pharmaceutical Excipients, 2nd Edition, (1994), Edited by A Wade and PJ Weller. Examples of suitable carriers include lactose, starch, glucose, methyl cellulose, magnesium stearate, mannitol, sorbitol and the like. Examples of suitable diluents include ethanol, glycerol and water. The choice of carrier, diluent and/or excipient may be made with regard to the intended route of administration and standard pharmaceutical practice.

A pharmaceutical composition comprising a compound of formula I may further comprise any suitable binders, lubricants, suspending agents, coating agents and solubilising agents. Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilising agents, dyes and even flavouring agents may be provided in the pharmaceutical composition. Examples of preservatives include sodium benzoate, sorbic acid and esters of p-hydroxybenzoic acid. Anti-oxidants and suspending agents may be also used.

A pharmaceutical composition comprising a compound of formula I may be adapted for oral, rectal, vaginal, parenteral, intramuscular, intraperitoneal, intraarterial, intrathecal, intrabronchial, subcutaneous, intradermal, intravenous, nasal, buccal or sublingual routes of administration. For oral administration, particular use may be made of compressed tablets, pills, tablets, gellules, drops, and capsules. For other forms of administration, a pharmaceutical composition may comprise solutions or emulsions which may be injected intravenously, intraarterially, intrathecally, subcutaneously, intradermally, intraperitoneally or intramuscularly, and which are prepared from sterile or sterilisable solutions. A pharmaceutical composition comprising a compound of formula I may also be in form of suppositories, pessaries, suspensions, emulsions, lotions, ointments, creams, gels, sprays, solutions or dusting powders. A pharmaceutical composition may be formulated in unit dosage form (ie in the form of discrete portions containing a unit dose, or a multiple or sub-unit of a unit dose).

The compounds of formula I may be provided as a pharmaceutically acceptable salt including, for example, suitable acid addition or base salts thereof. A review of suitable pharmaceutical salts may be found in Berge et al., J Pharm Sci 66:1-19 (1977). Salts are formed, for example with strong inorganic acids such as mineral acids (eg sulfuric acid, phosphoric acid or hydrohalic acids), with strong organic carboxylic acids, such as alkanecarboxylic acids of 1 to 4 carbon atoms which are unsubstituted or substituted (eg by halogen), such as acetic acid, with saturated or unsaturated dicarboxylic acids (eg oxalic, malonic, succinic, maleic, fumaric, phthalic or tetraphthalic acid), with hydroxycarboxylic acids (eg ascorbic, glycolic, lactic, malic, tartaric or citric acid), with amino acids (eg aspartic or glutamic acid), with benzoic acid, or with organic sulfonic acids (eg (C₁-C₄)-alkyl- or aryl-sulfonic acids which are unsubstituted or substituted by, for example, halogen) such as methane- or p-toluene sulfonic acid).

The compounds of formula I may be provided in their various crystalline forms, polymorphic forms and (an)hydrous forms. In this regard, it is well known to those skilled in the art that chemical compounds may be isolated in any of such forms by slightly varying the method of purification and or isolation from the solvents used in the synthetic preparation of such compounds.

The present disclosure further provides a method of synthesising a compound according to formula I, or a pharmaceutically acceptable salt, solvate thereof.

With regard to the description of the synthetic methods described below and in the referenced synthetic methods that are used to prepare starting materials, it will be understood by those skilled in the art that all proposed reaction conditions, including choice of solvent, reaction atmosphere, reaction temperature, duration of the experiment and workup procedures, can be readily selected. Moreover, it will be understood by those skilled in the art that the functionality present on various portions of the molecule must be compatible with the reagents and reaction conditions utilised.

Necessary starting materials may be obtained by standard procedures of organic chemistry. The preparation of such starting materials is described in conjunction with the following representative process variants and within the examples hereinafter. Alternatively, necessary starting materials may be obtainable by analogous procedures to those illustrated which are within the ordinary skill of those skilled in the art. Further, it will be appreciated that during the synthesis of the compounds, in the processes described below, or during the synthesis of certain starting materials, it may be desirable to protect certain substituent groups to prevent their undesired reaction. Those skilled in the art will readily recognise when such protection is required, and how such protecting groups may be put in place, and later removed. Examples of protecting groups are described in, for example, Protective Groups in Organic Synthesis by Theodora Green (publisher: John Wiley & Sons). Protecting groups may be removed by any convenient method well known to those skilled in the art as appropriate for the removal of the protecting group in question, such methods being chosen so as to effect removal of the protecting group with the minimum disturbance of groups elsewhere in the molecule. Thus, if reactants include, for example, groups such as amino, carboxyl or hydroxyl, it may be desirable to protect the group in some of the reactions mentioned herein.

The compounds of formula I may be prepared by, for example, the general synthetic methodologies described in International Patent Publication No WO2013156780.

In a further aspect of the present disclosure, a method of synthesising a compound of formula I (or a pharmaceutically acceptable salt, solvate thereof) is provided wherein the method comprises:
a) reacting a compound of formula A: 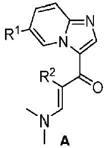
   wherein R¹ and R² are as defined above in respect of formula I, with a suitable pyridinylguanidine derivative;
   or, if necessary, reacting a compound of formula B 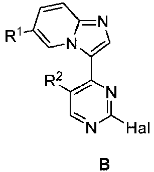
   wherein Hal is F, Cl, Br or I; and R¹ and R² are as defined above in respect of formula I, with a suitable pyridine-3-amine derivative;
   and if necessary
b) removing any protecting groups present, and/or forming a pharmaceutically acceptable salt, solvate thereof.

The coupling reaction between the compound of formula A or formula B and the pyridinylguanidine or pyridin-3-amine derivative may take place in the presence of a suitable solvent or solvent mixture. Those skilled in the art will be able to readily select a suitable solvent or solvent mixture for use in this reaction. Examples of suitable solvents include alcohols, acetonitrile, halogenated solvents, etc.

In addition, those skilled in the art will be able to select appropriate reaction conditions to use in the coupling reaction of the compound of formula A or formula B. However, typically, the reaction will be carried out in anhydrous conditions and in the presence of an inert atmosphere, such as argon or nitrogen. The reaction may also be carried out an elevated temperature, such as, for example, within the range of 80 to 180°C for a suitable time period of, for example, 20 minutes to 48 hours. Suitably, the reaction is carried out under microwave heating, for example, at 80 to 180 °C for 20 minutes to 1.5 hour.

The resultant compound can be isolated and purified using techniques well known to those skilled in the art.

The method of synthesising a compound of formula I (or a pharmaceutically acceptable salt, solvate thereof) may further comprise:
c) subjecting the compound of formula I to a salt exchange (particularly in situations where the compound is formed as a mixture of different salt forms).

The salt exchange may comprise immobilising the compound on a suitable solid support or resin, and eluting the compound with an appropriate acid to yield salt of the compound of formula I.

An example of a particularly suitable method for synthesising a compound of the present disclosure is shown as Scheme 1 below. 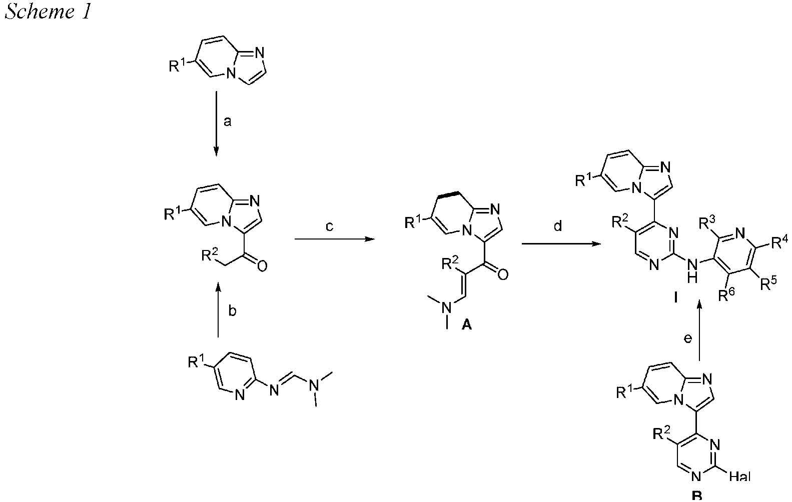 wherein the general reaction conditions are: (a) acetyl anhydride or propionic anhydride, AlCl₃, DCM, 0 °C to reflux, o/n; (b) NaI, chloroacetone, DMF, 80 °C, o/n; (c) DMF-DMA, reflux, o/n; (d) pyridinylguanidines, NaOH, 2-methoxyethanol, microwave, 160-200 °C, 1 h; (e) pyridin-3-amines, Cs₂CO₃, Pd₂(dba)₃ or Pd(OAc)₂, xantphos or BINAP, 1,4-dioxane, microwave, 160-200 °C, 30-60 min.

The disclosure is hereinafter described with reference to the following, non-limiting examples and accompanying figures.

### EXAMPLES

### Example 1 Synthesis

### General

¹H and ¹³C NMR spectra were recorded at 298 K (unless otherwise specified) on a Bruker AVANCE III HD 500 spectrometer (¹H at 500.20 MHz and ¹³C at 125.79 MHz), and were analysed using Bruker Topspin 3.2 software. ¹H NMR signals are reported with chemical shift values *δ* (ppm), multiplicity (s = singlet, d = doublet, t = triplet, q = quartet, dd = doublet of doublets, dt = doublet of triplets, td = triplet of doublets, ddd = doublet of doublet of doublets, m = multiplet and br = broad), relative integral, coupling constants *J* (Hz) and assignments. High resolution mass spectra were recorded on an AB SCIEX TripleTOF 5600 mass spectrometer (Concord, ON, Canada), and ionisation of all samples was carried out using ESI.

*General synthetic procedure A:* To DMF-DMA (5.00-10.00 eq.) was added an ethanone (1.00 eq.). The reaction mixture was heated at reflux overnight, cooled down to room temperature and filtered to give the desired enaminone after washing with Et₂O.

Example: (E)-1-(6-Bromoimidazo[1,2-a]pyridin-3-yl)-3-(dimethylamino)prop-2-en-1-one. 1-(6-Bromoimidazo[1,2-*a*]pyridin-3-yl)ethan-1-one (5.00 g, 20.9 mmol) and DMF-DMA (15.0 mL, 112 mmol) were reacted using general synthetic procedure A to give (*E*)-1-(6-bromoimidazo[1,2-*a*]pyridin-3-yl)-3-(dimethylamino)prop-2-en-1-one as a pale yellow solid (4.60, 75%). ¹H NMR (CDCl₃) *δ* 2.89 (s, 3H), 3.10 (s, 3H), 5.57 (d, 1H, *J* 12.5), 7.35 (dd, 1H, *J* 9.5 & 2.0), 7.50 (d, 1H, *J* 9.5), 7.71 (d, 1H, *J* 12.5), 8.11 (s, 1H), 9.94 (d, 1H, *J* 2.0). HRMS *m*/*z* 294.0241 [M(⁷⁹Br)+H]⁺, 296.0221 [M(⁸¹Br)+H]⁺.

*General synthetic procedure B:* To a solution of an amine (1.00 eq.) and cyanamide (2.00 eq.) in MeCN (100 mL) on an ice bath, was added TMSCl (2.00 eq.) dropwise. The reaction mixture was heated at reflux overnight and filtered while hot. The precipitate was washed with Et₂O or EtOAc (100-150 mL) to give the desired guanidine.

Example: 1-(6-(4-Methylpiperazin-1-yl)pyridin-3-yl)guanidine hydrochloride. 6-(4-Methylpiperazin-1-yl)pyridin-3-amine (8.46 g, 44.0 mmol) and cyanamide (3.70 g, 88.0 mmol) were reacted using general synthetic procedure B in the presence of TMSCl (11.2 mL, 88.2 mmol) to give 1-(6-(4-Methylpiperazin-1-yl)pyridin-3-yl)guanidine hydrochloride as a dark purple sticky solid (11.9 g, 100%). ¹H NMR (D₂O) *δ* 2.94 (s, 3H), 3.16-3.22 (m, 2H), 3.28-3.34 (m, 2H), 3.62 (d, 2H, *J* 12.0), 4.34 (d, 2H, *J* 14.0), 7.01 (d, 1H, *J* 9.0), 7.63 (dd, 1H, *J* 9.0 & 2.5), 8.08 (d, 1H, *J* 3.0)*.* HRMS *m*/*z* 235.1646.

*General synthetic procedure C:* NBS (1.20 eq.) was added to a solution of a 4-(2-butoxyvinyl)pyrimidine derivative (1.00-3.00 eq.) in 1,4-dioxane/H₂O (3:1), and the reaction mixture was stirred at room temperature for 1 h. Upon addition of an amine (1.00 eq.), the reaction mixture was heated at 85 °C for 2.5 h and concentrated under reduced pressure. The residue was triturated with EtOAc and filtered, and the solid washed with EtOAc and/or purified by flash column chromatography (silica gel, P.E. ramping to 10-50% DCM in P.E.) to give the desired 4-(3-heteroaryl)pyrimidine.

Example: 3-(2-Chloro-5-fluoropyrimidin-4-yl)imidazo[1,2-a]pyridine. (E)-4-(2-Butoxyvinyl)-2-chloro-5-fluoropyrimidine (900 mg, 3.90 mmol) was reacted with NBS (830 mg, 4.67 mmol) and pyridin-2-amine (370 mg, 3.90 mmol) according to general synthetic procedure C to give 3-(2-chloro-5-fluoropyrimidin-4-yl)imidazo[1,2-*a*]pyridine as a white solid (800 mg, 93%). ¹H NMR (DMSO-*d*₆): *δ* 7.35 (t, 1H, *J* 7.0), 7.65 (t, 1H, *J* 8.0), 7.88 (d, 1H, *J* 9.0), 8.51 (d, 1H, *J* 4.0), 8.85 (d, 1H, *J* 3.0), 9.73 (d, 1H, *J* 7.0). HRMS *m*/*z* 249.0309 [M(³⁵Cl)+H]⁺, 251.0280 [M(³⁷Cl)+H]⁺.

*General synthetic procedure D:* To a solution of a guanidine (1.10-1.50 eq.) in 2-methoxyethanol (4 mL) were added an enaminone (1.00 eq.), if necessary, and NaOH (1.50-2.20 eq.). The reaction mixture was heated at 160-200 °C under microwave irradiation for 1 h, cooled down to room temperature and concentrated under reduced pressure. The residue was purified by FlashMaster Personal⁺ chromatography or flash column chromatography (silica gel, DCM ramping to DCM:CH₃OH:32% NH₃ in H₂O = 455:45:1) and washed with ice-cold CH₃OH to give the desired pyrimidine.

*General synthetic procedure E:* A microwave-tube charged with a halide (1.00 eq.), a pyridin-3-amine (1.00 eq.), Cs₂CO₃ (2.00 eq.), Pd₂(dba)₃ or Pd(OAc)₂ (0.05 eq.), and xantphos or BINAP (0.05 eq.) in 1,4-dioxane (100 mM in halide) was heated at 160-200 °C under microwave irradiation for 1 h. The reaction mixture was cooled down to room temperature, concentrated and purified by flash column chromatography (silica gel, DCM ramping to DCM:CH₃OH = 91:9) to afford the desired product.

*General synthetic procedure F:* To a suspension of a halide (1.00 eq.) in MeCN (167 mM in halide) were added a boronic acid or boronate ester (0.90-2.00 eq.), Ph(PPh₃)₄ or Pd(dppf)Cl₂·CH₂Cl₂ (0.05 eq.) and 0.5 M Na₂CO₃ (1.40 eq.). The reaction mixture was heated at 130-140 °C under microwave irradiation for 1 h. The reaction mixture was cooled down to room temperature, concentrated and purified by flash column chromatography (silica gel, DCM ramping to DCM:CH₃OH = 91:9) to afford the desired product.

### Examples

**4-(Imidazo[1,2-*a*]pyridin-3-yl)-*N*-(6-(4-methylpiperazin-1-yl)pyridin-3-yl)pyrimidin-2-amine (1).** Prepared by treatment of 1-(6-(4-methylpiperazin-1-yl)pyridin-3-yl)guanidine hydrochloride (469 mg, 2.00 mmol) and (*E*)-3-(dimethylamino)-1-(imidazo[1,2-*a*]pyridin-3-yl)prop-2-en-1-one (220 mg, 1.02 mmol) using the synthetic procedure D. A yellow solid (165 mg, 42%). ¹H NMR (DMSO-*d*₆) *δ* 2.23 (s, 3H), 2.42 (t, 4H, *J* 4.5)*,* 3.44 (t, 4H, *J* 4.5)*,* 6.88 (d, 1H, *J* 9.0), 7.07 (app br s, 1H), 7.33 (d, 1H, *J* 5.5), 7.49 (app t, 1H, *J* 7.5), 7.76 (d, 1H, *J* 9.0), 7.89 (d, 1H, *J* 7.5), 8.35 (s, 1H), 8.36 (d, 1H, *J* 5.5), 8.59 (s, 1H), 9.40 (br s, 1H), 10.00 (app br s, 1H). HRMS *m*/*z* 387.2042 [M+H]⁺.

**4-(6-Fluoro-2-methylimidazo[1,2-*a*]pyridin-3-yl)-*N*-(6-(4-methylpiperazin-1-yl)pyridin-3-yl)pyrimidin-2-amine (2).** Prepared by treatment of 1-(6-(4-methylpiperazin-1-yl)pyridin-3-yl)guanidine hydrochloride (469 mg, 2.00 mmol) and (E)-1-(6-chloro-2-methylimidazo[1,2-*b*]pyridazin-3-yl)-3-(dimeth-ylamino)prop-2-en-1-one (250 mg, 1.02 mmol) using the general procedure D. A yellow solid (105 mg, 25%). ¹H NMR (DMSO-*d*₆) *δ* 2.22 (s, 3H), 2.41 (m, 4H), 2.65 (s, 3H), 3.42 (m, 4H), 6.85 (d, 1H, *J* 9.0), 7.03 (d, 1H, *J* 5.5), 7.51 (d, 1H, *J* 8.0), 7.68 (dd, 1H, J9.0 & 5.5), 7.84 (d, 1H, *J* 7.0), 8.33 (s, 1H), 8.45 (d, 1H, *J* 5.0), 9.43 (s, 1H), 9.81 (br s, 1H). HRMS *m*/*z* 419.2109 [M+H]⁺.

**4-(Imidazo[1,2-*a*]pyridin-3-yl)-5-methyl-*N*-(6-(4-methylpiperazin-1-yl)pyridin-3-yl) pyrimidin-2-amine (3).** Prepared by reaction between 1-(6-(4-methylpiperazin-1-yl)pyridin-3-yl)guanidine hydrochloride (469 mg, 2.00 mmol) and (*E*)-3-(dimethylamino)-1-(imidazo[1,2-*a*]pyridin-3-yl)-2-methylprop-2-en-1-one (230 mg, 1.00 mmol) using the synthetic procedure D. A pinkish solid (110 mg, 28%). ¹H NMR (CDCl₃) *δ* 2.37 (s, 3H), 2.44 (s, 3H), 2.56 (t, 4H, *J* 5.0), 3.55 (t, 4H, *J* 5.0), 6.69 (d, 1H, *J* 9.0), 6.78 (t, 1H, *J* 7.0), 6.88 (s, 1H), 7.33 (t, 1H, *J* 7.5), 7.71 (d, 1H, *J* 9.0), 7.79 (dd, 1H, *J* 9.0 & 2.5), 8.18 (s, 1H), 8.29 (m, 2H), 9.55 (d, 1H, *J* 7.5). HRMS *m*/*z* 401.2205 [M+H]⁺.

**5-Fluoro-4-(imidazo[1,2-*a*]pyridin-3-yl)-*N*-(pyridin-3-yl)pyrimidin-2-amine (4). Prepared** by reaction between 3-(2-chloro-5-fluoropyrimidin-4-yl)imidazo[1,2-*a*]pyridine (250 mg, 1.01 mmol) and 3-aminopyridine (100 mg, 1.06 mmol) using general synthetic procedure E. A pale yellow solid (85 mg, 27%). ¹H NMR (DMSO-*d*₆) *δ* 7.19 (t, 1H, *J* 6.5), 7.37 (dd, 1H, *J* 8.0 & 4.5), 7.58 (t, 1H, *J* 8.0), 7.84 (d, 1H, *J* 8.5), 8.18 (d, 1H, *J* 8.0), 8.22 (d, 1H, *J* 4.0), 8.43 (d, 1H, *J* 4.0), 8.60 (d, 1H, *J* 3.0), 8.85 (m, 1H), 9.94 (s, 1H), 10.09 (d, 1H, *J* 6.5). HRMS *m*/*z* 307.1105 [M+H]⁺.

**4-(6-Bromoimidazo[1,2-*a*]pyridin-3-yl)-*N*-(6-(4-methylpiperazin-1-yl)pyridin-3-yl)pyrimidin-2-amine (5).** Prepared by treatment of 1-(6-(4-methylpiperazin-1-yl)pyridin-3-yl)guanidine hydrochloride (469 mg, 2.00 mmol) with (*E*)-1-(6-bromoimidazo[1,2-*a*]pyridin-3-yl)-3-(dimethylamino)prop-2-en-1-one (300 mg, 1.02 mmol) using the general synthetic procedure D. A yellow solid (185 mg, 39%). ¹H NMR (DMSO-*d*₆) *δ* 2.22 (s, 3H), 2.40 (t, 4H, *J* 4.5), 3.44 (t, 4H, *J* 4.5), 6.88 (d, 1H, *J* 9.0), 7.32 (d, 1H, *J* 5.5), 7.56 (d, 1H, *J* 9.5), 7.72 (d, 1H, *J* 9.5), 7.79 (d, 1H, *J* 6.0), 8.26 (br s, 1H), 8.38 (d, 1H, *J* 5.5), 8.59 (s, 1H), 9.40 (br s, 1H), 9.92 (app br s, 1H). HRMS *m*/*z* 465.1151 [M(⁷⁹Br)+H]⁺.

***N*-(6-(4-Methylpiperazin-1-yl)pyridin-3-yl)-4-(6-phenylimidazo[1,2-*a*]pyridin-3-yl)pyrimidin-2-amine (6).** Prepared by treatment of 4-(6-bromoimidazo[1,2-*a*]pyridin-3-yl)-*N*-(6-(4-methylpiperazin-1-yl)pyridin-3-yl)pyrimidin-2-amine (240 mg, 0.52 mmol) and 4,4,5,5-tetramethyl-2-phenyl-1,3,2-dioxaborolane (106 mg, 0.52 mmol) using the synthetic procedure F. A yellow solid (115 mg, 47%). ¹H NMR (DMSO-*d*₆) *δ* 2.21 (s, 3H), 2.36 (m, 4H), 6.52 (br s, 1H), 7.32 (d, 1H, *J* 5.5), 7.42 (m, 3H), 7.56 (br s, 2H), 7.78 (m, 2H), 7.84 (d, 1H, *J* 9.0), 8.30 (s, 1H), 8.41 (d, 1H, *J* 5.0), 8.55 (s, 1H), 9.37 (s, 1H), 9.96 (br s, 1H). HRMS *m*/*z* 463.2357 [M+H]⁺.

***N*-(6-(4-Methylpiperazin-1-yl)pyridin-3-yl)-4-(6-(pyridin-3-yl)imidazo[1,2-*a*]pyridin-3-yl)pyrimidin-2-amine (7).** Prepared by reaction of 4-(6-bromoimidazo[1,2-*a*]pyridin-3-yl)-*N*-(6-(4-methylpiperazin-1-yl)pyridin-3-yl)pyrimidin-2-amine (240 mg, 0.52 mmol) and 3-(4,4,5,5-tetramethyl-1,3,2-dioxaborolan-2-yl)pyridine (108 mg, 0.53 mmol) using the synthetic procedure F. A yellow solid (98 mg, 41%). ¹H NMR (DMSO-*d*₆) *δ* 2.21 (s, 3H), 2.36 (m, 4H), 3.27 (br s, 4H), 6.53 (br s, 1H), 7.33 (d, 1H, *J* 5.5), 7.44 (br s, 1H), 7.78 (d, 1H, *J* 8.5), 7.82 (d, 1H, *J* 9.5), 7.88 (d, 1H, *J* 9.0)*,* 7.94 (br s, 1H), 8.29 (s, 1H), 8.40 (d, 1H, *J* 5.0), 8.58 (s, 1H), 8.62 (d, 1H, *J* 4.0), 8.89 (br s, 1H), 9.39 (s, 1H), 10.04 (br s, 1H). HRMS *m*/*z* 464.2314 [M+H]⁺.

***N*-(6-(4-Methylpiperazin-1-yl)pyridin-3-yl)-4-(6-(pyrimidin-5-yl)imidazo[1,2-*a*]pyridin-3-yl)pyrimidin-2-amine (8).** Prepared by reaction of 4-(6-bromoimidazo[1,2-*a*]pyridin-3-yl)-*N*-(6-(4-methylpiperazin-1-yl)pyridin-3-yl)pyrimidin-2-amine (240 mg, 0.52 mmol) and 5-(4,4,5,5-tetramethyl-1,3,2-dioxaborolan-2-yl)pyrimidine (110 mg, 0.53 mmol) using the synthetic procedure F. A yellow solid (105 mg, 43%). ¹H NMR (DMSO-*d*₆) *δ* 2.21 (s, 3H), 2.37 (br s, 4H), 3.27 (s, 2H), 6.60 (br s, 1H), 7.34 (d, 1H, *J* 5.0), 7.77 (d, 1H, *J* 7.0), 7.86 (d, 1H, *J* 9.5), 7.91 (d, 1H, *J* 9.5), 8.31 (s, 1H), 8.40 (d, 1H, *J* 5.0), 8.61 (s, 1H), 9.09 (br s, 2H), 9.24 (s, 1H), 9.41 (s, 1H), 10.14 (br s, 1H). HRMS *m*/*z* 465.2268 [M+H]⁺.

**4-(6-(1-Methyl-1*H*-pyrazol-4-yl)imidazo[1,2-*a*]pyridin-3-yl)-*N*-(6-(4-methylpiperazin-1-yl)pyridin-3-yl)pyrimidin-2-amine (9).** Prepared by reaction of 4-(6-Bromoimidazo[1,2-*a*]pyridin-3-yl)-N-(6-(4-methylpiperazin-1-yl)pyridin-3-yl)pyrimidin-2-amine (240 mg, 0.52 mmol) and 1-methyl-4-(4,4,5,5-tetramethyl-1,3,2-dioxaborolan-2-yl)-1H-pyrazole (108 mg, 0.52 mmol) using the synthetic procedure F. A yellow solid (92 mg, 38%). ¹H NMR (DMSO-*d*₆) *δ* 2.76 (s, 3H), 3.13 (br s, 4H), 3.88 (s, 3H), 6.86 (d, 1H, *J* 6.5), 7.34 (d, 2H, *J* 5.0), 7.70 (d, 1H, *J* 9.5), 7.78 (d, 1H, *J* 9.5), 7.92 (d, 1H, *J* 8.5), 8.06 (br s, 1H), 8.39 (d, 1H, *J* 5.0), 8.47 (s, 1H), 8.53 (s, 1H), 9.48 (s, 1H), 9.94 (s, 1H). HRMS *m*/*z* 467.2422 [M+H]⁺.

**1-(4-(5-((5-Fluoro-4-(imidazo[1,2-*a*]pyridin-3-yl)pyrimidin-2-yl)amino)pyridin-2-yl)piperazin-1-yl)ethan-1-one (10).** Prepared by treatment of 3-(2-chloro-5-fluoropyrimidin-4-yl)imidazo[1,2-*a*]pyridine (250 mg, 1.01 mmol) and 1-(4-(5-aminopyridin-2-yl)piperazin-1-yl)ethan-1-one (230 mg, 1.04 mmol) using general synthetic procedure E. A yellow solid (75 mg, 17%). ¹H NMR (DMSO-*d*₆) *δ* 2.06 (s, 3H), 3.43 (m, 2H), 3.51 (m, 2H), 3.56 (m, 4H), 6.93 (d, 1H, *J* 9.0), 7.11 (m, 1H), 7.57 (t, 1H, *J* 7.5), 7.83 (d, 1H, *J* 9.0), 7.88 (d, 1H, *J* 8.5), 8.36 (s, 1H), 8.41 (d, 1H, *J* 3.0), 8.51 (d, 1H, *J* 3.0), 9.50 (s, 1H), 10.01 (br s, 1H). HRMS *m*/*z* 433.1899 [M+H]⁺.

**1-(4-(5-((4-(Imidazo[1,2-*a*]pyridin-3-yl)-5-methylpyrimidin-2-yl)amino)pyridin-2-yl)piperazin-1-yl)ethan-1-one (11).** Prepared by treatment of 3-(2-chloro-5-methylpyrimidin-4-yl)imidazo[1,2-*a*]pyridine (250 mg, 1.02 mmol) and 1-(4-(5-aminopyridin-2-yl)piperazin-1-yl)ethan-1-one (230 mg, 1.04 mmol) using general synthetic procedure E. A pinkish solid (84 mg, 19%). ¹H NMR (DMSO-*d*₆) *δ* 2.05 (s, 3H), 2.39 (s, 3H), 3.40 (m, 2H), 3.47 (m, 2H), 3.55 (m, 4H), 6.89 (d, 1H, *J* 9.0), 7.02 (t, 1H, *J* 6.5), 7.48 (t, 1H, *J* 8.0), 7.76 (d, 1H, *J* 9.0), 7.90 (d, 1H, *J* 9.0), 8.29 (s, 1H), 8.37 (s, 1H), 8.39 (m, 1H), 9.28 (s, 1H), 9.75 (br s, 1H). HRMS *m*/*z* 429.2152 [M+H]⁺.

**4-(6-Fluoroimidazo[1,2-*a*]pyridin-3-yl)-*N*-(6-(4-methylpiperazin-1-yl)pyridin-3-yl) pyrimidin-2-amine (12).** Prepared by treatment of 1-(6-(4-methylpiperazin-1-yl)pyridin-3-yl)guanidine hydrochloride (469 mg, 2.00 mmol) and (*E*)-3-(dimethylamino)-1-(6-fluoroimidazo[1,2-*a*]pyridin-3-yl)prop-2-en-1-one (235 mg, 1.02 mmol) using the general procedure D. A yellow solid (94 mg, 23%). ¹H NMR (DMSO-*d*₆) *δ* 2.32 (s, 3H), 2.54 (m, 4H), 3.47 (m, 4H), 6.89 (d, 1H, *J* 9.0), 7.35 (d, 1H, *J* 5.5), 7.60 (d, 1H, *J* 8.0), 7.82 (dd, 1H, *J* 9.0 & 5.5), 7.84 (d, 1H, *J* 7.0), 8.35 (s, 1H), 8.39 (d, 1H, *J* 5.0), 8.66 (s, 1H), 9.51 (br s, 1H), 10.07 (app br s, 1H). HRMS *m*/*z* 405.1948 [M+H]⁺.

**4-(Imidazo[1,2-*a*]pyridin-3-yl)-5-methyl-*N*-(6-(piperazin-1-yl)pyridin-3-yl)pyrimidin-2-amine (13).** Prepared by reacting 3-(2-chloro-5-methylpyrimidin-4-yl)imidazo[1,2-*a*]pyridine (200 mg, 0.82 mmol) and *tert-*butyl 4-(5-aminopyridin-2-yl)piperazine-1-carboxylate (228 mg, 0.82 mmol), followed by heating at 180 °C for 2 h under microwave conditions in the presence of NaOH (17 mg, 0.41 mmol) in 2-methoxyethan-1-ol (4 mL). A pink solid (20 mg, 7%). ¹H NMR (CDCl₃) *δ* 2.43 (s, 3H), 3.04 (t, 4H, *J* 4.5), 3.48 (s, 1H), 3.51 (t, 4H, *J* 4.5), 6.68 (d, 1H, J9.0), 6.77 (t, 1H, J7.0), 7.06 (s, 1H), 7.32 (t, 1H, *J* 8.0), 7.75 (d, 1H, *J* 8.5), 7.81 (dd, 1H, *J* 8.5 & 1.5), 8.17 (s, 1H), 8.28 (s, 1H), 8.29 (s, 1H), 9.55 (d, 1H, *J* 7.0). HRMS *m*/*z* 387.2042 [M+H]⁺.

**4-(Imidazo[1,2-*a*]pyridin-3-yl)-5-methyl-*N*-(6-morpholinopyridin-3-yl)pyrimidin-2-amine (14)**. Prepared by treatment of 3-(2-chloro-5-methylpyrimidin-4-yl)imidazo[1,2-*α*]pyridine (250 mg, 1.02 mmol) and 6-morpholinopyridin-3-amine (183 mg, 1.02 mmol) according to the general procedure E. A yellow solid (70 mg, 18%). ¹H NMR (DMSO-*d*₆) *δ* 2.41 (s, 3H), 3.39 (t, 4H, *J* 4.5), 3.74 (t, 4H, *J* 5.0), 6.88 (d, 1H, *J* 9.0), 7.04 (t, 1H, *J* 6.0), 7.50 (d, 1H, *J* 7.5), 7.78 (d, 1H, *J* 9.0), 7.92 (d, 1H, *J* 8.5), 8.31 (s, 1H), 8.39 (s, 1H), 8.41 (s, 1H), 9.30 (s, 1H), 9.76 (s, 1H). HRMS *m*/*z* 388.1884 [M+H]⁺.

**5-Fluoro-4-(imidazo[1,2-*a*]pyridin-3-yl)-*N*-(6-morpholinopvridin-3-yl)pyrimidin-2-amine (15)**. Prepared by treatment of 3-(2-chloro-5-fluoropyrimidin-4-yl)imidazo[1,2-a]pyridine (250 mg, 1.02 mmol) and 6-morpholinopyridin-3-amine (180 mg, 1.02 mmol) according to the general synthetic procedure E. A yellow solid (90 mg, 23%). ¹H NMR (DMSO-*d*₆) *δ* 3.40 (t, 4H, *J* 4.5), 3.73 (t, 4H, *J* 4.5), 6.90 (d, 1H, *J* 9.0), 7.11 (t, 1H, *J* 6.5), 7.57 (t, 1H, *J* 8.0), 7.83 (d, 1H, *J* 9.0), 7.88 (d, 1H, *J* 8.0), 8.36 (s, 1H), 8.41 (d, 1H, *J* 3.5), 8.51 (d, 1H, *J* 3.5), 9.50 (s, 1H), 10.00 (br s, 1H). HRMS *m*/*z* 392.1633 [M+H]⁺.

**4-(6-Bromoimidazo[1,2-*a*]pyridin-3-yl)-*N*-(6-(4-(methylsulfonyl)piperazin-1-yl)pyridin-3-yl)pyrimidin-2-amine (16).** Prepared by treatment of 1-(6-(4-(methylsulfonyl)piperazin-1-yl)pyridin-3-yl)guanidine (598 mg, 2.00 mmol) with (*E*)-1-(6-bromoimidazo[1,2-*a*]pyridin-3-yl)-3-(dimethylamino) prop-2-en-1-one (300 mg, 1.02 mmol) using general procedure D. A yellow solid (210 mg, 40%). ¹H NMR (DMSO-*d*₆) *δ* 2.92 (s, 3H), 3.22 (t, 4H, *J* 5.0), 3.59 (t, 4H, *J* 5.0), 6.96 (d, 1H, *J* 9.0), 7.35 (d, 1H, *J* 5.0), 7.58 (dd, 1H, *J* 9.5 & 1.5), 7.74 (d, 1H, *J9.5),* 7.87 (d, 1H, *J* 8.0), 8.32 (br s, 1H), 8.39 (d, 1H, *J* 5.5), 8.61 (s, 1H), 9.49 (br s, 1H), 9.96 (app br s, 1H). HRMS *m*/*z* 529.0766 [M(⁷⁹Br)+H]⁺, 531.0753 [M(⁸¹Br)+H]⁺.

***N*-(6-(4-(Methylsulfonyl)piperazin-1-yl)pyridin-3-yl)-4-(6-phenylimidazo[1,2-*a*]pyridin-3-yl)pyrimidin-2-amine (17)**. Prepared by treatment of 4-(6-bromoimidazo[1,2-*a*]pyridin-3-yl)-*N*-(6-(4-(methylsulfonyl)piperazin-1-yl)pyridin-3-yl)pyrimidin-2-amine (270 mg, 0.51 mmol) and 4,4,5,5-tetramethyl-2-phenyl-1,3,2-dioxaborolane (106 mg, 0.52 mmol) using the synthetic procedure F. A yellow solid (108 mg, 40%). ¹H NMR (DMSO-*d*₆) *δ* 2.91 (s, 3H), 3.16 (t, 4H, *J* 5.0), 3.42 (t, 4H, *J* 5.0), 6.58 (br s, 1H), 7.34 (d, 1H, *J* 5.5), 7.44 (m, 3H), 7.58 (br s, 2H), 7.77 (d, 1H, *J* 9.0), 7.84 (m, 2H), 8.34 (s, 1H), 8.42 (d, 1H, *J* 5.0), 8.56 (s, 1H), 9.43 (s, 1H), 9.98 (br s, 1H). HRMS *m*/*z* 527.1978 [M+H]⁺.

***N*-(6-(4-(Methylsulfonyl)piperazin-1-yl)pyridin-3-yl)-4-(6-(pyridin-4-yl)imidazo[1,2-*a*] pyridin-3-yl)pyrimidin-2-amine (18)**. Prepared by reaction of 4-(6-bromoimidazo[1,2-*a*]pyridin-3-yl)-N-(6-(4-(methylsulfonyl)piperazin-1-yl)pyridin-3-yl)pyrimidin-2-amine (270 mg, 0.51 mmol) and 4-(4,4,5,5-tetramethyl-1,3,2-dioxaborolan-2-yl)pyridine (108 mg, 0.53 mmol) using the synthetic procedure F. A yellow solid (100 mg, 37%). ¹H NMR (DMSO-*d*₆) *δ* 2.92 (s, 3H), 3.16 (m, 4H), 3.40 (br s, 4H), 6.65 (br s, 1H), 7.36 (d, 1H, *J* 5.0), 7.53 (br s, 2H), 7.80 (d, 1H, *J* 8.0), 7.88 (s, 2H), 8.36 (s, 1H), 8.43 (d, 1H, *J* 5.0), 8.58 (s, 2H), 8.61 (s, 1H), 9.44 (s, 1H), 10.16 (br s, 1H). HRMS *m*/*z* 528.1931 [M+H]⁺.

***N*-(6-(4-(Methylsulfonyl)piperazin-1-yl)pyridin-3-yl)-4-(6-(pyridin-3-yl)imidazo[1,2-*a*] pyridin-3-yl)pyrimidin-2-amine (19)**. Prepared by reaction of 4-(6-bromoimidazo[1,2-*a*]pyridin-3-yl)-N-(6-(4-(methylsulfonyl)piperazin-1-yl)pyridin-3-yl)pyrimidin-2-amine (270 mg, 0.51 mmol) and 3-(4,4,5,5-tetramethyl-1,3,2-dioxaborolan-2-yl)pyridine (108 mg, 0.53 mmol) using the synthetic procedure F. A beige solid (96 mg, 36%). ¹H NMR (DMSO-*d*₆) *δ* 2.91 (s, 3H), 3.17 (t, 4H, *J* 5.0), 3.42 (br s, 4H), 6.62 (br s, 1H), 7.35 (d, 1H, *J* 5.5), 7.46 (br s, 1H), 7.81 (d, 1H, *J* 1.5), 7.83 (d, 1H, *J* 1.5), 7.89 (d, 1H, *J* 9.0), 7.97 (br s, 1H), 8.34 (s, 1H), 8.42 (d, 1H, *J* 5.0), 8.60 (s, 1H), 8.65 (dd, 1H, *J* 4.5 & 1.5), 8.89 (br s, 1H), 9.45 (s, 1H), 10.06 (br s, 1H). HRMS *m*/*z* 528.1928 [M+H]⁺.

***N*-(6-(4-(Methylsulfonyl)piperazin-1-yl)pyridin-3-yl)-4-(6-(pyrimidin-5-yl)imidazo|1,2-*a*] pyridin-3-yl)pyrimidin-2-amine (20)**. Prepared by reaction of 4-(6-bromoimidazo[1,2-*a*]pyridin-3-yl)-N-(6-(4-(methylsulfonyl)piperazin-1-yl)pyridin-3-yl)pyrimidin-2-amine (270 mg, 0.51 mmol) and 5-(4,4,5,5-tetramethyl-1,3,2-dioxaborolan-2-yl)pyrimidine (110 mg, 0.53 mmol) using the synthetic procedure F. A yellow solid (85 mg, 32%). ¹H NMR (DMSO-*d*₆) *δ* 2.92 (s, 3H), 3.18 (t, 4H, *J* 5.0), 3.43 (br s, 4H), 6.69 (br s, 1H), 7.36 (d, 1H, *J* 5.0), 7.82 (d, 1H, *J* 7.0), 7.87 (d, 1H, *J* 9.0), 7.93 (d, 1H, *J* 9.0), 8.37 (s, 1H), 8.42 (d, 1H, *J* 5.0), 8.62 (s, 1H), 9.09 (br s, 2H), 9.27 (s, 1H), 9.47 (s, 1H), 10.14 (br s, 1H). HRMS *m*/*z* 529.1881 [M+H]⁺.

***N*-(6-(4-(Methylsulfonyl)piperazin-1-yl)pyridin-3-yl)-4-(6-(thiophen-2-yl)imidazo[1,2-*a*] pyridin-3-yl)pyrimidin-2-amine (21)**. Prepared by reaction of 4-(6-bromoimidazo[1,2-*a*]pyridin-3-yl)-N-(6-(4-(methylsulfonyl)piperazin-1-yl)pyridin-3-yl)pyrimidin-2-amine (270 mg, 0.51 mmol) and thiophene-2-boronic acid (67 mg, 0.52 mmol) using the synthetic procedure F. A beige solid (90 mg, 33%). ¹H NMR (DMSO-*d*₆) *δ* 2.92 (s, 3H), 3.18 (t, 4H, *J* 5.0), 3.45 (br s, 4H), 6.70 (d, 1H, *J* 5.5), 7.14 (t, 1H, *J* 4.0), 7.32 (s, 1H), 7.33 (s, 1H), 7.59 (d, 1H, *J* 5.0), 7.72 (d, 1H, *J* 9.5), 7.84 (m, 2H), 8.35 (d, 1H,*J* 2.0), 8.42 (d, 1H, *J* 5.0), 8.55 (s, 1H), 9.43 (s, 1H), 10.00 (br s, 1H). HRMS *m*/*z* 533.1539 [M+H]⁺.

**4-(6-Chloroimidazo[1,2-*a*]pyridin-3-yl)-*N*-(6-(4-methylpiperazin-1-yl)pyridin-3-yl) pyrimidin-2-amine (22)**. Prepared by reaction of 1-(6-(4-methylpiperazin-1-yl)pyridin-3-yl)guanidine dinitrate (720 mg, 2.00 mmol) with (*E*)-3-(dimethylamino)-1-(6-chloroimidazo[1,2-*a*]pyridin-3-yl)prop-2-en-1-one (250 mg, 1.00 mmol) using general synthetic procedure D. A gold solid (130 mg, 31%). ¹H NMR (DMSO-*d*₆) *δ* 2.24 (s, 3H), 2.44 (t, 4H, *J* 4.5), 3.45 (t, 4H, *J* 4.5), 6.88 (d, 1H, *J* 9.0), 7.33 (d, 1H, *J* 5.5), 7.50 (d, 1 H, *J* 9.5), 7.78 (d, 2H, *J* 9.5), 8.27 (br s, 1H), 8.38 (d, 1H, *J* 5.5), 8.62 (s, 1 H), 9.41 (br s, 1H), 9.88 (br s, 1H). HRMS *m*/*z* 421.1652 [M+H]⁺.

**4-(6-(2-Aminopyrimidin-5-yl)imidazo[1,2-*a*]pyridin-3-yl)-*N*-(6-(4-methylpiperazin-1-yl) pyridin-3-yl)pyrimidin-2-amine (23)**. Prepared by treatment of 4-(6-bromoimidazo[1,2-*a*]pyridin-3-yl)-N-(6-(4-methylpiperazin-1-yl)pyridin-3-yl)pyrimidin-2-amine (240 mg, 0.52 mmol) and 5-(4,4,5,5-tetramethyl-1,3,2-dioxaborolan-2-yl)pyrimidin-2-amine (115 mg, 0.52 mmol) using the synthetic procedure F. A yellow solid (124 mg, 52%). ¹H NMR (DMSO-*d*₆) *δ* 2.21 (s, 3H), 2.38 (t, 4H, *J* 4.5), 6.62 (br s, 1H), 6.88 (s, 2H), 7.31 (d, 1H, *J* 5.5), 7.71 (d, 1H, *J* 9.0), 7.81 (d, 1H, *J* 9.0), 7.85 (dd, 1H, *J* 9.0 & 2.5), 8.29 (s, 1H), 8.38 (d, 1H, *J* 5.5), 8.54 (s, 1H), 8.58 (m, 2H), 9.40 (s, 1H), 9.91 (br s, 1H). HRMS *m*/*z* 480.2369 [M+H]⁺.

**4-(6-(1*H*-Pyrazol-4-yl)imidazo[1,2-*a*]pyridin-3-yl)-*N*-(6-(4-methylpiperazin-1-yl)pyridin-3-yl)pyrimidin-2-amine (24)**. Prepared by treatment of 4-(6-bromoimidazo[1,2-*a*]pyridin-3-yl)-*N*-(6-(4-methylpiperazin-1-yl)pyridin-3-yl)pyrimidin-2-amine (240 mg, 0.52 mmol) and 4-(4,4,5,5-tetramethyl-1,3,2-dioxaborolan-2-yl)-1H-pyrazole (100 mg, 0.52 mmol) using the synthetic procedure F. A yellow solid (85 mg, 38%). ¹H NMR (DMSO-*d*₆) *δ* 2.76 (s, 3H), 3.13 (br s, 4H), 6.86 (d, 1H, *J* 6.5), 7.36 (d, 2H, *J* 5.0), 7.72 (d, 1H, *J* 9.5), 7.80 (d, 1H, *J* 9.5), 7.94 (d, 1H, *J* 8.5), 8.08 (br s, 1H), 8.41 (d, 1H, *J* 5.0), 8.49 (s, 1H), 8.55 (s, 1H), 9.50 (s, 1H), 9.96 (s, 1H), 10.12 (s, 1H). HRMS *m*/*z* 453.2261 [M+H]⁺.

***N*-(6-(Azetidin-1-yl)pyridin-3-yl)-4-(6-phenylimidazo[1,2-*a*]pyridin-3-yl)pyrimidin-2-amine (25).** Prepared by treatment of *N*-(6-(azetidin-1-yl)pyridin-3-yl)-4-(6-bromoimidazo[1,2-*a*]pyridin-3-yl) pyrimidin-2-amine (211 mg, 0.50 mmol) and 4,4,5,5-tetramethyl-2-phenyl-1,3,2-dioxaborolane (102 mg, 0.50 mmol) using the synthetic procedure F. A yellow solid (47 mg, 22%). ¹H NMR (DMSO-*d*₆) *δ* 1.81 (s, 2H), 3.21 (s, 2H), 3.88 (s, 2H), 6.90 (d, 1H, *J* 9.0), 7.49 (m, 4H), 7.63 (s, 2H), 7.88 (m, 2H), 8.30 (s, 1H), 8.50 (d, 1H, *J* 5.0), 8.66 (s, 1H), 8.92 (s, 1H), 9.80 (s, 1H), 10.09 (s, 1H). HRMS *m*/*z* 420.1937 [M+H]⁺.

**4-(6-Bromoimidazo[1,2-*a*]pyridin-3-yl)-*N*-(6-(pyrrolidin-1-yl)pyridin-3-yl)pyrimidin-2-amine (26).** Prepared by treatment of 1-(6-(pyrrolidin-1-yl)pyridin-3-yl)guanidine hydrochloride (484 mg, 2.00 mmol) with (*E*)-1-(6-bromoimidazo[1,2-*a*]pyridin-3-yl)-3-(dimethylamino)prop-2-en-1-one (300 mg, 1.02 mmol) using general procedure D. A yellow solid (243 mg, 56%). ¹H NMR (DMSO-*d*₆) *δ* 1.95 (t, 4H, *J* 6.5), 3.39 (t, 4H, *J* 6.5), 6.51 (d, 1H, *J* 9.0), 7.30 (d, 1H, *J* 5.5), 7.56 (d, 1H, *J* 9.0), 7.72 (m, 2H), 8.16 (s, 1H), 8.36 (d, 1H, *J* 5.5), 8.59 (s, 1H), 9.27 (br s, 1H), 9.89 (app br s, 1H). HRMS *m*/*z* 436.0886 [M+H]⁺.

**4-(6-Phenylimidazo[1,2-*a*]pyridin-3-yl)-*N*-(6-(oyrrolidin-1-yl)oyridin-3-yl)oyrimidin-2-amine (27).** Prepared by treatment of 4-(6-bromoimidazo[1,2-α]pyridin-3-yl)-*N*-(6-(pyrrolidin-1-yl)pyridin-3-yl)pyrimidin-2-amine (218 mg, 0.50 mmol) and 4,4,5,5-tetramethyl-2-phenyl-1,3,2-dioxaborolane (102 mg, 0.50 mmol) using the synthetic procedure F. A yellow solid (72 mg, 33%). ¹H NMR (DMSO-*d*₆) *δ* 1.90 (t, 4H, *J* 6.5), 3.19 (t, 4H, *J* 6.5), 6.09 (br s, 1H), 7.29 (d, 1H, *J* 5.5), 7.37 (m, 3H), 7.48 (br s, 2H), 7.66 (d, 1H, *J* 7.5), 7.77 (dd, 1H, *J* 9.5 & 1.5), 7.82 (d, 1H, *J* 9.5), 8.18 (s, 1H), 8.38 (d, 1H, *J* 5.5), 8.55 (s, 1H), 9.23 (s, 1H), 9.99 (br s, 1H). HRMS *m*/*z* 434.2090 [M+H]⁺.

**5-Fluoro-*N*-(6-(4-methylpiperazin-1-yl)pyridin-3-yl)-4-(6-phenylimidazo[1,2-*a*]pyridin-3-yl)pyrimidin-2-amine (28).** Prepared by treatment of 3-(2-chloro-5-fluoropyrimidin-4-yl)-6-phenylimidazo[1,2-*a*]pyridine (162 mg, 0.50 mmol) and 6-(4-methylpiperazin-1-yl)pyridin-3-amine (115 mg, 0.60 mmol) using the general procedure E. A yellow solid (33 mg, 14%). ¹H NMR (DMSO-*d*₆) *δ* 2.23 (s, 3H), 2.39 (t, 4H, *J* 5.0), 3.28 (t, 4H, *J* 5.0), 6.50 (br s, 1H), 7.41 (m, 3H), 7.53 (br s, 2H), 7.75 (dd, 1H, *J* 9.0 & 2.5), 7.84 (dd, 1H, J9.0 & 1.5), 7.89 (d, 1H, J9.0), 8.27 (d, 1H, *J* 2.5), 8.37 (d, 1H, *J* 4.0), 8.56 (d, 1H, *J* 3.5), 9.44 (s, 1H), 9.92 (br s, 1H). HRMS *m*/*z* 481.2263 [M+H]⁺.

**Methyl 4-(5-((5-fluoro-4-(6-phenylimidazo[1,2-*a*]pyridin-3-yl)pyrimidin-2-yl)amino)pyridin-2-yl)piperidine-1-carboxylate (29)**. Prepared by treatment of 3-(2-chloro-5-fluoropyrimidin-4-yl)-6-phenylimidazo[1,2-*a*]pyridine (162 mg, 0.50 mmol) and methyl 4-(5-aminopyridin-2-yl)piperidine-1-carboxylate (141 mg, 0.60 mmol) using the general procedure E. A yellow solid (35 mg, 13%). ¹H NMR (DMSO-*d*₆) *δ* 1.51 (m, 2H), 1.86 (m, 2H), 2.57 (m, 1H), 2.80 (m, 2H), 3.62 (s, 3H), 3.93 (m, 2H), 6.55 (br s, 1H), 7.42 (m, 3H), 7.56 (br s, 2H), 7.78 (d, 1H, *J* 8.5), 7.86 (dd, 1H, J9.0 & 1.5), 7.90 (d, 1H, *J* 9.0), 8.25 (d, 1H, *J* 2.5), 8.39 (d, 1H, *J* 4.0), 8.57 (d, 1H, *J* 3.0), 9.48 (s, 1H), 9.93 (br s, 1H). HRMS *m*/*z* 524.2209 [M+H]⁺.

**5-Fluoro-*N*-(6-(4-(2-morpholinoethyl)piperazin-1-yl)pyridin-3-yl)-4-(6-phenylimidazo[1,2-*a*]pyridin-3-yl)pyrimidin-2-amine (30)**. Prepared by treatment of 3-(2-chloro-5-fluoropyrimidin-4-yl)-6-phenylimidazo[1,2-*a*]pyridine (162 mg, 0.50 mmol) and 6-(4-(2-morpholinoethyl)piperazin-1-yl)pyridin-3-amine (175 mg, 0.60 mmol) using the general procedure E. A yellow solid (28 mg, 10%). ¹H NMR (DMSO-*d*₆) *δ* 2.36 (m, 1H), 2.40 (t, 4H, J4.0), 2.45 (t, 4H, J4.0), 2.64 (m, 1H), 3.17 (m, 1H), 3.26 (t, 4H, *J* 4.0), 3.45 (m, 1H), 3.56 (t, 4H, *J* 4.0), 6.48 (br s, 1H), 7.41 (m, 3H), 7.52 (br s, 2H), 7.74 (d, 1H, *J* 8.5), 7.84 (dd, 1H, *J* 9.0 & 1.5), 7.89 (d, 1H, *J* 9.0), 8.27 (d, 1H, *J* 2.5), 8.37 (d, 1H, *J* 4.0), 8.56 (d, 1H, *J* 3.0), 9.43 (s, 1H), 9.92 (br s, 1H). HRMS *m*/*z* 580.2949 [M+H]⁺.

***N*-(6-Ethoxypyridin-3-yl)-5-fluoro-4-(6-phenylimidazo[1,2-*a*]pyridin-3-yl)pyrimidin-2-amine (31).** Prepared by treatment of 3-(2-chloro-5-fluoropyrimidin-4-yl)-6-phenylimidazo[1,2-*a*]pyridine (162 mg, 0.50 mmol) and 6-ethoxypyridin-3-amine (83 mg, 0.60 mmol) using the general procedure E. A yellow solid (48 mg, 23%). ¹H NMR (DMSO-*d*₆) *δ* 1.25 (t, 3H, *J* 7.0), 4.08 (m, 2H), 6.53 (d, 1H, *J* 9.0), 7.40 (m, 3H), 7.48 (br s, 2H), 7.82 (d, 1H, *J* 8.5), 7.85 (dd, 1H, *J* 9.0 & 1.5), 7.90 (d, 1H, *J* 9.0), 8.31 (d, 1H, *J* 2.5), 8.40 (d, 1H, *J* 4.0), 8.59 (d, 1H, *J* 3.0), 9.59 (s, 1H), 9.94 (s, 1H). HRMS *m*/*z* 427.1682 [M+H]⁺.

**(5-((5-Fluoro-4-(6-phenylimidazo[1,2-*a*]oyridin-3-yl)pyrimidin-2-yl)amino)oyridin-2-yl) (piperidin-1-yl)methanone (32)**. Prepared by treatment of 3-(2-chloro-5-fluoropyrimidin-4-yl)-6-phenylimidazo[1,2-*a*]pyridine (162 mg, 0.50 mmol) and (5-aminopyridin-2-yl)(piperidin-1-yl)methanone (123 mg, 0.60 mmol) using the general procedure E. A pale white solid (40 mg, 16%). ¹H NMR (DMSO-*d*₆) *δ* 1.45 (m, 2H), 1.55 (m, 2H), 1.60 (m, 2H), 3.41 (m, 2H), 3.58 (m, 2H), 7.38 (m, 2H), 7.45 (m, 2H), 7.66 (d, 2H, *J* 7.5), 7.88 (dd, 1H, *J* 9.0 & 1.5), 7.93 (d, 1H, *J* 9.0), 8.22 (dd, 1H, *J* 8.5 & 2.5), 8.44 (d, 1H, *J* 4.0), 8.70 (d, 1H, *J* 3.0), 8.83 (d, 1H, J2.5), 9.98 (s, 1H), 10.20 (s, 1H). HRMS *m*/*z* 494.2101 [M+H]⁺.

***N*-Cyclopropyl-5-((5-fluoro-4-(6-phenylimidazo[1,2-*a*]pyridin-3-yl)pyrimidin-2-yl)amino) picolinamide (33)**. Prepared by treatment of 3-(2-chloro-5-fluoropyrimidin-4-yl)-6-phenylimidazo[1,2-*a*]pyridine (162 mg, 0.50 mmol) and 5-amino-*N*-cyclopropylpicolinamide (106 mg, 0.60 mmol) using the general procedure E. A pale white solid (45 mg, 19%). ¹H NMR (DMSO-*d*₆) *δ* 0.63 (m, 2H), 0.68 (m, 2H), 2.85 (m, 1H), 7.35 (m, 3H), 7.53 (t, 2H, *J* 3.0), 7.85 (d, 1H, *J* 8.5), 7.88 (dd, 1H, *J* 9.0 & 1.5), 7.92 (d, 1H, *J* 9.0), 8.19 (dd, 1H, *J* 8.5 & 2.5), 8.40 (d, 1H, *J* 4.5), 8.44 (d, 1H, *J* 4.0), 8.71 (d, 1H, *J* 3.5), 8.86 (d, 1H, *J* 2.5), 9.94 (s, 1H), 10.26 (s, 1H). HRMS *m*/*z* 466.1790 [M+H]⁺.

***N*-(6-Chloropyridin-3-yl)-5-fluoro-4-(6-phenylimidazo[1,2-*a*]pyridin-3-yl)pyrimidin-2-amine (34).** Prepared by treatment of 3-(2-chloro-5-fluoropyrimidin-4-yl)-6-phenylimidazo[1,2-*a*]pyridine (162 mg, 0.50 mmol) and 6-chloropyridin-3-amine (77 mg, 0.60 mmol) using the general procedure E. A yellow solid (35 mg, 17%). ¹H NMR (DMSO-*d*₆) 7.16 (d, 1H, *J* 8.5), 7.43 (m, 3H), 7.57 (d, 2H, *J* 3.5), 7.88 (dd, 1H, *J* 9.0 & 1.5), 7.92 (d, 1H, J9.0), 8.13 (dd, 1H, *J* 8.5 & 2.5), 8.43 (d, 1H, *J* 4.0), 8.62 (d, 1H, *J* 2.5), 8.68 (d, 1H, *J* 3.5), 9.94 (s, 1H), 10.09 (s, 1H). HRMS *m*/*z* 417.1028 [M+H]⁺.

**4-(6-Methylimidazo[1,2-*a*]pyridin-3-yl)-*N*-(6-(4-methylpiperazin-1-yl)pyridin-3-yl)pyrimidin-2-amine (35).** Prepared by reaction of 1-(6-(4-methylpiperazin-1-yl)pyridin-3-yl)guanidine hydrochloride (615 mg, 2.00 mmol) with (*E*)-3-(dimethylamino)-1-(6-methylimidazo[1,2-*a*]pyridin-3-yl)prop-2-en-1-one (230 mg, 1.00 mmol) using general synthetic procedure D. A yellow solid (130 mg, 32%). ¹H NMR (DMSO-*d*₆) *δ* 2.27 (s, 3H), 2.37 (s, 3H), 2.63 (t, 4H, *J* 4.5), 6.91 (d, 1H, *J* 9.0), 7.29 (d, 1H, *J* 5.5), 7.32 (d, 1H, *J* 9.5), 7.63 (d, 1H, *J* 9.5), 7.78 (br s, 1H), 8.33 (d, 1 H, *J* 5.5), 8.36 (s, 1H), 8.49 (s, 1H), 9.31 (br s, 1H), 9.61 (br s, 1H). HRMS *m*/*z* 401.2200 [M+H]⁺.

***N*-(6-(4-Methylpiperazin-1-yl)pyridin-3-yl)-4-(6-(trifluoromethyl)imidazo[1,2-*a*]pyridin-3-yl)pyrimidin-2-amine (36).** Prepared by reaction of 1-(6-(4-methylpiperazin-1-yl)pyridin-3-yl)guanidine hydrochloride (615 mg, 2.00 mmol) with (E)-3-(dimethylamino)-1-(6-(trifluoromethyl)imidazo[1,2-*a*]pyridin-3-yl)prop-2-en-1-one (284 mg, 1.00 mmol) using general synthetic procedure D. A yellow solid (50 mg, 11%). ¹H NMR (DMSO-*d*₆) *δ* 2.22 (s, 3H), 2.41 (t, 4H, *J* 4.5), 3.42 (t, 4H, *J* 4.5), 6.80 (d, 1H, *J* 9.0), 7.36 (d, 1H, *J* 5.5), 7.67 (d, 1H, *J9.5),* 7.74 (d, 1H, *J9.5),* 7.94 (d, 1H, *J9.5),* 8.25 (s, 1H), 8.43 (d, 1H, *J* 5.5), 8.70 (s, 1H), 9.38 (br s, 1H), 10.09 (br s, 1H). HRMS *m*/*z* 455.1918 [M+H]⁺.

**4-(6-Chloroimidazo[1,2-*a*]pyridin-3-yl)-*N*-(6-(4-isopropylpiperazin-1-yl)pyridin-3-yl)pyrimidin-2-amine (37).** Prepared by reaction of 1-(6-(4-isopropylpiperazin-1-yl)pyridin-3-yl)guanidine (1.01 g, 3.01 mmol) with (*E*)-1-(6-chloroimidazo[1,2-*a*]pyridin-3-yl)-3-(dimethylamino) prop-2-en-1-one (250 mg, 1.00 mmol) using general synthetic procedure D. A yellow solid (116 mg, 26%). ¹H NMR (DMSO-*d*₆) *δ* 1.00 (s, 3H), 1.01 (s, 3H), 2.53 (t, 4H, *J* 4.5), 2.67 (m, 1H), 6.86 (d, 1H, *J* 9.0), 7.33 (d, 1H, *J* 5.5), 7.50 (d, 1H, *J* 9.5), 7.77 (d, 2H, *J* 9.5), 8.26 (br s, 1H), 8.38 (d, 1H, *J* 5.5), 8.62 (s, 1H), 9.40 (br s, 1H), 9.88 (br s, 1H). HRMS *m*/*z* 449.1965 [M(³⁵Cl)+H]⁺, 451.1936 [M(³⁷Cl)+H]⁺.

**4-(6-Bromoimidazo[1,2-*a*]pyridin-3-yl)-*N*-(6-(4-isopropylpiperazin-1-yl)pyridin-3-yl)pyrimidin-2-amine (38).** Prepared by treatment of 1-(6-(4-isopropylpiperazin-1-yl)pyridin-3-yl)guanidine (525 mg, 2.00 mmol) with (*E*)-1-(6-bromoimidazo[1,2-*a*]pyridin-3-yl)-3-(dimethylamino)prop-2-en-1-one (300 mg, 1.02 mmol) using the general synthetic procedure D. A yellow solid (195 mg, 40%). ¹H NMR (DMSO-*d*₆) *δ* 1.02 (s, 6H), 2.54 (t, 4H, *J* 4.5), 2.67 (m, 1H), 3.43 (t, 4H,*J* 4.5), 6.89 (d, 1H, *J* 8.5), 7.34 (d, 1H, *J* 5.0), 7.59 (d, 1H, *J* 9.5), 7.73 (d, 1H, *J* 9.5), 7.80 (s, 1H), 8.27 (br s, 1H), 8.39 (d, 1H, *J* 5.0), 8.61 (s, 1H), 9.42 (br s, 1H), 9.94 (br s, 1H). HRMS *m*/*z* 493.1465 [M(⁷⁹Br)+H]⁺, 495.1446 [M(⁸¹Br)+H]⁺.

***N*-(6-(4-Isopropylpiperazin-1-yl)pyridin-3-yl)-4-(6-phenylimidazo[1,2-*a*]pyridin-3-yl)pyrimidin-2-amine (39).** Prepared by treatment of 4-(6-bromoimidazo[1,2-a]pyridin-3-yl)-*N*-(6-(4-isopropylpiperazin-1-yl)pyridin-3-yl)pyrimidin-2-amine (125 mg, 0.26 mmol) with phenylboronic acid (61 mg, 0.50 mmol) using the general synthetic procedure F. A yellow solid (82 mg, 64%). ¹H NMR (DMSO-*d*₆) *δ* 1.00 (s, 6H), 2.67 (m, 1H), 3.28 (t, 4H, *J* 4.5), 6.52 (s, 1H), 7.32 (d, 1H, *J* 5.0), 7.43 (m, 3H), 7.57 (s, 2H), 7.77 (m, 2H), 7.84 (d, 1H, *J* 9.0), 8.30 (s, 1H), 8.40 (d, 1H, *J* 4.5), 8.55 (s, 1H), 9.37 (s, 1H), 9.96 (br s, 1H). HRMS *m*/*z* 491.2671 [M+H]⁺.

***N*-(6-(4-Isopropylpiperazin-1-yl)pyridin-3-yl)-4-(6-(pyridin-4-yl)imidazo[1,2-*a*]pyridin-3-yl)pyrimidin-2-amine (40).** Prepared by treatment of 4-(6-bromoimidazo[1,2-*a*]pyridin-3-yl)-*N*-(6-(4-isopropylpiperazin-1-yl)pyridin-3-yl)pyrimidin-2-amine (125 mg, 0.26 mmol) with 4-(4,4,5,5-tetramethyl-1,3,2-dioxaborolan-2-yl)pyridine (108 mg, 0.53 mmol) using the general synthetic procedure F. A yellow solid (62 mg, 49%). ¹H NMR (DMSO-*d*₆) *δ* 1.02 (s, 6H), 2.68 (m, 1H), 3.29 (t, 4H, *J* 4.5), 6.66 (br s, 1H), 7.36 (d, 1H, *J* 5.0), 7.52 (m, 2H), 7.80 (s, 1H), 7.89 (m, 2H), 8.36 (s, 1H), 8.44 (d, 1H, *J* 5.0), 8.58 (s, 2H), 8.61 (s, 1H), 9.45 (br s, 1H), 10.15 (br s, 1H). HRMS *m*/*z* 492.2645 [M+H]⁺.

**1-(4-(5-((4-(Imidazo[1,2-*a*]pyridin-3-yl)pyrimidin-2-yl)amino)pyridin-2-yl)piperazin-1-yl)ethan-1-one (41).** Prepared by treatment of 1-(6-(4-acetylpiperazin-1-yl)pyridin-3-yl)guanidine (525 mg, 2.00 mmol) and (*E*)-3-(dimethylamino)-1-(imidazo[1,2-*a*]pyridin-3-yl)prop-2-en-1-one (220 mg, 1.02 mmol) using the general synthetic procedure D. A beige solid (165 mg, 39%). ¹H NMR (DMSO-*d*₆) *δ* 2.06 (s, 3H), 3.43 (t, 2H, *J* 5.0), 3.50 (t, 2H, *J* 5.0), 3.57 (t, 4H, *J* 4.5), 6.93 (d, 1H, *J* 9.0), 7.08 (s, 1H), 7.34 (d, 1H, *J* 5.0), 7.49 (ddd, 1H, J9.0 & 7.0 & 1.0), 7.77 (d, 1H, J9.0), 7.93 (d, 1H, *J* 7.5), 8.37 (d, 1H, *J* 5.0), 8.39 (s, 1H), 8.60 (s, 1H), 9.44 (s, 1H), 10.02 (br s, 1H). HRMS *m*/*z* 415.2002 [M+H]⁺.

**4-(6-Bromoimidazo[1,2-*a*]pyridin-3-yl)-*N*-(6-(piperazin-1-yl)pyridin-3-yl)pyrimidin-2-amine (42).** Prepared by treatment of *tert*-butyl 4-(5-guanidinopyridin-2-yl)piperazine-1-carboxylate (641 mg, 2.00 mmol) with (*E*)-1-(6-bromoimidazo[1,2-*a*]pyridin-3-yl)-3-(dimethylamino)prop-2-en-1-one (300 mg, 1.02 mmol) using the general synthetic procedure D. A brown solid (84 mg, 19%). ¹H NMR (DMSO-*d*₆) *δ* 2.85 (m, 2H), 3.41 (m, 2H), 3.49 (m, 4H), 6.92 (td, 1H, *J* 7.0 & 1.0), 7.34 (d, 1H, *J* 5.5), 7.58 (m, 1H), 7.74 (d, 1H, *J* 9.0), 7.86 (d, 1H, *J* 8.5), 8.29 (d, 1H, *J* 9.0), 8.40 (t, 1H, *J* 5.0), 8.61 (s, 1H), 9.45 (m, 1H), 9.95 (br s, 1H). HRMS *m*/*z* 451.0998 [M(⁷⁹Br)+H]⁺, 453.0971 [M(⁸¹Br)+H]⁺.

**4-(6-(6-Methoxypyridin-3-yl)imidazo[1,2-*a*]pyridin-3-yl)-*N*-(6-(4-methylpiperazin-1-yl)pyridin-3-yl)pyrimidin-2-amine (43)**. Prepared by treatment of 4-(6-bromoimidazo[1,2-*a*]pyridin-3-yl)-N-(6-(4-methylpiperazin-1-yl)pyridin-3-yl)pyrimidin-2-amine (120 mg, 0.26 mmol) with 2-methoxy-5-pyridineboronic acid (80 mg, 0.52 mmol) using the general synthetic procedure F. A yellow solid (64 mg, 50%). ¹H NMR (DMSO-*d*₆) *δ* 2.36 (s, 3H), 3.93 (s, 3H), 6.60 (br s, 1H), 6.86 (br s, 1H), 7.33 (d, 1H, *J* 4.5), 7.75-7.85 (m, 4H), 8.32 (s, 1H), 8.40 (d, 1H, *J* 4.5), 8.49 (s, 1H), 8.56 (s, 1H), 9.41 (s, 1H), 9.97 (br s, 1H). HRMS *m*/*z* 494.2423 [M+H]⁺.

**1-(4-(5-((4-(6-Bromoimidazo[1,2-*a*]pyridin-3-yl)pyrimidin-2-yl)amino)pyridin-2-yl)piperazin-1-yl)ethan-1-one (44)**. Prepared by treatment of 1-(6-(4-acetylpiperazin-1-yl)pyridin-3-yl)guanidine (1.05 g, 4.00 mmol) with (*E*)-1-(6-bromoimidazo[1,2-*a*]pyridin-3-yl)-3-(dimethylamino)prop-2-en-1-one (600 mg, 2.04 mmol) using the general synthetic procedure D. A yellow solid (430 mg, 44%). ¹H NMR (DMSO-*d*₆) *δ* 2.05 (s, 3H), 3.43 (m, 2H), 3.52 (m, 2H), 3.55 (m, 4H), 6.92 (d, 1H, *J* 9.0), 7.34 (d, 1H, *J* 5.0), 7.58 (d, 1H, *J9.5),* 7.73 (d, 1H, *J* 9.5), 7.85 (d, 1H, *J* 8.0), 8.31 (s, 1H), 8.40 (d, 1H, *J* 5.0), 8.61 (s, 1H), 9.46 (br s, 1H), 9.96 (br s, 1H). HRMS *m*/*z* 493.1096 [M(⁷⁹Br)+H]⁺, 495.1078 [M(⁸¹Br)+H]⁺.

**1-(4-(5-((4-(6-Phenylimidazo[1,2-*a*]pyridin-3-yl)pyrimidin-2-yl)amino)pyridin-2-yl) piperazin-1-yl)ethan-1-one (45)**. Prepared by treatment of 1-(4-(5-((4-(6-bromoimidazo[1,2-*a*]pyridin-3-yl)pyrimidin-2-yl)amino)pyridin-2-yl)piperazin-1-yl)ethan-1-one (125 mg, 0.25 mmol) with phenylboronic acid (61 mg, 0.50 mmol) using the general synthetic procedure F. A beige solid (60 mg, 49%). ¹H NMR (DMSO-*d*₆) *δ* 2.05 (s, 3H), 3.26 (m, 2H), 3.50 (m, 4H), 6.56 (br s, 1H), 7.33 (d, 1H,*J* 5.0), 7.42 (m, 3H), 7.57 (m, 2H), 7.77-7.85 (m, 3H), 8.33 (s, 1H), 8.41 (d, 1H, *J* 5.0), 8.56 (s, 1H), 9.41 (s, 1H), 9.98 (br s, 1H). HRMS *m*/*z* 491.2315 [M+H]⁺.

**1-(4-(5-((4-(6-(Pyridin-4-yl)imidazo[1,2-*a*]oyridin-3-yl)oyrimidin-2-yl)amino)oyridin-2-yl)piperazin-1-yl)ethan-1-one (46)**. Prepared by treatment of 1-(4-(5-((4-(6-bromoimidazo[1,2-*α*]pyridin-3-yl)pyrimidin-2-yl)amino)pyridin-2-yl)piperazin-1-yl)ethan-1-one (125 mg, 0.25 mmol) with 4-(4,4,5,5-tetramethyl-1,3,2-dioxaborolan-2-yl)pyridine (108 mg, 0.53 mmol) using the general synthetic procedure F. A yellow solid (78 mg, 63%). ¹H NMR (DMSO-*d*₆) *δ* 2.05 (s, 3H), 3.23 (m, 2H), 3.50 (m, 4H), 6.61 (br s, 1H), 7.35 (d, 1H, *J* 5.0), 7.51 (br s, 2H), 7.78 (d, 1H, *J* 7.5), 7.88 (m, 2H), 8.34 (s, 1H), 8.43 (d, 1H, *J* 5.0), 8.56 (s, 2H), 8.61 (s, 1H), 9.42 (s, 1H), 10.16 (br s, 1H). HRMS *m*/*z* 492.2265 [M+H]⁺.

**1-(4-(5-((4-(6-(1*H*-Pyrazol-4-yl)imidazo[1,2-aloyridin-3-yl)oyrimidin-2-yl)amino)oyridin-2-yl)piperazin-1-yl)ethan-1-one (47)**. Prepared by treatment of 1-(4-(5-((4-(6-bromoimidazo[1,2-*a*]pyridin-3-yl)pyrimidin-2-yl)amino)pyridin-2-yl)piperazin-1-yl)etlian-1-one (125 mg, 0.25 mmol) with 4-(4,4,5,5-tetramethyl-1,3,2-dioxaborolan-2-yl)-1*H*-pyrazole (100 mg, 0.52 mmol) using the general synthetic procedure F. A yellow solid (75 mg, 62%). ¹H NMR (DMSO-*d*₆) *δ* 2.05 (s, 3H), 3.41 (m, 2H), 3.52 (m, 4H), 6.75 (br s, 1H), 7.31 (d, 1H, *J* 5.5), 7.74-7.87 (m, 4H), 8.20 (br s, 1H), 8.39 (m, 2H), 8.52 (s, 1H), 9.43 (s, 1H), 9.94 (br s, 1H), 13.06 (br s, 1H). HRMS *m*/*z* 481.2219 [M+H]⁺.

**1-(4-(5-((4-(6-(6-Methoxypyridin-3-ylimidazo[1,2-*a*]pyridin-3-yl)pyrimidin-2-yl)amino) pyridin-2-yl)oioerazin-1-yl)ethan-1-one (48)**. Prepared by treatment of 1-(4-(5-((4-(6-bromoimidazo[1,2-*a*]pyridin-3-yl)pyrimidin-2-yl)amino)pyridin-2-yl)piperazin-1-yl)ethan-1-one (125 mg, 0.25 mmol) with 2-methoxy-5-pyridineboronic acid (80 mg, 0.52 mmol) using the general synthetic procedure F. A yellow solid (66 mg, 51%). ¹H NMR (DMSO-*d*₆) *δ* 2.05 (s, 3H), 3.22 (m, 2H), 3.49 (m, 4H), 3.90 (s, 3H), 6.60 (br s, 1H), 6.83 (d, 1H, *J* 6.0), 7.33 (d, 1H, *J* 5.0), 7.75-7.85 (m, 4H), 8.33 (s, 1H), 8.41 (d, 1H, *J* 5.0), 8.47 (br s, 1H), 8.57 (s, 1H), 9.42 (s, 1H), 9.98 (br s, 1H). HRMS *m*/*z* 522.2372 [M+H]⁺.

**1-(4-(5-((4-(6-(1*H*-Indol-4-yl)imidazo[1,2-*a*]pyridin-3-yl)pyrimidin-2-yl)amino)pyridin-2-yl)piperazin-1-yl)ethan-1-one (49).** Prepared by treatment of 1-(4-(5-((4-(6-bromoimidazo[1,2-*a*]pyridin-3-yl)pyrimidin-2-yl)amino)pyridin-2-yl)piperazin-1-yl)ethan-1-one (125 mg, 0.25 mmol) with 4-(4,4,5,5-tetramethyl-1,3,2-dioxaborolan-2-yl)-1*H*-indole (122 mg, 0.50 mmol) using the general synthetic procedure F. A yellow solid (97 mg, 73%). ¹H NMR (DMSO-*d*₆) *δ* 2.05 (s, 3H), 3.03 (m, 2H), 3.10 (m, 2H), 3.44 (m, 4H), 5.78 (br s, 1H), 6.54 (s, 1H), 7.17-7.22 (m, 2H), 7.33 (d, 1H, *J* 5.0), 7.42 (s, 1H), 7.51 (d, 1H, *J* 8.0), 7.71-7.73 (m, 2H), 7.74 (d, 1H, *J* 9.5), 8.11 (s, 1H), 8.41 (d, 1H, *J* 5.0), 8.54 (s, 1H), 9.26 (s, 1H), 9.95 (br s, 1H), 11.38 (s, 1H). HRMS *m*/*z* 530.2422 [M+H]⁺.

**5-Methyl-*N*-(6-(4-methylpiperazin-1-yl)pyridin-3-yl)-4-(6-phenylimidazo[1,2-*a*]pyridin-3-yl)pyrimidin-2-amine (50).** Prepared by treatment of 3-(2-chloro-5-methylpyrimidin-4-yl)-6-phenylimidazo[1,2-*a*]pyridine (160 mg, 0.50 mmol) and 6-(4-methylpiperazin-1-yl)pyridin-3-amine (115 mg, 0.60 mmol) using the general synthetic procedure E. A beige solid (75 mg, 31%). ¹H NMR (DMSO-*d*₆) *δ* 2.24 (s, 3H), 2.38 (s, 3H), 2.41 (m, 4H), 3.26 (m, 4H), 6.42 (d, 1H, *J* 9.0), 7.39 (m, 3H), 7.49 (m, 2H), 7.76 (m, 2H), 7.83 (d, 1H, *J* 9.0), 8.25 (s, 1H), 8.30 (s, 1H), 8.41 (s, 1H), 9.27 (s, 1H), 9.64 (s, 1H). HRMS *m*/*z* 477.2518 [M+H]⁺.

**4-(6-(Furan-2-yl)imidazo[1,2-*a*]pyridin-3-yl)-*N*-(6-(4-(methylsulfonyl)piperazin-1-yl) pyridin-3-yl)pyrimidin-2-amine (51).** Prepared by treatment of 4-(6-bromoimidazo[1,2-*a*]pyridin-3-yl)-N-(6-(4-(methylsulfonyl)piperazin-1-yl)pyridin-3-yl)pyrimidin-2-amine (135 mg, 0.25 mmol) with 2-(furan-3-yl)-4,4,5,5-tetramethyl-1,3,2-dioxaborolane (92 mg, 0.50 mmol) using the general synthetic procedure F. A yellow solid (88 mg, 68%). ¹H NMR (DMSO-*d*₆) *δ* 2.92 (s, 3H), 3.19 (m, 4H), 3.50 (m, 4H), 6.64 (s, 1H), 6.80 (d, 1H, *J* 8.5), 6.89 (br s, 1H), 7.33 (d, 1H, *J* 5.0), 7.65 (s, 1H), 7.81 (m, 2H), 7.88 (d, 1H, *J* 9.0), 8.39-8.42 (m, 2H), 8.55 (s, 1H), 9.45 (s, 1H), 10.04 (br s, 1H). HRMS *m*/*z* 517.1776 [M+H]⁺.

**4-(6-(Benzofuran-2-yl)imidazo[1,2-*a*]pyridin-3-yl)-*N*-(6-(4-(methylsulfonyl)piperazin-1-yl)pyridin-3-yl)pyrimidin-2-amine (52).** Prepared by treatment of 4-(6-bromoimidazo[1,2-*a*]pyridin-3-yl)-N-(6-(4-(methylsulfonyl)piperazin-1-yl)pyridin-3-yl)pyrimidin-2-amine (135 mg, 0.25 mmol) with 2-(benzofuran-2-yl)-4,4,5,5-tetramethyl-1,3,2-dioxaborolane (122 mg, 0.50 mmol) using the general synthetic procedure F. A yellow solid (94 mg, 66%). ¹H NMR (DMSO-*d*₆) *δ* 2.83 (s, 3H), 2.93 (m, 4H), 3.18 (m, 4H),6.70 (d, 1H, *J* 8.0), 7.28-7.40 (m, 5H), 7.67(d, 1H *J* 7.5), 7.89 (d, ¹H *J* 9.5), 7.94-7.98 (m, 2H), 8.47 (s, 2H), 8.57 (s, 1H), 9.54 (s, 1H), 10.25 (br s, 1H). HRMS *m*/*z* 567.1912 [M+H]⁺.

***N*-(6-(4-Isopropylpiperazin-1-yl)pyridin-3-yl)-4-(6-(pyrimidin-5-yl)imidazo[1,2-*a*]pyridin-3-yl)pyrimidin-2-amine (53)**. Prepared by treatment of 4-(6-bromoimidazo[1,2-*a*]pyridin-3-yl)-*N*-(6-(4-isopropylpiperazin-1-yl)pyridin-3-yl)pyrimidin-2-amine (125 mg, 0.26 mmol) with 5-(4,4,5,5-tetramethyl-1,3,2-dioxaborolan-2-yl)pyrimidine (110 mg, 0.53 mmol) using the general synthetic procedure F. A yellow solid (48 mg, 37%). ¹H NMR (DMSO-*d*₆) *δ* 1.00 (s, 3H), 1.01 (s, 3H), 2.67 (m, 1H), 3.26 (m, 4H), 6.55 (br s, 1H), 7.35 (d, 1H, *J* 4.0), 7.77 (d, 1H, *J* 7.5), 7.86-7.93 (m, 2H), 8.31 (s, 1H), 8.41 (m, 1H), 8.62 (s, 1H), 9.11 (br s, 2H), 9.25 (s, 1H), 9.41 (s, 1H), 10.15 (br s, 1H). HRMS *m*/*z* 493.2573 [M+H]⁺.

***N*-(6-(4-Isopropylpiperazin-1-yl)pyridin-3-yl)-4-(6-(thiophen-3-yl)imidazo[1,2-*a*]pyridin-3-yl)pyrimidin-2-amine (54).** Prepared by treatment of 4-(6-bromoimidazo[1,2-*a*]pyridin-3-yl)-*N*-(6-(4-isopropylpiperazin-1-yl)pyridin-3-yl)pyrimidin-2-amine (125 mg, 0.26 mmol) with thiophene-2-boronic acid (67 mg, 0.52 mmol) using the general synthetic procedure F. A beige solid (40 mg, 31%). ¹H NMR (DMSO-*d*₆) *δ* 1.00 (s, 3H), 1.01 (s, 3H), 2.67 (m, 1H), 3.34 (m, 4H), 6.68 (br s, 1H), 7.31 (d, 2H, *J* 5.0), 7.61 (br s, 1H), 7.78-7.85 (m, 4H), 8.34 (s, 1H), 8.40 (d, 1H, *J* 5.0), 8.54 (s, 1H), 9.36 (s, 1H), 10.01 (br s, 1H). HRMS *m*/*z* 497.2238 [M+H]⁺.

**4-(6-(6-Fluoropyridin-3-yl)imidazo[1,2-*a*]pyridin-3-yl)-*N*-(6-(4-isopropylpiperazin-1-yl) pyridin-3-yl)pyrimidin-2-amine (55).** Prepared by treatment of 4-(6-bromoimidazo[1,2-*a*]pyridin-3-yl)-N-(6-(4-isopropylpiperazin-1-yl)pyridin-3-yl)pyrimidin-2-amine (125 mg, 0.26 mmol) with (6-fluoropyridin-3-yl)boronic acid (75 mg, 0.53 mmol) using the general synthetic procedure F. A yellow solid (69 mg, 52%). ¹H NMR (DMSO-*d*₆) *δ* 1.01 (m, 6H), 2.67 (m, 1H), 3.25 (m, 4H), 6.56 (br s, 1H), 7.24 (br s, 1H), 7.34 (d, 1H, *J* 4.0), 7.76-7.81 (m, 2H), 7.88 (d, 1H, *J* 8.5), 8.10 (br s, 1H), 8.30 (s, 1H), 8.41 (m, 1H), 8.53 (br s, 1H), 8.59 (s, 1H), 9.38 (s, 1H), 10.05 (br s, 1H). HRMS *m*/*z* 510.2532 [M+H]⁺.

**4-(6-(6-Fluoropyridin-3-yl)imidazo[1,2-*a*]pyridin-3-yl)-*N*-(6-(4-(methylsulfonyl)piperazin-1-yl)pyridin-3-yl)pyrimidin-2-amine (56)**. Prepared by treatment of 4-(6-bromoimidazo[1,2-*a*]pyridin-3-yl)-*N*-(6-(4-(methylsulfonyl)piperazin-1-yl)pyridin-3-yl)pyrimidin-2-amine (135 mg, 0.25 mmol) with (6-fluoropyridin-3-yl)boronic acid (75 mg, 0.53 mmol) using the general synthetic procedure F. A yellow solid (63 mg, 46%). ¹H NMR (DMSO-*d*₆) *δ* 2.91 (s, 3H), 3.17 (m, 4H), 3.42 (m, 4H), 6.66 (br s, 1H), 7.27 (s, 1H), 7.35 (d, 1H, *J* 5.0), 7.80 (m, 2H), 7.89 (d, 1H, *J* 9.5), 8.16 (br s, 1H), 8.35 (s, 1H), 8.42 (d, 1H, *J* 4.5), 8.51 (br s, 1H), 8.60 (s, 1H), 9.44 (s, 1H), 10.07 (br s, 1H). HRMS *m*/*z* 546.1837 [M+H]⁺.

***N*⁵-(4-(6-Bromoimidazo|1,2-*a*]pyridin-3-yl)pyrimidin-2-yl)-*N*²-(tetrahydro-2*H*-pyran-4-yl)pyridine-2,5-diamine (57)**. Prepared by treatment of 1-(6-((tetrahydro-2*H*-pyran-4-yl)amino)pyridin-3-yl)guanidine (470 mg, 2.00 mmol) with (*E*)-1-(6-bromoimidazo[1,2-*a*]pyridin-3-yl)-3-(dimethylamino) prop-2-en-1-one (300 mg, 1.02 mmol) using the general synthetic procedure D. A yellow solid (190 mg, 41%). ¹H NMR (DMSO-*d*₆) *δ* 1.43 (d, 2H, *J* 9.5), 1.88 (m, 2H), 3.40 (t, 2H, *J* 11.5), 3.86-3.89 (m, 3H), 6.37 (d, 1H, *J* 6.0), 6.56 (d, 1H, *J* 8.5), 7.29 (d, 1H, *J* 5.5), 7.56 (m, 2H), 7.72 (d, 1H, *J* 9.5), 8.04 (br s, 1H), 8.35 (d, 1H, *J* 5.5), 8.59 (s, 1H), 9.21 (br s, 1H), 9.91 (br s, 1H). HRMS *m*/*z* 466.0994 [M(⁷⁹Br)+H]⁺, 468.0969 [M(⁸¹Br)+H]⁺.

***N*⁵-(4-(6-Phenylimidazo[1,2-*a*]pyridin-3-yl)pyrimidin-2-yl)-*N*²-(tetrahydro-2*H*-pyran-4-yl)pyridine-2,5-diamine (58).** Prepared by treatment of *N*⁵-(4-(6-bromoimidazo[1,2-*a*]pyridin-3-yl)pyrimidin-2-yl)-*N*²-(tetrahydro-2*H*-pyran-4-yl)pyridine-2,5-diamine (120 mg, 0.26 mmol) with phenylboronic acid (61 mg, 0.50 mmol) using the general synthetic procedure F. A yellow solid (87 mg, 72%). ¹H NMR (DMSO-*d*₆) *δ* 1.38 (d, 2H, *J* 9.0), 1.83 (m, 2H), 3.37 (t, 2H, *J* 11.5), 3.77-3.85 (m, 3H), 6.25 (d, 1H, J7.0), 6.37 (d, 1H, *J* 7.5), 7.28 (d, 1H, *J* 5.5), 7.38-7.58 (m, 6H), 7.79 (d, 1H, *J9.5),* 7.84 (d, 1H, *J* 9.0), 8.13 (s, 1H), 8.37 (d, 1H, *J* 5.0), 8.54 (s, 1H), 9.18 (s, 1H), 9.99 (br s, 1H). HRMS *m*/*z* 464.2202 [M+H]⁺.

**4-(6-Bromoimidazo[1,2-*a*]pyridin-3-yl)-*N*-(6-(4-(ethylsulfonyl)piperazin-1-yl)pyridin-3-yl)pyrimidin-2-amine (59).** Prepared by treatment of 1-(6-(4-(ethylsulfonyl)piperazin-1-yl)pyridin-3-yl)guanidine (625 mg, 2.00 mmol) with (*E*)-1-(6-bromoimidazo[1,2-*a*]pyridin-3-yl)-3-(dimethylamino) prop-2-en-1-one (300 mg, 1.02 mmol) using the general synthetic procedure D. A yellow solid (246 mg, 45%). ¹H NMR (DMSO-*d*₆) *δ* 1.23 (t, 3H, *J* 6.5), 3.11 (d, 2H, *J* 7.0), 3.29 (m, 4H), 3.55 (m, 4H), 6.95 (d, 1H, *J* 8.5), 7.35 (d, 1H, *J* 4.5), 7.58 (d, 1H, *J* 9.0), 7.74 (d, 1H, *J* 9.0), 7.87 (m, 1H), 8.32 (s, 1H), 8.40 (d, 1H, *J* 4.0), 8.61 (s, 1H), 9.48 (br s, 1H), 9.96 (br, s, 1H). HRMS *m*/*z* 543.0925 [M(⁷⁹Br)+H]⁺, 545.0906 [M(⁸¹Br)+H]⁺.

***N*-(6-(4-(Ethylsulfonyl)piperazin-1-yl)pyridin-3-yl)-4-(6-phenylimidazo[1.2-*a*]pyridin-3-yl)pyrimidin-2-amine (60).** Prepared by treatment of 4-(6-bromoimidazo[1,2-*a*]pyridin-3-yl)-*N*-(6-(4-(ethylsulfonyl)piperazin-1-yl)pyridin-3-yl)pyrimidin-2-amine (135 mg, 0.25 mmol) with phenylboronic acid (61 mg, 0.50 mmol) using the general synthetic procedure F. A yellow solid (83 mg, 61%). ¹H NMR (DMSO-*d*₆) *δ* 1.23 (t, 3H, *J* 7.5), 3.09 (d, 2H, *J* 7.5), 3.23 (m, 4H), 3.38 (m, 4H), 6.57 (br s, 1H), 7.34 (d, 1H, *J* 5.5), 7.43 (m, 3H), 7.57 (m, 2H), 7.78 (d, 1H, *J* 9.5), 7.84 (d, 2H, *J* 9.0), 8.34 (s, 1H), 8.42 (d, 1H, *J* 5.0), 8.56 (s, 1H), 9.43 (s, 1H), 9.98 (br s, 1H). HRMS *m*/*z* 541.2137 [M+H]⁺.

***N*-(6-(4-(Ethylsulfonyl)piperazin-1-yl)pyridin-3-yl)-4-(6-(6-fluoropyridin-3-yl)imidazo[1,2-*a*]pyridin-3-yl)pyrimidin-2-amine (61)**. Prepared by treatment of 4-(6-bromoimidazo[1,2-*a*]pyridin-3-yl)-*N*-(6-(4-(ethylsulfonyl)piperazin-1-yl)pyridin-3-yl)pyrimidin-2-amine (135 mg, 0.25 mmol) with (6-fluoropyridin-3-yl)boronic acid (70 mg, 0.50 mmol) using the general synthetic procedure F. A beige solid (103 mg, 74%). ¹H NMR (DMSO-*d*₆) *δ* 1.24 (t, 3H, *J* 7.5), 3.08 (d, 2H, *J* 7.5), 3.24 (m, 4H), 3.38 (m, 4H), 6.64 (br s, 1H), 7.25 (s, 1H), 7.35 (d, 1H, *J* 5.0), 7.80 (d, 2H, *J* 9.0), 7.88 (d, 1H, *J* 9.5), 8.14 (br s, 1H), 8.35 (s, 1H), 8.42 (d, 1H, *J* 5.0), 8.50 (br s, 1H), 8.60 (s, 1H), 9.43 (s, 1H), 10.06 (br s, 1H). HRMS *m*/*z* 560.2015 [M+H]⁺.

**(4-(5-((4-(6-Bromoimidazo[1,2-*a*]pyridin-3-yl)pyrimidin-2-yl)amino)pyridin-2-yl)piperazin-1-yl)(furan-2-yl)methanone (62)**. Prepared by treatment of 1-(6-(4-(furan-2-carbonyl)piperazin-1-yl)pyridin-3-yl)guanidine (629 mg, 2.00 mmol) with (*E*)-1-(6-bromoimidazo[1,2-*a*]pyridin-3-yl)-3-(dimethylamino) prop-2-en-1-one (300 mg, 1.02 mmol) using the general synthetic procedure D. A yellow solid (132 mg, 24%). ¹H NMR (DMSO-*d*₆) *δ* 3.56 (m, 4H), 3.80 (m, 4H), 6.65 (s, 1H), 6.93 (d, 1H, J9.0), 7.05 (m, 1H), 7.34 (d, 1H, *J* 5.0), 7.58 (d, 1H, *J* 9.0), 7.73 (d, 1H, *J* 9.5), 7.87 (m, 2H), 8.32 (br s, 1H), 8.40 (d, 1H, *J* 5.0), 8.61 (s, 1H), 9.48 (br s, 1H), 9.95 (br s, 1H). HRMS *m*/*z* 545.1044 [M(⁷⁹Br)+H]⁺, 547.1023 [M(⁸¹Br)+H]⁺.

**Furan-2-yl(4-(5-((4-(6-phenylimidazo[1,2-*a*]pyridin-3-yl)pyrimidin-2-yl)amino)pyridin-2-yl)piperazin-1-yl)methanone (63)**. Prepared by treatment of (4-(5-((4-(6-bromoimidazo[1,2-*a*]pyridin-3-yl)pyrimidin-2-yl)amino)pyridin-2-yl)piperazin-1-yl)(furan-2-yl)methanone (135 mg, 0.25 mmol) with phenylboronic acid (61 mg, 0.50 mmol) using the general synthetic procedure F. A yellow solid (39 mg, 29%). ¹H NMR (DMSO-*d*₆) *δ* 3.58 (m, 4H), 3.82 (m, 4H), 6.68 (s, 1H), 6.95 (d, 1H, *J* 9.0), 7.08 (m, 1H), 7.34 (d, 1H, *J* 5.0), 7.44 (m, 2H), 7.55-7.59 (m, 4H), 7.76 (d, 1H, *J* 9.0), 7.89 (m, 2H), 8.35 (br s, 1H), 8.43 (d, 1H, *J* 5.0), 8.64 (s, 1H), 9.51 (br s, 1H), 9.98 (br s, 1H). HRMS *m*/*z* 543.2246 [M+H]⁺.

**4-(6-(3-Fluoro-5-methoxyphenyl)imidazo[1,2-*a*]pyridin-3-yl)-*N*-(6-(4-methylpiperazin-1-yl)pyridin-3-yl)pyrimidin-2-amine (64)**. Prepared by treatment of 4-(6-bromoimidazo[1,2-*a*]pyridin-3-yl)-*N*-(6-(4-methylpiperazin-1-yl)pyridin-3-yl)pyrimidin-2-amine (120 mg, 0.26 mmol) with 2-(3-fluoro-5-methoxyphenyl)-4,4,5,5-tetramethyl-1,3,2-dioxaborolane (126 mg, 0.50 mmol) using the general synthetic procedure F. A yellow solid (82 mg, 62%). ¹H NMR (DMSO-*d*₆) *δ* 2.61 (s, 3H), 2.91 (m, 4H), 3.80 (s, 3H), 6.53 (br s, 1H), 6.90 (d, 1H, *J* 10.5), 7.03 (m, 2H), 7.34 (d, 1H, *J* 5.5), 7.78- 7.88 (m, 3H), 8.35 (s, 1H), 8.42 (d, 1H, *J* 5.0), 8.56 (s, 1H), 9.51 (s, 1H), 10.02 (br s, 1H). HRMS *m*/*z* 511.2371 [M+H]⁺.

***N*⁵-(4-(6-Bromoimidazo[1,2-*a*]pyridin-3-yl)pyrimidin-2-yl)-*N*²*N*²-dimethylpyridine-2,5-diamine (65).** Prepared by treatment of 1-(6-(dimethylamino)pyridin-3-yl)guanidine (358 mg, 2.00 mmol) with (*E*)-1-(6-bromoimidazo[1,2-*a*]pyridin-3-yl)-3-(dimethylamino) prop-2-en-1-one (300 mg, 1.02 mmol) using the general synthetic procedure D. A yellow solid (230 mg, 56%). ¹H NMR (DMSO-*d*₆) *δ* 3.02 (s, 6H), 6.71 (d, 1H, *J* 9.0), 7.31 (d, 1H, *J* 5.0), 7.57 (d, 1H, J 9.5), 7.71-7.75 (m, 2H), 8.21 (br s, 1H), 8.37 (d, 1H, J 5.0), 8.60 (s, 1H), 9.33 (br s, 1H), 9.89 (br s, 1H). HRMS *m*/*z* 410.0723 [M(⁷⁹Br)+H]⁺, 412.0710 [M(⁸¹Br)+H]⁺.

***N*²,*N*²-Dimethyl-*N*⁵-(4-(6-phenylimidazo[1,2-*a*]pyridin-3-yl)pyrimidin-2-yl)pyridine-2,5-diamine (66).** Prepared by treatment of *N*⁵-(4-(6-bromoimidazo[1,2-*a*]pyridin-3-yl)pyrimidin-2-yl)-*N*²,*N*²-dimethylpyridine-2,5-diamine (105 mg, 0.26 mmol) with phenylboronic acid (61 mg, 0.50 mmol) using the general synthetic procedure F. A yellow solid (66 mg, 65%). ¹H NMR (DMSO-*d*₆) *δ* 2.87 (s, 6H), 6.32 (br s, 1H), 7.30 (d, 1H, *J* 5.0), 7.39 (s, 3H), 7.51 (br s, 2H), 7.71 (d, 1H, *J* 8.5), 7.77 (d, 1H, *J* 9.0), 7.83 (d, 1H, *J* 9.0), 8.22 (s, 1H), 8.39 (d, 1H, *J* 5.0), 8.55 (s, 1H), 9.27 (s, 1H), 9.98 (br s, 1H). HRMS *m*/*z* 408.1935 [M+H]⁺.

***Tert*-butyl 4-(3-(2-((6-(dimethylamino)pyridin-3-yl)amino)pyrimidin-4-yl)imidazo[1,2-*a*]pyridin-6-yl)-3,6-dihydropyridine-1(2*H*)-carboxylate (67)**. Prepared by treatment of *N*⁵-(4-(6-bromoimidazo[1,2-*a*]pyridin-3-yl)pyrimidin-2-yl)-*N*²,*N*²-dimethylpyridine-2,5-diamine (105 mg, 0.26 mmol) with *tert*-butyl 4-(4,4,5,5-tetramethyl-1,3,2-dioxaborolan-2-yl)-3,6-dihydropyridine-1(2*H*)-carboxylate (155 mg, 0.50 mmol) using the general synthetic procedure F. A beige solid (98 mg, 74%). ¹H NMR (DMSO-*d*₆) *δ* 1.43 (s, 9H), 2.21 (br s, 2H), 2.99 (s, 6H), 3.41 (br s, 2H), 3.94 (br s, 2H), 6.15 (br s 1H), 6.62 (d, 1H, *J* 9.0), 7.26 (d, 1H, *J* 5.0), 7.62-7.72 (m, 3H), 8.29 (s, 1H), 8.37 (d, 1H, *J* 4.0), 8.48 (s, 1H), 9.22 (s, 1H), 9.66 (br s, 1H). HRMS *m*/*z* 513.2709 [M+H]⁺.

***N*⁵-(4-(6-(6-Fluoropyridin-3-yl)imidazo[1,2-*a*]pyridin-3-yl)pyrimidin-2-yl)-*N*²,*N*²**-**dimethylpyridine-2,5-diamine (68).** Prepared by treatment of *N*⁵-(4-(6-bromoimidazo[1,2-*a*]pyridin-3-yl)pyrimidin-2-yl)-*N*²,*N*²-dimethylpyridine-2,5-diamine (105 mg, 0.26 mmol) with (6-fluoropyridin-3-yl)boronic acid (70 mg, 0.50 mmol) using the general synthetic procedure F. A yellow solid (80 mg, 72%). ¹H NMR (DMSO-*d*₆) *δ* 2.85 (s, 6H), 6.32 (br s, 1H), 7.17 (br s, 1H), 7.32 (d, 1H, *J* 5.0), 7.66 (d, 1H, *J* 7.5), 7.79 (d, 1H, *J* 9.0), 7.87 (d, 1H, *J* 9.0), 8.20 (br s, 1H), 8.21 (s, 1H), 8.40 (m, 2H), 8.59 (s, 1H), 9.27 (s, 1H), 10.05 (br s, 1H). HRMS *m*/*z* 427.1794 [M+H]⁺.

***N*²,*N*²-Dimethyl-*N*⁵-(4-(6-(1,2,3,6-tetrahydropyridin-4-yl)imidazo[1,2-*a*]pyridin-3-yl) pyrimidin-2-yl)pyridine-2,5-diamine (69)**. *Tert*-butyl 4-(3-(2-((6-(dimethylamino)pyridin-3-yl)amino)pyrimidin-4-yl)imidazo[1,2-*a*]pyridin-6-yl)-3,6-dihydropyridine-1(2*H*)-carboxylate (95mg, 0.19 mmol) was dissolved in a mixture of TFA/DCM/H₂O (28 mL, 18:9:1). The reaction mixture was heated at 50 °C for overnight, concentrated under reduced pressure and purified by preparative HPLC (H₂O/MeOH) to give the titled product. A beige solid (75 mg, 96%). ¹H NMR (DMSO-*d*₆) *δ* 2.61 (m, 1H), 3.14 (s, 6H), 3.29 (m, 2H), 3.80 (m, 2H), 6.35 (s, 1H), 7.08 (br s, 1H), 7.42 (d, 1H, *J* 4.0), 7.78 (d, 1H, *J* 9.0), 7.83 (d, 1H, *J* 9.0), 8.00 (br s, 1H), 8.48 (d, 2H, *J* 5.0), 9.02 (br s, 2H), 9.64 (s, 1H), 9.81 (br s, 1H). HRMS *m*/*z* 413.2208 [M+H]⁺.

**4-(6-(1-(Difluoromethyl)-1*H*-pyrazol-4-yl)imidazo[1,2-*a*]pyridin-3-yl)-*N*-(6-(4-methyl piperazin-1-yl)pyridin-3-yl)pyrimidin-2-amine (70)**. Prepared by treatment of 4-(6-bromoimidazo[1,2-*a*]pyridin-3-yl)-*N*-(6-(4-methylpiperazin-1-yl)pyridin-3-yl)pyrimidin-2-amine (120 mg, 0.26 mmol) with 1-(difluoromethyl)-4-(4,4,5,5-tetramethyl-1,3,2-dioxaborolan-2-yl)-1*H*-pyrazole (122 mg, 0.50 mmol) using the general synthetic procedure F. A yellow solid (85 mg, 65%). ¹H NMR (DMSO-*d*₆) *δ* 2.21 (s, 3H), 2.38 (m, 4H), 6.73 (br s, 1H), 7.32 (d, 1H, *J* 5.0), 7.75-7.98 (m, 5H), 8.39 (s, 2H), 8.54 (m, 1H), 8.80 (br s, 1H), 9.43 (s, 1H), 10.06 (br s, 1H). HRMS *m*/*z* 503.2249 [M+H]⁺.

**8-(5-((4-(6-Bromoimidazo[1,2-*a*]pyridin-3-yl)pyrimidin-2-yl)amino)pyridin-2-yl)-2,8-diazaspiro[4.5]decan-1-one (71)**. Prepared by treatment of 1-(6-(1-oxo-2,8-diazaspiro[4.5]decan-8-yl)pyridin-3-yl)guanidine (577 mg, 2.00 mmol) with (*E*)-1-(6-bromoimidazo[1,2-*a*]pyridin-3-yl)-3-(dimethylamino) prop-2-en-1-one (300 mg, 1.02 mmol) using the general synthetic procedure D. A yellow solid (268 mg, 52%). ¹H NMR (DMSO-*d*₆) *δ* 1.40 (d, 2H, *J* 13.0), 1.69 (td, 2H, *J* 12.5 & 3.5), 2.03 (t, 2H, *J* 6.5), 2.98 (t, 2H, *J* 12.0), 3.21 (t, 2H, *J* 6.5), 4.16 (d, 2H, *J* 13.0), 6.91 (d, 1H, *J* 9.0), 7.33 (d, 1H, *J* 5.5), 7.57 (m, 2H), 7.73 (d, 1H, *J* 9.5), 7.80 (m, 1H), 8.26 (br s, 1H), 8.39 (d, 1H, *J* 5.0), 8.60 (s, 1H), 9.40 (br s, 1H), 9.96 (br s, 1H). HRMS *m*/*z* 519.1256 [M(⁷⁹Br)+H]⁺, 521.1231 [M(⁸¹Br)+H]⁺.

**8-(5-((4-(6-Phenylimidazo[1,2-*a*]pyridin-3-yl)pyrimidin-2-yl)amino)pyridin-2-yl)-2,8-diazaspiro[4.5]decan-1-one (72)**. Prepared by treatment of 8-(5-((4-(6-bromoimidazo[1,2-*a*]pyridin-3-yl)pyrimidin-2-yl)amino)pyridin-2-yl)-2,8-diazaspiro[4.5]decan-1-one (130 mg, 0.25 mmol) with phenylboronic acid (61 mg, 0.50 mmol) using the general synthetic procedure F. A yellow solid (74 mg, 57%). ¹H NMR (DMSO-*d*₆) *δ* 1.36 (d, 2H, *J* 13.0), 1.64 (t, 2H, *J* 12.5), 2.00 (t, 2H, *J* 6.5), 2.83 (t, 2H, *J* 12.0), 3.20 (t, 2H, *J* 6.5), 3.97 (m, 2H), 6.52 (br s, 1H), 7.32 (d, 1H, *J* 5.0), 7.43-7.58 (m, 6H), 7.77 (d, 2H, *J* 8.5), 7.84 (d, 1H, *J* 9.5), 8.28 (s, 1H), 8.40 (d, 1H, *J* 5.0), 8.55 (s, 1H), 9.35 (s, 1H), 9.97 (br s, 1H). HRMS *m*/*z* 517.2461 [M+H]⁺.

**8-(5-((4-(6-(1-(Difluoromethyl)-1*H*-pyrazol-4-yl)imidazo[1,2-*a*]pyridin-3-yl)pyrimidin-2-yl)amino)pyridin-2-yl)-2,8-diazaspiro[4.5]decan-1-one (73)**. Prepared by treatment of 8-(5-((4-(6-bromoimidazo[1,2-*a*]pyridin-3-yl)pyrimidin-2-yl)amino)pyridin-2-yl)-2,8-diazaspiro[4.5]decan-1-one (130 mg, 0.25 mmol) with 1-(difluoromethyl)-4-(4,4,5,5-tetramethyl-1,3,2-dioxaborolan-2-yl)-1*H-*pyrazole (122 mg, 0.50 mmol) using the general synthetic procedure F. A yellow solid (83 mg, 60%). ¹H NMR (DMSO-*d*₆) *δ* 1.37 (d, 2H, *J* 13.0), 1.66 (t, 2H, *J* 11.5), 2.01 (t, 2H, *J* 6.5), 2.90 (t, 2H, *J* 12.0), 3.20 (t, 2H, *J* 6.5), 4.04 (m, 2H), 6.75 (br s, 1H), 7.31 (d, 1H, *J* 5.0), 7.58 (s, 1H), 7.76-8.00 (m, 5H), 8.38 (m, 2H), 8.54 (s, 1H), 8.81 (br s, 1H), 9.40 (s, 1H), 10.06 (br s, 1H). HRMS *m*/*z* 557.2329 [M+H]⁺.

***N*⁵-(4-(6-Bromoimidazo[1,2-*a*]pyridin-3-yl)pyrimidin-2-yl)-*N*²-propylpyridine-2,5-diamine (74)**. Prepared by treatment of 1-(6-(propylamino)pyridin-3-yl)guanidine (387 mg, 2.00 mmol) with (*E*)-1-(6-bromoimidazo[1,2-*a*]pyridin-3-yl)-3-(dimetliylamino) prop-2-en-1-one (300 mg, 1.02 mmol) using the general synthetic procedure D. A yellow solid (205 mg, 48%). ¹H NMR (DMSO-*d*₆) *δ* 0.92 (t, 3H,*J* 7.5), 1.54 (q, 2H, *J* 7.5), 3.19 (q, 2H, *J* 6.5), 6.38 (br s, 1H), 6.54 (d, 1H, *J* 8.5), 7.29 (d, 1H, *J* 5.5), 7.55-7.57 (m, 2H), 7.72 (d, 1H, *J9.5),* 8.03 (br s, 1H), 8.35 (d, 1H, *J* 5.0), 8.59 (s, 1H), 9.20 (br s, 1H), 9.90 (br s, 1H). HRMS *m*/*z* 424.0881 [M(⁷⁹Br)+H]⁺, 426.0864 [M(⁸¹Br)+H]⁺.

***N*⁵-(4-(6-Phenylimidazo[1,2-*a*]pyridin-3-yl)pyrimidin-2-yl)-*N*²-propylpyridine-2,5-diamine (75)**. Prepared by treatment of *N*⁵-(4-(6-bromoimidazo[1,2-*a*]pyridin-3-yl)pyrimidin-2-yl)-*N*²-propylpyridine-2,5-diamine (106 mg, 0.25 mmol) with phenylboronic acid (61 mg, 0.50 mmol) using the general synthetic procedure F. A yellow solid (72 mg, 68%). ¹H NMR (DMSO-*d*₆) *δ* 0.89 (t, 3H, *J* 7.5), 1.50 (q, 2H, *J* 7.5), 3.07 (q, 2H, *J* 6.5), 6.24 (br s, 1H), 6.32 (d, 1H, *J* 7.0), 7.28 (d, 1H, *J* 5.5), 7.37-7.57 (m, 6H), 7.78 (d, 1H, *J* 9.5), 7.83 (d, 1H, *J* 9.0), 8.11 (s, 1H), 8.36 (d, 1H, *J* 5.0), 8.54 (s, 1H), 9.17 (s, 1H), 9.98 (br s, 1H). HRMS *m*/*z* 422.2097 [M+H]⁺.

***N*⁵-(4-(6-(1-(Difluoromethyl)-1*H*-pyrazol-4-yl)imidazo[1,2-*a*]pyridin-3-yl)pyrimidin-2-yl)-*N*²-propyloyridine-2,5-diamine (76)** Prepared by treatment of *N*⁵-(4-(6-bromoimidazo[1,2-*a*]pyridin-3-yl)pyrimidin-2-yl)-*N*²-propylpyridine-2,5-diamine (106 mg, 0.25 mmol) with 1-(difluoromethyl)-4-(4,4,5,5-tetramethyl-1,3,2-dioxaborolan-2-yl)-1H-pyrazole (122 mg, 0.50 mmol) using the general synthetic procedure F. A yellow solid (76 mg, 66%). ¹H NMR (DMSO-*d*₆) *δ* 0.89 (t, 3H, *J* 7.5), 1.50 (q, 2H, *J* 7.5), 3.12 (m, 2H), 6.24 (br s, 1H), 6.41 (d, 1H, *J* 5.5), 7.27 (d, 1H, *J* 5.0), 7.62 (d, 1H, *J* 8.5), 7.74-7.97 (m, 3H), 8.19 (s, 1H), 8.34 (d, 1H, *J* 5.0), 8.54 (s, 1H), 8.79 (br s, 1H), 9.24 (s, 1H), 10.09 (br s, 1H). HRMS *m*/*z* 462.1963 [M+H]⁺.

**4-(6-Bromoimidazo[1,2-*a*]pyridin-3-yl)-*N*-(6-((4-methylpiperazin-1-yl)methyl)pyridin-3-yl)pyrimidin-2-amine (77).** Prepared by treatment of 1-(6-((4-methylpiperazin-1-yl)methyl)pyridin-3-yl)guanidine (497 mg, 2.00 mmol) with (*E*)-1-(6-bromoimidazo[1,2-*a*]pyridin-3-yl)-3-(dimethylamino) prop-2-en-1-one (300 mg, 1.02 mmol) using the general synthetic procedure D. A beige solid (176 mg, 37%). ¹H NMR (DMSO-*d*₆) *δ* 2.18 (s, 3H), 2.36 (m, 4H), 2.44 (m, 4H), 3.56 (s, 2H), 7.42 (d, 1H, *J* 8.0), 7.46 (d, 1H, *J* 5.0), 7.62 (d, 1H, *J* 9.5), 7.76 (d, 1H, *J* 9.5), 8.18 (d, 1H, *J* 8.5), 8.48 (d, 1H, *J* 5.0), 8.65 (s, 1H), 8.69 (s, 1H), 9.91 (s, 1H), 10.05 (br s, 1H). HRMS *m*/*z* 479.1314 [M(⁷⁹Br)+H]⁺, 481.1292 [M(⁸¹Br)+H]⁺.

***N*-(6-((4-Methylpiperazin-1-yl)methyl)pyridin-3-yl)-4-(6-phenylimidazo[1,2-*a*]pyridin-3-yl)pyrimidin-2-amine (78)**. Prepared by treatment of 4-(6-bromoimidazo[1,2-*a*]pyridin-3-yl)-*N*-(6-((4-methylpiperazin-1-yl)methyl)pyridin-3-yl)pyrimidin-2-amine (120 mg, 0.25 mmol) with phenylboronic acid (61 mg, 0.50 mmol) using the general synthetic procedure F. A white solid (58 mg, 49%). ¹H NMR (DMSO-*d*₆) *δ* 2.14 (s, 3H), 2.31 (m, 4H), 2.36 (m, 4H), 4.41 (s, 2H), 7.12 (d, 1H, *J* 8.0), 7.42-7.45 (m, 4H), 7.62 (d, 2H *J* 6.0), 7.80 (d, 1H, *J* 9.0), 7.87 (d, 1H, *J* 9.5), 8.10 (d, 1H, *J* 8.0), 8.49 (d, 1H, *J* 5.0), 8.60 (s, 1H), 8.71 (s, 1H), 9.82 (s, 1H), 10.01 (br s, 1H). HRMS *m*/*z* 477.2530 [M+H]⁺.

**4-(6-Bromoimidazo[1,2-*a*]pyridin-3-yl)-*N*-(6-((4-ethylpiperazin-1-yl)methyl)pyridin-3-yl)pyrimidin-2-amine (79).** Prepared by treatment of 1-(6-((4-ethylpiperazin-1-yl)methyl)pyridin-3-yl)guanidine (525 mg, 2.00 mmol) with (*E*)-1-(6-bromoimidazo[1,2-*a*]pyridin-3-yl)-3-(dimethylamino) prop-2-en-1-one (300 mg, 1.02 mmol) using the general synthetic procedure D. A beige solid (239 mg, 48%). ¹H NMR (DMSO-*d*₆) *δ* 0.98 (t, 3H, *J* 7.0), 2.31 (m, 4H), 2.44 (m, 4H), 3.55 (s, 2H), 7.42 (d, 1H, *J* 8.0), 7.46 (d, 1H, *J* 5.0), 7.62 (d, 1H, *J* 9.5), 7.76 (d, 1H, *J* 9.5), 8.18 (d, 1H, *J* 8.5), 8.48 (d, 1H, *J* 5.5), 8.65 (s, 1H), 8.69 (s, 1H), 9.90 (s, 1H), 10.05 (br s, 1H). HRMS *m*/*z* 493.1488 [M(⁷⁹Br)+H]⁺, 495.1468 [M(⁸¹Br)+H]⁺.

***N*-(6-((4-Ethylpiperazin-1-yl)methyl)pyridin-3-yl)-4-(6-phenylimidazo[1,2-*a*]pyridin-3-yl)pyrimidin-2-amine (80).** Prepared by treatment of 4-(6-bromoimidazo[1,2-*a*]pyridin-3-yl)-*N*-(6-((4-ethylpiperazin-1-yl)methyl)pyridin-3-yl)pyrimidin-2-amine (125 mg, 0.25 mmol) with phenylboronic acid (61 mg, 0.50 mmol) using the general synthetic procedure F. A yellow solid (90 mg, 73%). ¹H NMR (DMSO-*d*₆) *δ* 0.97 (t, 3H, *J* 7.0), 2.29 (m, 4H), 2.34 (m, 4H), 3.41 (s, 2H), 7.12 (d, 1H, *J* 8.0), 7.43-7.45 (m, 4H), 7.62 (m, 2H), 7.80 (d, 1H, *J* 9.5), 7.86 (d, 1H, *J* 9.5), 8.09 (d, 1H, *J* 8.0), 8.49 (d, 1H, *J* 5.0), 8.60 (s, 1H), 8.71 (s, 1H), 9.82 (s, 1H), 10.01 (br s, 1H). HRMS *m*/*z* 491.2674 [M+H]⁺.

**4-(6-Bromoimidazo[1,2-*a*]pyridin-3-yl)-*N*-(6-((4-(dimethylamino)piperidin-1-yl)methyl) pyridin-3-yl)pyrimidin-2-amine (81).** Prepared by treatment of 1-(6-((4-(dimethylamino)piperidin-1-yl)methyl)pyridin-3-yl)guanidine (553 mg, 2.00 mmol) with (*E*)-1-(6-bromoimidazo[1,2-*a*]pyridin-3-yl)-3-(dimethylamino) prop-2-en-1-one (300 mg, 1.02 mmol) using the general synthetic procedure D. A yellow solid (125 mg, 25%). ¹H NMR (DMSO-*d*₆) *δ* 1.41 (m, 2H), 1.73 (m, 2H), 2.00 (t, 2H, *J* 11.0), 2.15 (m, 2H), 2.21 (s, 6H), 2.88 (d, 2H, *J* 11.5), 7.42 (d, 1H, *J* 8.5), 7.46 (d, 1H, *J* 5.0), 7.62 (d, 1H, *J* 9.5), 7.76 (d, 1H, *J* 9.5), 8.17 (d, 1H, *J* 8.0), 8.48 (d, 1H, *J* 5.0), 8.65 (s, 1H), 8.69 (s, 1H), 9.90 (s, 1H), 10.05 (br s, 1H). HRMS *m*/*z* 507.1618 [M(⁷⁹Br)+H]⁺, 509.1595 [M(⁸¹Br)+H]⁺.

**Methyl 5-((4-(6-bromoimidazo[1,2-*a*]pyridin-3-yl)pyrimidin-2-yl)amino)picolinate (82).** Prepared by treatment of methyl 5-guanidinopicolinate (388 mg, 2.00 mmol) with (*E*)-1-(6-bromoimidazo[1,2-*a*]pyridin-3-yl)-3-(dimethylamino) prop-2-en-1-one (300 mg, 1.02 mmol) using the general synthetic procedure D. A yellow solid (136 mg, 32%). ¹H NMR (DMSO-*d*₆) δ 3.86 (s, 3H), 7.57 (d, 1H, *J* 5.5), 7.64 (d, 1H, *J* 9.5)*,* 7.77 (d, 1H, *J* 9.5*),* 8.08 (d, 1H, J 9.0), 8.51 (d, 1H, *J* 8.5), 8.57 (d, 1H, *J* 5.0), 8.68 (s, 1H), 8.93 (s, 1H), 10.09 (s, 1H), 10.42 (s, 1H). HRMS *m*/*z* 425.0380 [M(⁷⁹Br)+H]⁺, 427.0361 [M(⁸¹Br)+H]⁺.

### Example 2 Biological Activity

### Kinase assays

Inhibition of CDKs were determined using ADP Glo Kinase assays (Promega Corporation, Madison, WI, United States of America). Test compounds are used in 1:3 serial dilutions for 10 concentrations (from 10 - 0.0005 µM) in 0.5% DMSO. Each kinase reaction is performed with kinase reaction buffer (40 nM Tris base pH 7.5, 20 mM MgCl₂, 0.4 mM DTT, 0.1 mg/mL BSA), substrates for each kinase (Histone H1 peptide: CDK1/CyclinB and CDK6/CyclinD3; Histone H1 protein: CDK2/CyclinA2, CDK2/CyclinE1, CDK3/CyclinE1 and CDK5/p25; RB-CTF:CDK4/CyclinD1 and CDK6/CyclinD1; RBER-IRStide: CDK7/CyclinH/Mat1, CDK8/CyclinC and CDK9/CyclinT1), *K*ₘ ATP for each kinase in a total assay volume of 5 µL. The kinase reactions are run for optimised time period at room temperature and then the reaction is stopped by adding 5 µL of ADP Glo reagent. After incubation at room temperature in the dark for 40 min, 8 µL of kinase detection reagent is added to each well and incubated for 30-40 min. Luminescence is measured using an EnVision Multilabel plate reader (PerkinElmer, Buckinghamshire, UK) with an integration time of 1 sec per well. Positive and negative controls were performed in 0.5% DMSO in the presence and absence of each CDK kinase, respectively. Similarly FLT3 kinase reaction is performed with buffer (40 nM Tris base pH 7.5, 20 mM MgCl₂, 0.4 mM DTT, 0.1 mg/mL BSA), Poly (4:1) Glu, Tyr peptide substrate, *K*ₘ ATP for each kinase (FLT3-WT: 200 µM, FLT3-ITD: 100 µM and FLT3-D835Y: 35 µM) and each FLT3 kinase (FLT3-WT: 20 nM, FLT3-ITD: 20 nM and FLT3-D835Y: 5 nM) respectively in a total assay volume of 5 µL. Serial dilutions of 1:3 were prepared for test compounds for 10 concentrations (from 10 µM to 0.5 nM). The kinase reactions are run for an optimised time period (FLT3-WT: 120 min, FLT3-ITD: 80 min, FLT3-D835Y: 100 min) at room temperature and then the reaction was stopped by adding 5 µL of ADP Glo reagent. After incubation at room temperature in the dark for 40 min, 8 µL of kinase detection reagent was added to each well and incubated for 30-40 min. Half-maximal inhibition (IC₅₀) values were calculated using a 4-parameter logistic non-linear regression model with Graphpad prism (Version 6.0). Apparent inhibition constants (*K*_{¡}) values were calculated from *K*ₘ (ATP) and IC₅₀ values for the respective kinases.

### Proliferation assay

Compounds from Example 1 were subjected to standard MTT (3-(4,5-dimethylthiazol-2-yl)-2,5-diphenyltetrazolium bromide) and resazurin assays on solid tumor cell lines and leukemia cell lines, respectively, as previously reported (Wang S et al., J Med Chem 47:1662-1675, 2004 and Diab S. et al. CheMedChem 9:962-972, 2014). Compound concentrations required to inhibit 50% of cell growth (GI₅₀) were calculated using non-linear regression analysis. The following compounds were used as comparators.

| | | |
|---|---|---|
| Comparator compound A | 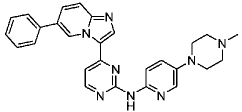 | *N*-(5-(4-methylpiperazin-1-yl)pyridin-2-yl)-4-(6-phenyl imidazo[1,2-*a*]pyridin-3-yl)pyrimidin-2-amine |
| Comparator compound B | 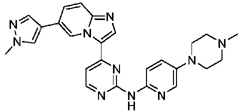 | 4-(6-(1-methyl-1*H*-pyrazol-4-yl)imidazo[1,2-a]pyridin -3-yl)-*N*-(5-(4-methylpiperazin-1-yl)pyridin-2-yl)pyrimidin-2-amine |
| Comparator compound C | 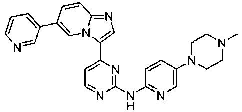 | N-(5-(4-methylpiperazin-1-yl)pyridin-2-yl)-4-(6-(pyridin-3-yl)imidazo[1,2-*a*]pyridin-3-yl)pyrimidin-2-amine |

The results of the kinase and proliferation assays are shown in Table 2. Notably, many of the compounds of the present invention strongly inhibited CDK9. For example, it was found that the presence of a N-(pyridin-3-yl)pyrimidin-2-amine moiety in compounds 6, 7 and 9 instead of the N-(pyridin-2-yl)pyrimidin-2-amine moiety of the comparator compounds A, B and C dramatically and surprisingly increased the potency of inhibition of CDK9.

**Table 2 Enzymatic and cellular activity of example compounds**

| Cmpd | Enzymatic inhibition, *K*_{¡} (µM), or Residual enzymatic activity (%) at 1 µM | | | | | | | 72 h Growth inhibition GI₅₀ (µM) | |
|---|---|---|---|---|---|---|---|---|---|
| | CDK1 B | CDK2 A | CDK4 D1 | CDK6 D3 | CDK7 H | CDK9 T1 | FLT3-ITD | MV-4-11 | A2780 |
| **A** | 87% | 86% | 63% | 86% | 66% | 0.083 | 0.005 | 0.007 | >5 |
| **B** | 98% | 99% | 76% | 87% | 70% | >0.5 | 0.029 | 0.007 | >5 |
| **C** | 67% | 94% | 76% | 87% | 75% | 0.140 | 0.030 | 0.032 | 2.76 |
| **1** | 0.141 | 0.059 | 0.008 | 0.043 | 1.090 | 0.002 | 0.002 | 0.001 | 0.058 |
| **2** | 0.152 | 0.063 | 0.055 | 0.054 | 0.560 | 0.168 | 0.003 | 0.078 | 0.213 |
| **3** | 0.505 | 0.083 | 0.046 | 0.522 | 2.900 | 0.136 | 0.009 | 0.032 | 0.031 |
| **4** | 0.002 | 0.001 | 0.074 | 2.896 | 2.540 | 0.041 | 0.224 | 0.057 | 0.133 |
| **5** | 0.317 | 0.098 | 0.712 | 0.716 | 2.360 | 0.005 | 0.079 | 0.007 | 0.367 |
| **6** | 0.265 | 0.177 | 0.229 | >5 | 0.238 | <0.0001 | 0.004 | 0.004 | 0.045 |
| **7** | 1.100 | 0.825 | >0.5 | 2.159 | 2.725 | 0.0004 | 0.004 | 0.029 | 0.276 |
| **8** | >5 | 2.855 | >0.5 | >5 | >5 | 0.006 | 0.031 | 0.019 | 3.533 |
| **9** | 0.645 | 1.030 | >0.5 | 2.937 | 0.930 | 0.002 | 0.015 | 0.010 | 0.260 |
| **10** | 0.061 | 0.002 | >0.5 | 0.451 | 0.895 | 0.050 | 0.018 | 0.006 | 0.165 |
| **11** | 54% | 0.101 | 21% | 1.008 | 20% | 1.341 | 0.020 | 0.088 | 0.216 |
| **12** | 0.865 | 0.156 | 0.380 | 0.779 | 1.715 | 0.029 | 0.007 | 0.013 | 0.301 |
| **13** | 0.500 | 0.170 | 0.408 | 0.299 | 0.410 | 0.494 | 0.006 | 0.007 | 0.024 |
| **14** | 0.150 | 0.064 | 50% | 1.596 | 1.730 | >5 | 0.009 | 0.049 | 1.128 |
| **15** | 0.010 | 0.003 | 0.543 | 1.004 | 1.630 | 0.234 | 0.015 | 0.020 | 0.230 |
| **16** | 0.153 | 0.124 | 0.718 | 2.094 | 4.502 | 0.077 | 0.007 | 0.010 | 0.067 |
| **17** | >5 | >1 | >0.5 | >5 | >1 | 0.028 | 0.016 | 0.006 | 0.490 |
| **18** | 0.450 | 0.557 | 47% | >5 | 28% | 0.021 | 0.027 | 0.001 | 0.113 |
| **19** | 0.776 | 33% | 83% | >5 | 1.694 | 0.016 | 0.004 | 0.017 | 0.415 |
| **20** | >5 | 41% | 86% | >5 | 0.890 | 0.056 | 1.447 | 0.094 | 0.192 |
| **21** | 0.635 | 28% | 33% | >5 | 3.968 | 0.008 | 0.006 | 0.011 | 0.058 |
| **22** | 0.199 | 0.038 | 0.094 | 0.243 | 0.770 | 0.010 | 0.005 | 0.006 | 0.058 |
| **23** | 67% | 26% | 72% | 46% | >5 | 0.020 | 0.023 | 0.181 | 0.449 |
| **24** | 0.029 | 0.042 | 21% | 0.051 | 0.013 | 0.004 | 0.008 | 0.033 | 0.104 |
| **25** | 100% | 1.085 | 94% | 44% | 0.297 | 0.096 | 0.005 | 0.740 | >3 |
| **26** | 60% | 27% | 92% | 53% | >5 | 0.261 | 0.091 | 0.037 | 0.020 |
| **27** | 84% | 37% | 97% | 61% | >5 | >5 | 0.109 | 0.196 | 2.410 |
| **28** | 61% | 1.120 | 76% | 45% | 0.314 | 0.086 | 0.065 | 0.233 | 0.438 |
| **29** | 69% | 25% | 98% | 61% | >5 | >5 | 0.257 | 0.337 | >3 |
| **30** | 52% | 0.520 | 65% | 23% | 0.223 | 0.075 | 0.148 | 0.056 | 0.442 |
| **31** | 97% | 55% | 95% | 41% | 100% | >1 | 70% | 0.600 | >3 |
| **32** | 96% | 89% | 91% | 44% | 100% | >1 | >1 | 0.620 | 1.921 |
| **33** | 59% | 83% | 96% | 46% | 100% | >0.5 | >1 | 0.296 | >3 |
| **34** | 100% | 86% | 92% | 47% | 92% | 0.330 | >1 | 0.920 | 2.296 |
| **35** | 0.048 | 0.015 | 0.034 | 0.058 | 0.084 | 0.004 | 0.016 | 0.004 | 0.021 |
| **36** | 0.198 | 0.131 | 0.530 | 34% | 31% | 0.034 | 0.010 | 0.018 | 0.209 |
| **37** | 0.151 | 0.072 | 0.154 | 0.215 | 0.174 | 0.014 | 0.026 | 0.024 | 0.046 |
| **38** | 0.145 | 0.044 | 0.049 | 0.295 | 0.132 | 0.010 | 0.010 | 0.039 | 0.033 |
| **39** | 46% | 0.563 | 55% | 41% | 0.149 | 0.009 | 0.012 | 0.088 | 0.258 |
| **40** | 41% | 0.344 | 57% | 52% | 32% | 0.012 | 0.011 | 0.047 | 0.158 |
| **41** | 31% | 22% | 53% | 70% | 77% | 0.091 | 0.032 | 0.027 | 0.717 |
| **42** | 0.078 | 0.031 | 24% | >0.5 | 27% | 0.029 | 0.009 | 0.022 | 0.108 |
| **43** | 67% | 46% | 68% | 46% | 41% | 0.066 | 0.024 | 0.040 | 0.383 |
| **44** | 56% | 28% | 84% | 55% | 77% | 0.719 | 0.137 | 0.021 | 0.366 |
| **45** | 60% | 63% | 91% | 60% | 79% | 0.054 | 0.444 | 0.012 | 0.520 |
| **46** | 21% | 62% | 92% | 31% | 71% | 0.310 | 0.080 | 0.025 | 0.654 |
| **47** | 0.116 | 0.039 | 51% | 0.444 | 0.262 | 0.045 | 0.025 | 0.029 | 0.525 |
| **48** | 58% | 34% | 86% | 30% | 75% | 0.274 | 0.086 | 0.018 | 0.784 |
| **49** | 94% | 51% | 92% | 38% | 81% | 0.171 | >0.5 | 0.017 | 6.114 |
| **50** | 91% | 90% | 94% | 79% | 51% | 0.410 | 0.210 | 0.813 | 0.659 |
| **51** | 77% | 85% | 96% | 43% | 80% | 0.286 | >0.5 | 0.017 | 0.274 |
| **52** | 62% | 97% | - | 100% | 99% | >0.5 | 0.057 | 0.023 | 0.677 |
| **53** | 71% | 75% | - | 66% | 56% | 0.092 | 0.071 | 0.046 | 0.024 |
| **54** | 32% | 0.376 | 48% | 24% | 29% | 0.014 | 0.011 | 0.050 | 0.234 |
| **55** | 50% | 62% | - | 55% | 38% | 0.036 | 0.028 | 0.116 | 0.666 |
| **56** | 78% | 36% | - | 96% | 94% | 0.380 | 0.084 | 0.023 | 0.804 |
| **57** | 57% | 0.056 | - | 93% | 88% | 0.103 | 0.040 | 0.030 | 0.757 |
| **58** | 77% | 73% | 33% | 45% | 73% | 0.401 | 0.140 | 0.028 | 0.335 |
| **59** | 70% | 44% | 52% | 49% | 88% | 0.234 | 0.086 | 0.032 | 0.382 |
| **60** | 61% | 91% | - | 100% | 100% | 0.509 | 0.142 | 0.020 | 0.929 |
| **61** | 38% | 71% | - | 72% | 57% | 0.428 | 0.149 | 0.020 | 0.920 |
| **62** | 39% | 57% | - | 76% | 60% | 0.541 | 0.188 | 0.052 | 0.684 |
| **63** | 26% | 49% | - | 100% | 93% | 0.242 | 0.082 | 0.025 | 0.505 |
| **64** | 57% | 90% | - | 66% | 38% | 0.026 | 0.006 | 0.002 | 0.570 |
| **65** | 56% | 39% | 86% | 0.159 | 71% | 0.142 | 0.024 | 0.041 | 0.036 |
| **66** | 76% | 73% | - | 79% | 64% | 0.468 | 0.220 | 0.017 | 0.507 |
| **67** | 67% | 93% | - | 78% | 68% | 1.627 | >0.5 | 0.362 | 0.757 |
| **68** | 37% | 61% | - | 81% | 71% | 0.174 | 0.062 | 0.013 | 1.464 |
| **69** | 0.116 | 0.082 | - | 81% | 65% | 0.016 | 30% | 0.152 | 0.144 |
| **70** | 32% | 55% | - | 57% | 40% | 0.007 | 0.012 | 0.008 | 0.282 |
| **71** | 95% | 74% | - | 97% | 91% | >0.5 | 0.024 | 0.012 | 0.160 |
| **72** | 75% | 64% | - | 73% | 59% | >0.5 | 0.055 | 0.005 | 0.420 |
| **73** | 73% | 80% | 82% | 97% | 83% | >0.5 | 0.116 | 0.014 | 0.387 |
| **74** | 82% | 41% | 54% | 86% | 78% | 0.102 | 0.040 | 0.020 | 0.590 |
| **75** | 89% | 100% | 89% | 93% | 80% | 0.368 | 0.160 | 0.078 | 2.816 |
| **76** | 96% | 62% | 97% | 71% | 57% | 0.629 | 0.100 | 0.039 | 0.639 |
| **77** | 74% | 0.040 | 52% | 48% | 40% | 0.051 | 0.070 | 0.035 | 0.266 |
| **78** | 99% | 46% | 95% | 64% | 38% | 0.065 | 0.090 | 0.105 | 0.490 |
| **79** | 70% | 0.042 | 48% | 27% | 0.168 | 0.057 | 0.070 | 0.038 | 0.209 |
| **80** | 92% | 37% | 81% | 59% | 0.222 | 0.060 | 0.060 | 0.102 | 0.365 |
| **81** | 84% | 0.041 | 47% | 22% | 0.205 | 0.143 | 0.070 | 0.098 | 0.361 |
| **82** | 90% | 0.052 | 94% | 99% | 93% | 0.163 | 0.180 | 0.262 | 0.103 |

### (-) not tested

Throughout the specification and the claims that follow, unless the context requires otherwise, the words "comprise" and "include" and variations such as "comprising" and "including" will be understood to imply the inclusion of a stated integer or group of integers, but not the exclusion of any other integer or group of integers.

The reference to any prior art in this specification is not, and should not be taken as, an acknowledgement of any form of suggestion that such prior art forms part of the common general knowledge.

It will be appreciated by those skilled in the art that the present disclosure is not restricted in its use to the particular application described. Neither is the present disclosure restricted in its preferred embodiment with regard to the particular elements and/or features described or depicted herein. It will be also appreciated that the disclosure is not limited to the embodiment or embodiments disclosed, but is capable of numerous rearrangements, modifications and substitutions without departing from the scope of the disclosure as set forth and defined by the following claims.

## Claims

1. A compound of formula I: 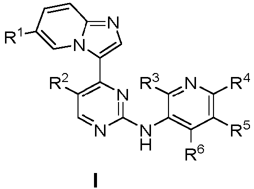 wherein:
R¹, R², R³, R⁴, R⁵ and R⁶ are each independently selected from the group consisting of H, alkyl, alkyl-R⁷, aryl, aryl-R⁷, aralkyl, aralkyl-R⁷, alicyclic, heterocyclic, halogen, NO₂, CN, CF₃, OH, O-alkyl, COR⁷, COOR⁷, O-aryl, O-R⁷, NH₂, NH-alkyl, NH-aryl, N-(alkyl)₂, N-(aryl)₂, N-(alkyl)(aryl), NH-R⁷, NH-alkyl-N(alkyl)₂, N-(R⁷)(R⁸), N-(alkyl)(R⁷), N-(aryl)(R⁷), COOH, CONH₂, CONH-alkyl, CONH-aryl, CONH-alicyclic, CON-(alkyl)(R⁷), CON(aryl)(R⁷), CONH-R⁷, CON-(R⁷)(R⁸), SH-alkyl, SO₃H, SO₂-alkyl, SO₂-alkyl-R⁷, SO₂-aryl, SO₂-aryl-R⁷, SO₂NH₂, SO₂NH-R⁷, SO₂N-(R⁷)(R⁸), CF₃, CO-alkyl, COO-alkyl, CO-alkyl-R⁷, CO-aryl, CO-aryl-R⁷ and R⁹, wherein said alkyl, aryl, aralkyl, alicyclic and heterocyclic groups may be optionally substituted with one or more groups selected from halogen, CN, OH, O-C₁₋₆ alkyl, NH₂, COOH, C₂₋₅ carboxylate, CONH₂ and haloalkyl;
R⁷ and R⁸ are independently selected from water solubilising groups of the group consisting of: mono-, di- and poly-hydroxylated alicyclic groups, di- or poly-hydroxylated aliphatic or aryl groups, N-, O- and/or S-containing heterocyclic groups optionally substituted with one or more hydroxyl, amino or alkoxy groups, aliphatic and aryl groups comprising one or more carboxamide, sulfoxide, sulfone or sulfonamide groups, and halogenated alkylcarbonyl groups; and COOH, SO₃H, OSO₃H, SONHCH₃, SONHCH₂CH₃, SO₂CH₃, SO₂CH₂CH₃, PO₃H₂ and OPO₃H₂; and
R⁹ is selected from water solubilising groups of the group consisting of:
(i) mono-, di- and poly-hydroxylated alicyclic groups, di- or poly-hydroxylated aliphatic or aryl groups; N-, O- and/or S-containing heterocyclic groups optionally substituted with one or more hydroxyl, sulfone, alkyl, (CH₂)ₙ-heterocycle, where n is selected from the integers 1 or 2 and the heterocycle is a saturated or unsaturated 5- or 6-membered cyclic group comprising one or two N, O or S heteroatoms, CO-heterocycle, NH-heterocycle, amino, alkoxy, carboxylate or alkylcarbonyl groups; aliphatic, alicyclic and aryl groups comprising one or more carboxamide, sulfoxide, sulfone or sulfonamide groups; and wherein the N-, O- and/or S-containing heterocyclic group may optionally comprise an alkyl bridge, amine bridge, amide bridge, alkoxy bridge or ketone bridge; and halogenated alkylcarbonyl groups;
(ii) COOH, SO₃H, OSO₃H, PO₃H₂ and OPO₃H₂;
(iii) NHCO(CH₂)ₘ[NHCO(CH₂)_{m'}]ₚ[NHCO(CH₂)_{m"}]_{q}A and NHCO(CH₂)ₜNH(CH₂)_{t'}A wherein p and q are each independently selected from integers 0 or 1, and m, m', m", t and' are each independently selected from integers 1 to 10, and A is selected from:
(a) alicyclic, aryl and heterocyclic groups comprising one or more O-, S- or N- heteroatoms, which may further comprise an alkyl bridge,
(b) alicyclic groups comprising one or more of -O-, NH₂, -NH-, =N-, quaternary amine salt, and amidine, and
(c) morpholine, piperazine or 1,4-diazepane groups, each of which may be optionally substituted by one or more substituents selected from SO₂-alkyl, alkyl optionally substituted by one or more OH groups, CO-alkyl, aralkyl, COO-alkyl, and an ether group optionally substituted by one or more OH groups;
(iv) (CH₂)ₙNR¹⁰COR¹¹, (CH₂)_{n'}NR¹⁰SO₂R¹¹ and SO₂R¹², wherein R¹¹ is selected from H and alkyl, R¹² and R¹² are each independently selected from alkyl groups optionally comprising one or more heteroatoms and/or optionally substituted with one or more substituents independently selected from OH, NH₂, halogen and NO₂, and n and n' are each independently selected from integers 0, 1, 2 and 3;
(v) ether and polyether groups optionally substituted with one or more OH groups or one or more A groups, wherein A is as defined above at (iii);
(vi) (CH₂)ᵣNH₂, wherein r is selected from integers 0, 1, 2 and 3;
(vii) (CH₂)ᵣ·OH, wherein r' is selected from integers 0, 1, 2 and 3;
(viii) (CH₂)_{n"}NR¹³COR¹⁴, wherein R¹³ is H or alkyl, n" is selected from integers 0, 1, 2 and 3, and R¹⁴ is an aryl group optionally substituted with one or more substituents selected from halogen, NO₂, OH, alkoxy, NH₂, COOH, CONH₂ and CF₃; and
(ix) SO₂NR¹⁵R¹⁶, wherein R¹⁵ and R¹⁶ are each independently selected from H, alkyl and aryl, with the proviso that at least one of R¹⁵ and R¹⁶ is other than H, or R¹⁵ and R¹⁶ together form a cyclic group optionally comprising one or more heteroatoms selected from N, O and S, and wherein said alkyl, aryl or cyclic group is optionally substituted by one or more substituents selected from halogen, NO₂, OH, alkoxy, NH₂, COOH, CONH₂ and CF₃;
or a pharmaceutically acceptable salt or solvate thereof.

2. The compound of claim 1, wherein R¹ is H, C₁₋₆ alkyl, aryl, CN, CF₃, NH₂, heterocyclic, O-C₁₋₃ alkyl, NH-C₁₋₆ alkyl, NH(C₁₋₃ alkyl)-N(C₁₋₃ alkyl)₂, NH-aryl, N-(C₁₋₃ alkyl)₂, N-(C₁₋₃ alkyl)(aryl), SH-C₁₋₆ alkyl, halogen or R⁹.

3. The compound of claim 2, wherein R¹ is R⁹ and R⁹ is an N-, O- and/or S-containing heterocyclic group substituted with one or more hydroxyl or amino group.

4. The compound of claim 2, wherein R¹ is H, halogen, CF₃, C₁₋₃ alkyl, phenyl optionally substituted with C₁₋₆ alkyl, O-C₁₋₆ alkyl, amino and/or halogen, or heterocyclic optionally substituted with one or more groups selected from halogen, CN, OH, O-C₁₋₆ alkyl, NH₂, COOH, C₂₋₅ carboxylate, CONH₂ and haloalkyl.

5. The compound of any one of the preceding claims, wherein R² is H, C₁₋₆ alkyl, CN or halogen.

6. The compound of any one of the preceding claims, wherein at least one of R³, R⁴, R⁵ and R⁶ is C₁₋₃ alkyl-R⁷ (wherein R⁷ is selected from COOH, SO₃H, OSO₃H, SONHCH₃, SONHCH₂CH₃, SO₂CH₃, SO₂CH₂CH₃, PO₃H₂ and OPO₃H₂), COOC₁₋alkyl, O-C₁₋₃ alkyl, NH-C₁₋₃ alkyl, N-(C₁₋₃ alkyl)₂, NH-R⁷ (wherein R⁷ is selected from COOH, SO₃H, OSO₃H, SONHCH₃, SONHCH₂CH₃, SO₂CH₃, SO₂CH₂CH₃, PO₃H₂ and OPO₃H₂), CONH-C₃₋₆ alicyclic, halogen or is R⁹.

7. The compound of claim 6, wherein at least one of R³, R⁴, R⁵ and R⁶ is R⁹ and R⁹ is an N-, O- and/or S-containing heterocyclic group substituted with one or more hydroxyl, sulfone, C₁₋₃ alkyl, amino, alkoxy, carboxylate or alkylcarbonyl.

8. The compound of any one of the preceding claims, wherein where R³, R⁵ and R⁶ are H, R⁴ is other than COOH, C(=O)NHOH, C(=O)NHO-tetrahydropyran or CONH-aryl substituted with NH₂.

9. The compound of claim 6, wherein at least one of R³, R⁴, R⁵ and R⁶ is R⁹ and R⁹ is selected from the following: 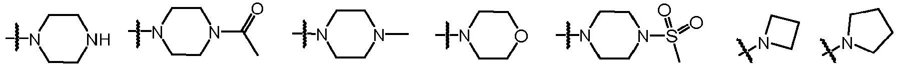 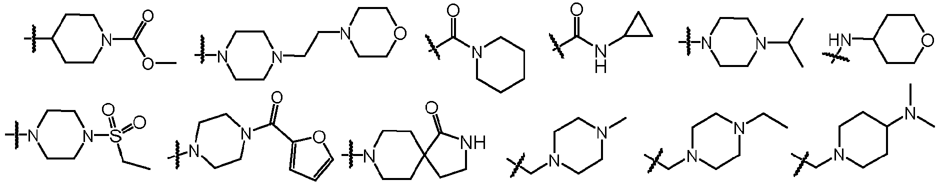 optionally further comprising an alkyl, amino, amide, alkoxy, ether or ketone bridge to the pyridine group.

10. The compound of any one of the preceding claims, wherein R⁴ is R⁹, and R³, R⁵ and R⁶ are H.

11. The compound of claim 1, wherein R¹ is halogen, phenyl or heterocyclic optionally substituted with one or more groups selected from halogen, CN, OH, O-C₁₋₆ alkyl, NH₂, COOH, C₂₋₅ carboxylate, CONH₂ and haloalkyl; R², R³, R⁵ and R⁶ are all H; and R⁴ is selected from the following:

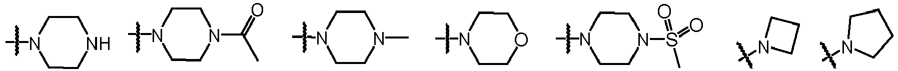

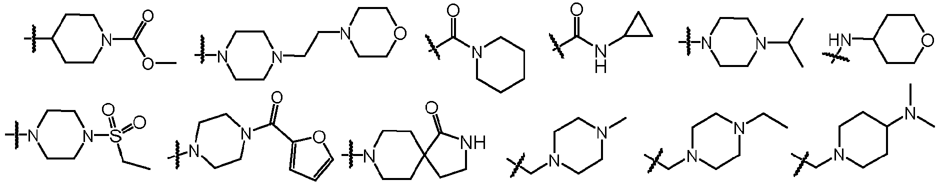

12. The compound of claim 1, wherein the compound is selected from the group consisting of:
*N*-(6-(4-Methylpiperazin-1-yl)pyridin-3-yl)-4-(6-phenylimidazo[1,2-*a*|pyridin-3-yl)pyrimidin-2-amine,
*N*-(6-(4-Methylpiperazin-1-yl)pyridin-3-yl)-4-(6-(pyridin-3-yl)imidazo[1,2-a]pyridin-3-yl)pyrimidin-2-amine,
*N*-(6-(4-(Methylsulfonyl)piperazin-1-yl)pyridin-3-yl)-4-(6-(pyridin-3-yl)imidazo[1,2-*a*]pyridin-3-yl)pyrimidin-2-amine,
*N-*(6-(4-(Methylsulfonyl)piperazin-1-yl)pyridin-3-yl)-4-(6-(thiophen-2-yl)imidazo [1,2-a]pyridin-3-yl)pyrimidin-2-amine,
4-(6-Chloroimidazol[1,2-*a*]pyridin-3-yl)-*N*-(6-(4-methylpiperazin-1-yl)pyridin-3-yl)pyrimidin-2-amine,
*N*-(6-(Azetidin-1-yl)pyridin-3-yl)-4-(6-phenylimidazo[1,2-*a*]pyridin-3-yl)pyrimidin-2-amine,
4-(6-Bromoimidazo[1,2-*a*]pyridin-3-yl)-*N*-(6-(4-isopropylpiperazin-1-yl)pyridin-3-yl)pyrimidin-2-amine,
*N-*(6-(4-Isopropylpiperazin-1-yl)pyridin-3-yl)-4-(6-phenylimidazo[1,2-a]pyridin-3-yl)pyrimidin-2-amine,
4-(6-(1-(Difluoromethyl)-1H-pyrazol-4-yl)imidazo[1,2-a]pyridin-3-yl)-*N*-(6-(4-methylpiperazin-1-yl)pyridin-3-yl)pyrimidin-2-amine, and
4-(6-Bromoimidazo[1,2-a]pyridin-3-yl)-*N*-(6-((4-ethylpiperazin-1-yl)methyl)pyridin-3-yl)pyrimidin-2-amine.

13. A compound according to any one of the preceding claims or a pharmaceutically acceptable salt or solvate thereof, for use in the treatment of cancer or another proliferative cell disease or condition.

14. The compound of claim 13, wherein the cancer or other proliferative cell disease or condition to be treated is **characterised by** over-activation of one or more of CDK2, CDK4, CDK6 and CDK9 or over-expression of FLT3 or FLT3-ITD, and/or other protein kinases selected from TRKs, DYRKs and/or mutant forms.

15. A pharmaceutical composition or medicament comprising the compound of any one of claims 1 to 12 or a pharmaceutically acceptable salt or solvate thereof, and a pharmaceutically acceptable carrier, diluent and/or excipient.

## Patentansprüche

1. Verbindung mit der Formel I: 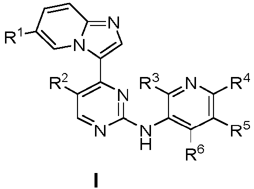 wobei:
R¹, R², R³, R⁴, R⁵ und R⁶ jeweils unabhängig voneinander ausgewählt sind aus der Gruppe bestehend aus H, Alkyl, Alkyl-R⁷, Aryl, Aryl-R⁷, Aralkyl, Aralkyl-R⁷, alizyklisch, heterozyklisch, Halogen, NO₂, CN, CF₃, OH, O-Alkyl, COR⁷, COOR⁷, O-Aryl, O-R⁷, NH₂, NH-Alkyl, NH-Aryl, N-(Alkyl)₂, N-(Aryl)₂, N-(Alkyl) (aryl), NH-R⁷, NH-Alkyl-N(alkyl)₂, N-(R⁷)(R⁶), N-(Alkyl)(R⁷), N-(Aryl) (R⁷), COOH, CONH₂, CONH-Alkyl, CONH-Aryl, CONH-alizyklisch, CON-(Alkyl) (R⁷), CON(Aryl) (R⁷), CONH-R⁷, CON-(R⁷)(R⁸), SH-Alkyl, SO₃H, SO₂-Alkyl, SO₂-Alkyl-R⁷, SO₂-Aryl, SO₂-Aryl-R⁷, SO₂NH₂, SO₂NH-R⁷, SO₂N-(R⁷) (R⁸), CF₃, CO-Alkyl, COO-Alkyl, CO-Alkyl-R⁷, CO-Aryl, CO-Aryl-R⁷ und R⁹, wobei die Alkyl-, Aryl-, Aralkyl-,
alizyklischen und heterozyklischen Gruppen wahlweise durch eine oder mehrere Gruppen, die ausgewählt sind aus Halogen, CN, OH, O-C₁₋₆-Alkyl, NH₂, COOH, C₂₋₅-Carboxylat, CONH₂ und Haloalkyl, substituiert sind;
R⁷ und R⁸ unabhängig voneinander ausgewählt sind aus wasserlöslichen Gruppen der Gruppe bestehend aus: mono-, di- and polyhydroxylierten alizyklischen Gruppen, di- oder polyhydroxylierten aliphatischen oder Arylgruppen, N-, O- und/oder S-haltigen heterozyklischen Gruppen, wahlweise substituiert durch eine oder mehrere Hydroxyl-, Amino- oder Alkoxygruppen, aliphatischen und Arylgruppen, umfassend eine oder mehrere Carboxamid-, Sulfoxid-, Sulfon- oder Sulfonamidgruppen, und halogenierten Alkylcarbonylgruppen; und
COOH, SO₃H, OSO₃H, SONHCH₃, SONHCH₂CH₃, SO₂CH₃, SO₂CH₂CH₃, PO₃H₂ und OPO₃H₂; und
R⁹ ausgewählt ist aus wasserlöslichen Gruppen der Gruppe bestehend aus:
(i) mono-, di- und polyhydroxylierten alizyklischen Gruppen, di- oder polyhydroxylierten aliphatischen oder Arylgruppen; N-, O- und/oder S-haltigen heterozyklischen Gruppen, wahlweise substituiert durch eine oder mehrere Hydroxyl-, Sulfon-, Akyl-, (CH₂)ₙ-Heterozyklusgruppen, wobei n ausgewählt ist aus den Ganzzahlen 1 oder 2 und der Heterozyklus eine gesättigte oder ungesättigte 5- oder 6-gliedrige zyklische Gruppe ist, umfassend ein oder zwei N-, O- oder S-Heteroatome, CO-Heterozyklus-, NH-Heterozyklus-, Amino-, Alkoxy-, Carboxylat- oder Alkylcarbonylgruppen; aliphatischen, alizyklischen und Arylgruppen, umfassend eine oder mehrere Carboxamid-, Sulfoxid-, Sulfon- oder Sulfonamidgruppen; und wobei die N-, O- und/oder S-haltige heterozyklische Gruppe wahlweise eine Alkylbrücke, Aminbrücke, Amidbrücke, Alkoxybrücke oder Ketonbrücke umfassen kann; und halogenierten Alkylcarbonylgruppen;
(ii) COOH, SO₃H, OSO₃H, PO₃H₂ und OPO₃H₂;
(iii) NHCO (CH₂)ₘ[NHCO (CH₂)_{m'}]ₚ[NHCO (CH₂)_{m"}]_{q}A und NHCO(CH₂)ₜNH(CH₂)_{t'}A, wobei p und q jeweils unabhängig voneinander ausgewählt sind aus den Ganzzahlen 0 oder 1, und m, m', m", t und ' jeweils unabhängig voneinander ausgewählt sind aus Ganzzahlen von 1 bis 10, und A ausgewählt ist aus:
(a) alizyklischen, Aryl- und heterozyklischen Gruppen, umfassend ein oder mehrere O-, S- oder N-Heteroatome, die ferner eine Alkylbrücke umfassen können,
(b) alizyklischen Gruppen, umfassend eine oder mehrere von -O-, NH₂, -NH-, =N-, quaternärem Aminsalz und Amidin, und (c) Morpholin-, Piperazin- oder 1,4-Diazepangruppen, von denen jede wahlweise durch einen oder mehrere Substituenten substituiert sein kann, die ausgewählt sind aus SO₂-Alkyl, Alkyl, wahlweise substituiert durch eine oder mehrere OH-Gruppen, CO-Alkyl, Aralkyl, COO-Alkyl, und einer Ethergruppe, wahlweise substituiert durch eine oder mehrere OH-Gruppen;
(iv) (CH₂)ₙNR¹⁰COR¹¹, (CH₂)_{n'}NR¹⁹SO₂R¹¹ und SO₂R¹², wobei R¹¹ ausgewählt ist aus H und Alkyl, R¹² und R¹² jeweils unabhängig voneinander ausgewählt sind aus Alkylgruppen, die wahlweise ein oder mehrere Heteroatome umfassen und/oder wahlweise durch ein oder mehrere Substituenten substituiert sind, die unabhängig voneinander ausgewählt sind aus OH, NH₂, Halogen und NO₂, und wobei n und n' jeweils unabhängig voneinander ausgewählt sind aus den Ganzzahlen 0, 1, 2 und 3;
(v) Ether- und Polyethergruppen, wahlweise substituiert durch eine oder mehrere OH-Gruppen oder eine oder mehrere A-Gruppen, wobei A wie weiter oben unter (iii) definiert ist;
(vi) (CH₂)ᵣNH₂, wobei r ausgewählt ist aus den Ganzzahlen 0, 1, 2 und 3;
(vii) (CH₂)_{r'}OH, wobei r' ausgewählt ist aus den Ganzzahlen 0, 1, 2 und 3;
(viii) (CH₂)_{n"}NR¹³COR¹⁴ wobei R¹³ H oder Alkyl ist, n" ausgewählt ist aus den Ganzzahlen 0, 1, 2 und 3, und R¹⁴ eine Arylgruppe ist, wahlweise substituiert durch einen oder mehrere Substituenten, die ausgewählt sind aus Halogen, NO₂, OH, Alkoxy, NH₂, COOH, CONH₂ und CF₃; und
(ix) SO₂NR¹⁵R¹⁶, wobei R¹⁵ und R¹⁶ jeweils unabhängig voneinander ausgewählt sind aus H, Alkyl und Aryl, mit der Maßgabe, dass mindestens eines von R¹⁵ und R¹⁶ sich von H unterscheidet, oder R¹⁵ und R¹⁶ gemeinsam eine zyklische Gruppe bilden, die wahlweise ein oder mehrere Heteroatome umfassen, die ausgewählt sind aus N, O und S, und wobei die Alkyl-, Aryl- oder zyklische Gruppe wahlweise substituiert ist durch einen oder mehrere Substituenten, die ausgewählt sind aus Halogen, NO₂, OH, Alkoxy, NH₂, COOH, CONH₂ und CF₃;
oder pharmazeutisch akzeptables Salz oder Solvat hiervon.

2. Verbindung nach Anspruch 1, wobei R¹ H, C₁₋₆-Alkyl, Aryl, CN, CF₃, NH₂, heterozyklisch, O-C₁₋₃-Alkyl, NH-C₁₋₆-Alkyl, NH(C₁₋₃-Alkyl)-N(C₁₋₃-alkyl)₂, NH-Aryl, N-(C₁₋₃-Alkyl)₂, N-(C₁₋₃-Alkyl) (aryl), SH-C₁₋₆-Alkyl, Halogen oder R⁹ ist.

3. Verbindung nach Anspruch 2, wobei R¹ R⁹ ist und R⁹ eine N-, O- und/oder S-haltige heterozyklische Gruppe ist, die durch eine oder mehrere Hydroxyl- oder Aminogruppe substituiert ist.

4. Verbindung nach Anspruch 2, wobei R¹ H, Halogen, CF₃, C₁₋₃-Alkyl, Phenyl, wahlweise substituiert durch C₁₋₆-Alkyl, O-C₁₋₆-Alkyl, Amino und/oder Halogen, oder heterozyklisch ist, wahlweise substituiert durch eine oder mehrere Gruppen, die ausgewählt sind aus Halogen, CN, OH, O-C₁₋₆-Alkyl, NH₂, COOH, C_{2- 5}-Carboxylat, CONH₂ und Haloalkyl.

5. Verbindung nach einem der vorhergehenden Ansprüche, wobei R² H, C₁₋₆-Alkyl, CN oder Halogen ist.

6. Verbindung nach einem der vorhergehenden Ansprüche, wobei mindestens eines von R³, R⁴, R⁵ und R⁶ C₁₋₃-Alkyl-R⁷ ist (wobei R⁷ ausgewählt ist aus COOH, SO₃H, OSO₃H, SONHCH₃, SONHCH₂CH₃, SO₂CH₃, SO₂CH₂CH₃, PO₃H₂ und OPO₃H₂), COOC₁-Alkyl, O-C₁₋₃-Alkyl, NH-C₁₋₃-Alkyl, N-(C₁₋₃-Alkyl)₂, NH-R⁷ (wobei R⁷ ausgewählt ist aus COOH, SO₃H, OSO₃H, SONHCH₃, SONHCH₂CH₃, SO₂CH₃, SO₂CH₂CH₃, PO₃H₂ und OPO₃H₂), CONH-C₃₋₆-alizyklisch, Halogen oder R⁹ ist.

7. Verbindung nach Anspruch 6, wobei mindestens eines von R³, R⁴, R⁵ und R⁶ R⁹ ist und R⁹ eine N-, O- und/oder S-haltige heterozyklische Gruppe ist, substituiert durch ein oder mehrere Hydroxyl-, Sulfon-, C₁₋₃-Alkyl-, Amino-, Alkoxy-, Carboxylat- oder Alkylcarbonylgruppen.

8. Verbindung nach einem der vorhergehenden Ansprüche, wobei R³, R⁵ und R⁶ H sind, R⁴ sich von COOH, C(=O)NHOH, C(=O)NHO-Tetrahydropyran unterscheidet oder CONH-Aryl durch NH₂ substituiert ist.

9. Verbindung nach Anspruch 6, wobei mindestens eines von R³, R⁴, R⁵ und R⁶ R⁹ ist und R⁹ aus Folgenden ausgewählt ist: 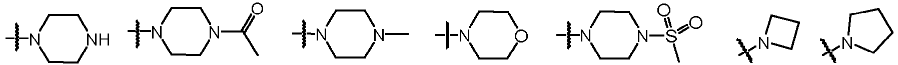 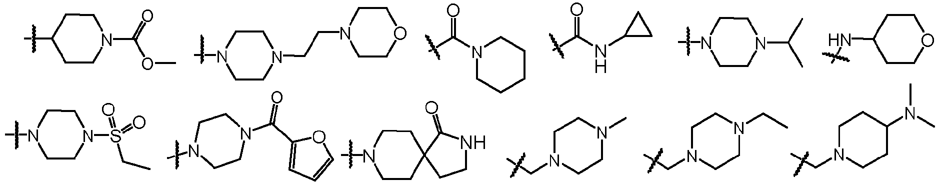 wahlweise ferner umfassend eine Alkyl-, Amino-, Amid-, Alkoxy-, Ether- oder Ketonbrücke an der Pyridingruppe.

10. Verbindung nach einem der vorhergehenden Ansprüche, wobei R⁴ R⁹ ist und R³, R⁵ und R⁶ H sind.

11. Verbindung nach Anspruch 1, wobei R¹ Halogen, Phenyl oder heterozyklisch ist, wahlweise substituiert durch eine oder mehrere Gruppen, die ausgewählt sind aus Halogen, CN, OH, O-C₁₋₆ Alkyl, NH₂, COOH, C₂₋₅-Carboxylat, CONH₂ und Haloalkyl; R², R³, R⁵ und R⁶ alle H sind; und R⁴ ausgewählt ist aus den Folgenden:

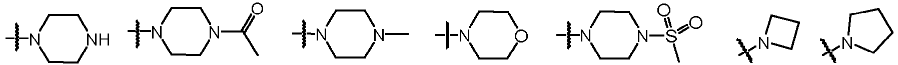

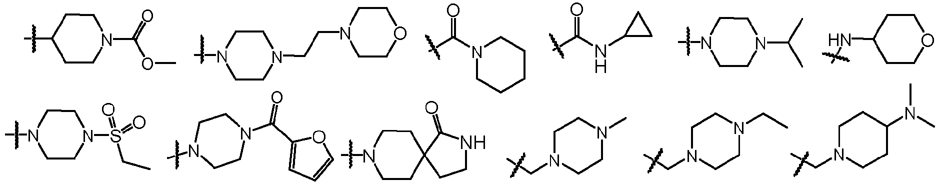

12. Verbindung nach Anspruch 1, wobei die Verbindung ausgewählt ist aus der Gruppe bestehend aus:
*N*-(6-(4-Methylpiperazin-1-yl)pyridin-3-yl)-4-(6-phenylimidazo[1,2-a]pyridin-3-yl)pyrimidin-2-amin,
*N*-(6-(4-Methylpiperazin-1-yl)pyridin-3-yl)-4-(6-(pyridin-3-yl)imidazo[1,2-a]pyridin-3-yl)pyrimidin-2-amin,
*N*-(6-(4-(Methylsulfonyl)piperazin-1-yl)pyridin-3-yl)-4-(6-(pyridin-3-yl)imidazo[1,2-a]pyridin-3-yl)pyrimidin-2-amin,
*N*-(6-(4-(Methylsulfonyl)piperazin-1-yl)pyridin-3-yl)-4-(6-(thiophen-2-yl)imidazo[1,2-a]pyridin-3-yl)pyrimidin-2-amin,
4-(6-Chlorimidazo[1,2-a]pyridin-3-yl)-*N*-(6-(4-methylpiperazin-1-yl)pyridin-3-yl)pyrimidin-2-amin,
*N*-(6-(Azetidin-1-yl)pyridin-3-yl)-4-(6-phenylimidazo[1,2-a]pyridin-3-yl)pyrimidin-2-amin,
4-(6-Bromimidazo[1,2-a]pyridin-3-yl)-*N*-(6-(4-isopropylpiperazin-1-yl)pyridin-3-yl)pyrimidin-2-amin,
*N*-(6-(4-Isopropylpiperazin-1-yl)pyridin-3-yl)-4-(6-phenylimidazo[1,2-a]pyridin-3-yl)pyrimidin-2-amin,
4-(6-(1-(Difluormethyl)-1H-pyrazol-4-yl)imidazo[1,2-a]pyridin-3-yl)-N-(6-(4-methylpiperazin-1-yl)pyridin-3-yl)pyrimidin-2-amin und
4-(6-Bromimidazo[1,2-a]pyridin-3-yl)-*N*-(6-((4-ethylpiperazin-1-yl)methyl)pyridin-3-yl)pyrimidin-2-amin.

13. Verbindung nach einem der vorhergehenden Ansprüche oder pharmazeutisch akzeptables Salz oder Solvat hiervon zur Anwendung bei der Behandlung von Krebs und einer anderen proliferativen Zellkrankheit oder -störung.

14. Verbindung nach Anspruch 13, wobei der zu behandelnde Krebs oder die andere zu behandelnde proliferative Zellerkrankheit oder -störung durch eine Überaktivierung von einem oder mehreren von CDK2, CDK4, CDK6 und CDK9 oder eine Überexpression von FLT3 oder FLT3-ITD und/oder anderer Proteinkinasen, die aus TRK, DYRK und/oder mutierten Formen ausgewählt sind, gekennzeichnet ist.

15. Pharmazeutische Zusammensetzung oder Medikament, umfassend die Verbindung nach einem der Ansprüche 1 bis 12 oder pharmazeutisch akzeptables Salz oder Solvat hiervon, und pharmazeutisch akzeptabler Träger, pharmazeutisch akzeptables Verdünnungsmittel und/oder pharmazeutisch akzeptabler Hilfsstoff.

## Revendications

1. Composé de formule I : 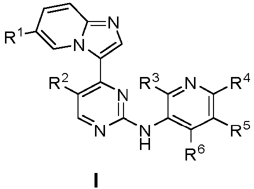 où :
R¹, R², R³, R⁴, R⁵ et R⁶ sont chacun indépendamment sélectionnés dans le groupe consistant en un H, alkyle, alkyl-R⁷, aryle, aryl-R⁷, aralkyle, aralkyl-R⁷, alicycle, hétérocycle, halogène, NO₂, CN, CF₃, OH, O-alkyle, COR⁷, COOR⁷, O-aryle, O-R⁷, NH₂, NH-alkyle, NH-aryle, N-(alkyle)₂, N-(aryle)₂, N-(alkyl)(aryle), NH-R⁷, NH-alkyl-N(alkyle)₂, N-(R⁷)(R⁸), N-(alkyl)(R⁷), N-(aryl)(R⁷), COOH, CONH₂, CONH-alkyle, CONH-aryle, CONH-alicycle, CON-(alkyl)(R⁷), CON(aryl)(R⁷), CONH-R⁷, CON-(R⁷)(R⁸), SH-alkyle, SO₃H, SO₂-alkyle, SO₂-alkyl-R⁷, SO₂-aryle, SO₂-aryl-R⁷, SO₂NH₂, SO₂NH-R⁷, SO₂N-(R⁷)(R⁸), CF₃, CO-alkyle, COO-alkyle, CO-alkyl-R⁷, CO-aryle, CO-aryl-R⁷ et R⁹, où lesdits groupements alkyle, aryle, aralkyle, alicycle et hétérocycle peuvent éventuellement être substitués par un ou plusieurs groupements sélectionnés parmi un halogène, CN, OH, O-alkyle en C₁₋₆, NH₂, COOH, carboxylate en C₂₋₅, CONH₂ et haloalkyle ;
R⁷ et R⁸ sont indépendamment sélectionnés parmi des groupements hydrosolubilisants du groupe consistant en les suivants : groupements alicycles mono-, di- et polyhydroxylés, groupements aliphatiques ou aryles di- ou polyhydroxylés, groupements hétérocycles contenant un N-, O- et/ou S éventuellement substitués par un ou plusieurs groupements hydroxyles, aminos ou alkoxys, groupements aliphatiques et aryles comprenant un ou plusieurs groupements carboxamides, sulfoxydes, sulfones ou sulfonamides et groupements alkylcarbonyles halogénés ; et COOH, SO₃H, OSO₃H, SONHCH₃, SONHCH₂CH₃, SO₂CH₃, SO₂CH₂CH₃, PO₃H₂ et OPO₃H₂ ; et
R⁹ est sélectionné parmi des groupements hydrosolubilisants du groupe consistant en les suivants :
(i) groupements alicycles mono-, di- et polyhydroxylés, groupements aliphatiques ou aryles di- ou polyhydroxylés ; groupements hétérocycles contenant un N-, O- et/ou S éventuellement substitués par un ou plusieurs groupements hydroxyles, sulfones, alkyles, (CH₂)ₙ-hétérocycles, où n est sélectionné parmi les nombres entiers 1 ou 2 et l'hétérocycle est un groupement cyclique à 5 ou 6 chaînons saturé ou insaturé comprenant un ou deux hétéroatomes de type N, O ou S, ou groupements CO-hétérocycles, NH-hétérocycles, aminos, alkoxys, carboxylates ou alkylcarbonyles ; groupements aliphatiques, alicycles et aryles comprenant un ou plusieurs groupements carboxamides, sulfoxydes, sulfones ou sulfonamides ; et où le groupement hétérocycle contenant un N-, O- et/ou S peut éventuellement comprendre un pont alkyle, un pont amine, un pont amide, un pont alkoxy ou un pont cétone ; et groupements alkylcarbonyles halogénés ;
(ii) COOH, SO₃H, OSO₃H, PO₃H₂ et OPO₃H₂ ;
(iii) NHCO(CH₂)ₘ[NHCO(CH₂)_{m'}]ₚ[NHCO(CH₂)_{m"}]_{q}A et NHCO(CH₂)ₜNH(CH₂)_{t'}A, où p et q sont chacun indépendamment sélectionnés parmi les nombres entiers 0 ou 1 et m, m', m", t et ' sont chacun indépendamment sélectionnés parmi les nombres entiers 1 à 10 et A est sélectionné parmi :
(a) des groupements alicycles, aryles et hétérocycles comprenant un ou plusieurs hétéroatomes de type O-, S- ou N- qui peuvent en outre comprendre un pont alkyle,
(b) des groupements alicycles comprenant un ou plusieurs des suivants : -O-, NH₂, -NH-, =N-, sel d'amine quaternaire et amidine et
(c) des groupements morpholines, pipérazines ou 1,4-diazépanes qui peuvent chacun être éventuellement substitués par un ou plusieurs substituants sélectionnés parmi les suivants : SO₂-alkyle, alkyle éventuellement substitué par un ou plusieurs groupements OH, CO-alkyle, aralkyle, COO-alkyle, et groupement éther éventuellement substitué par un ou plusieurs groupements OH ;
(iv) (CH₂)ₙNR¹⁰COR¹¹, (CH₂)_{n'}NR¹⁰SO₂R¹¹ et SO₂R¹², où R¹¹ est sélectionné parmi un H et un alkyle, R¹² et R¹² sont chacun indépendamment sélectionnés parmi des groupements alkyles comprenant éventuellement un ou plusieurs hétéroatomes et/ou éventuellement substitués par un ou plusieurs substituants indépendamment sélectionnés parmi un OH, NH₂, halogène et NO₂, et n et n' sont chacun indépendamment sélectionnés parmi les nombres entiers 0, 1, 2 et 3 ;
(v) des groupements éthers et polyéthers éventuellement substitués par un ou plusieurs groupements OH ou un ou plusieurs groupements A, où A est tel que défini à la rubrique (iii) ci-dessus ;
(vi) (CH₂)ᵣNH₂, où r est sélectionné parmi les nombres entiers 0, 1, 2 et 3 ;
(vii) (CH₂)_{r'}OH, où r' est sélectionné parmi les nombres entiers 0, 1, 2 et 3 ;
(viii) (CH₂)_{n"}NR¹³COR¹⁴, où R¹³ représente un H ou un alkyle, n" est sélectionné parmi les nombres entiers 0, 1, 2 et 3, et R¹⁴ représente un groupement aryle éventuellement substitué par un ou plusieurs substituants sélectionnés parmi un halogène, NO₂, OH, alkoxy, NH₂, COOH, CONH₂ et CF₃ ; et
(ix) SO₂NR¹⁵R¹⁶, où R¹⁵ et R¹⁶ sont chacun indépendamment sélectionnés parmi un H, un alkyle et un aryle, à condition qu'au moins un des radicaux R¹⁵ et R¹⁶ est autre qu'un H ; ou R¹⁵ et R¹⁶ forment éventuellement ensemble un groupement cyclique comprenant un ou plusieurs hétéroatomes sélectionnés parmi N, O et S, et où ledit groupement alkyle, aryle ou cyclique est éventuellement substitué par un ou plusieurs substituants sélectionnés parmi un halogène, NO₂, OH, alkoxy, NH₂, COOH, CONH₂ et CF₃ ;
ou sel ou solvate pharmaceutiquement acceptable de celui-ci.

2. Composé de la revendication 1, où R' représente un H ou un groupement alkyle en C₁₋₆, aryle, CN, CF₃, NH₂, hétérocycle, O-alkyle en C₁₋₃, NH-alkyle en C₁₋₆, NH(alkyl en C₁₋₃)-N(alkyle en C₁₋₃)₂, NH-aryle, N-(alkyle en C₁₋₃)₂, N-(alkyl en C₁₋₃)(aryle), SH-alkyle en C₁₋₆, halogène ou R⁹.

3. Composé de la revendication 2, où R¹ représente un R⁹ et R⁹ représente un groupement hétérocycle contenant un N-, O- et/ou S substitué par un ou plusieurs groupements hydroxyles ou aminos.

4. Composé de la revendication 2, où R¹ représente un H, halogène, CF₃, alkyle en C₁₋₃, phényle éventuellement substitué par un alkyle en C₁₋₆, un O-alkyle en C₁₋₆, un amino et/ou un halogène, ou composé hétérocycle éventuellement substitué par un ou plusieurs groupements sélectionnés parmi un halogène, CN, OH, O-alkyle en C₁₋₆, NH₂, COOH, carboxylate en C₂₋₅, CONH₂ et haloalkyle.

5. Composé de l'une quelconque des revendications précédentes, où R² représente un H, alkyle en C₁₋₆, CN ou halogène.

6. Composé de l'une quelconque des revendications précédentes, où au moins un des radicaux R³, R⁴, R⁵ et R⁶ représente un (alkyl en C₁₋₃)-R⁷ (où R⁷ est sélectionné parmi un COOH, SO₃H, OSO₃H, SONHCH₃, SONHCH₂CH₃, SO₂CH₃, SO₂CH₂CH₃, PO₃H₂ et OPO₃H₂), COO-alkyle en C₁, O-alkyle en C₁₋₃, NH-alkyle en C₁₋₃, N-(alkyle en C₁₋₃)₂, NH-R⁷ (où R⁷ est sélectionné parmi un COOH, SO₃H, OSO₃H, SONHCH₃, SONHCH₂CH₃, SO₂CH₃, SO₂CH₂CH₃, PO₃H₂ et OPO₃H₂), CONH-groupement alicycle en C₃₋₆, halogène ou R⁹.

7. Composé de la revendication 6, où au moins un des radicaux R³, R⁴, R⁵ et R⁶ représente un R⁹ et R⁹ représente un groupement hétérocycle contenant un N-, O- et/ou S substitué par un ou plusieurs groupements hydroxyles, sulfones, alkyles en C₁₋₃, aminos, alkoxys, carboxylates ou alkylcarbonyles.

8. Composé de l'une quelconque des revendications précédentes, où quand R³, R⁵ et R⁶ représente un H, R⁴ représente un groupement autre qu'un COOH, C(=O)NHOH, C(=O)NHO-tétrahydropyrane ou CONH-aryle substitué par un NH₂.

9. Composé de la revendication 6, où au moins un des radicaux R³, R⁴, R⁵ et R⁶ représente un R⁹ et R⁹ est sélectionné parmi les suivants : 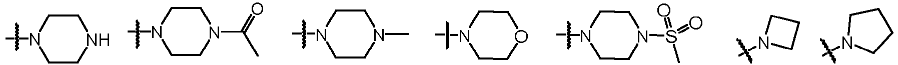 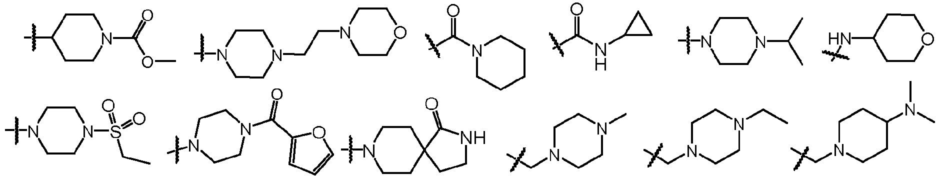 comprenant en outre éventuellement un pont alkyle, amino, amide, alkoxy, éther ou cétone relié au groupement pyridine.

10. Composé de l'une quelconque des revendications précédentes, où R⁴ représente un R⁹ et où R³, R⁵ et R⁶ représentent un H.

11. Composé de la revendication 1, où R' représente un halogène, un phényle ou un groupement hétérocycle éventuellement substitué par un ou plusieurs groupements sélectionnés parmi un halogène, CN, OH, O-alkyle en C₁₋₆, NH₂, COOH, carboxylate en C₂₋₅, CONH₂ et haloalkyle ; R², R³, R⁵ et R⁶ représentent chacun un H ; et R⁴ est sélectionné parmi les suivants :

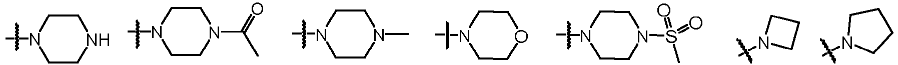

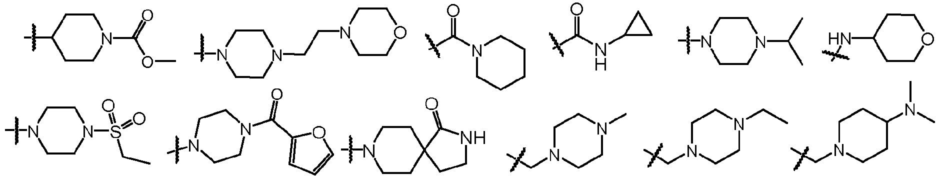

12. Composé de la revendication 1, où le composé est sélectionné dans le groupe consistant en les suivants :
*N*-(6-(4-Méthylpipérazin-1-yl)pyridin-3-yl)-4-(6-phénylimidazo[1,2-*a*]pyridin-3-yl)pyrimidin-2-amine,
*N*-(6-(4-Méthylpipérazin-1-yl)pyridin-3-yl)-4-(6-(pyridin-3-yl)imidazo[1,2-*a*]pyridin-3-yl)pyrimidin-2-amine,
*N*-(6-(4-(Méthylsulfonyl)pipérazin-1-yl)pyridin-3-yl)-4-(6-(pyridin-3-yl)imidazo[1,2-*a*]pyridin-3-yl)pyrimidin-2-amine,
*N*-(6-(4-(Méthylsulfonyl)pipérazin-1-yl)pyridin-3-yl)-4-(6-(thiophén-2-yl)imidazo[1,2*-a*]pyridin-3-yl)pyrimidin-2-amine,
4-(6-Chloroimidazo[1,2-*a*]pyridin-3-yl)-*N*-(6-(4-méthylpipérazin-1-yl)pyridin-3-yl)pyrimidin-2-amine,
*N*-(6-(Azétidin-1-yl)pyridin-3-yl)-4-(6-phénylimidazo[1,2-*a*]pyridin-3-yl)pyrimidin-2-amine,
4-(6-Bromoimidazo[1,2-*a*]pyridin-3-yl)-*N*-(6-(4-isopropylpipérazin-1-yl)pyridin-3-yl)pyrimidin-2-amine,
*N*-(6-(4-Isopropylpipérazin-1-yl)pyridin-3-yl)-4-(6-phénylimidazo[1,2-*a*]pyridin-3-yl)pyrimidin-2-amine,
4-(6-(1-(Difluorométhyl)-1*H*-pyrazol-4-yl)imidazo[1,2-*a*]pyridin-3-yl)-*N*-(6-(4-méthylpipérazin-1-yl)pyridin-3-yl)pyrimidin-2-amine et
4-(6-Bromoimidazo[1,2*-a*]pyridin-3-yl)-*N*-(6-((4-éthylpipérazin-1-yl)méthyl)pyridin-3-yl)pyrimidin-2-amine.

13. Composé selon l'une quelconque des revendications précédentes ou sel ou solvate pharmaceutiquement acceptable de celui-ci pour une utilisation dans le traitement d'un cancer ou d'une autre maladie ou affection marquée par une prolifération cellulaire.

14. Composé de la revendication 13, où le cancer ou l'autre maladie ou affection à traiter marquée par une prolifération cellulaire est **caractérisé par** une suractivation de une ou plusieurs parmi CDK2, CDK4, CDK6 et CDK9 ou par une surexpression de FLT3 ou de FLT3-ITD et/ou d'autres protéines kinases sélectionnées parmi des **TRK,** DYRK et/ou formes mutantes.

15. Composition pharmaceutique ou médicament comprenant le composé de l'une quelconque des revendications 1 à 12 ou un sel ou solvate pharmaceutiquement acceptable de celui-ci et un véhicule, diluant et/ou excipient pharmaceutiquement acceptable.
